(19) Europäisches Patentamt
European Patent Office
Office européen des brevets

(11) EP 3 677 676 A1

(12) EUROPEAN PATENT APPLICATION

(43) Date of publication:
08.07.2020 Bulletin 2020/28

(21) Application number: 19150242.6

(22) Date of filing: 03.01.2019

(51) Int Cl.:
*C12N 9/96* (2006.01) *C12N 9/28* (2006.01)
*C12N 9/54* (2006.01) *C11D 3/386* (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: BASF SE
67056 Ludwigshafen am Rhein (DE)

(72) Inventors:
- HUEFFER, Stephan
67056 Ludwigshafen (DE)
- SPANGENBERG, Olivier
67056 Ludwigshafen (DE)
- GARCIA MARCOS, Alejandra
67056 Ludwigshafen (DE)
- WEBER, Heike
67056 Ludwigshafen (DE)
- TUECKING, Katrin-Stephanie
67056 Ludwigshafen (DE)
- FISCHER, Stefan
68623 Lampertheim (DE)

(74) Representative: BASF IP Association
BASF SE
G-FLP-C006
67056 Ludwigshafen (DE)

# (54) COMPOUNDS STABILIZING AMYLASES IN LIQUIDS

(57) Enzyme preparation comprising
component (a): at least one compound according to general formula (I)

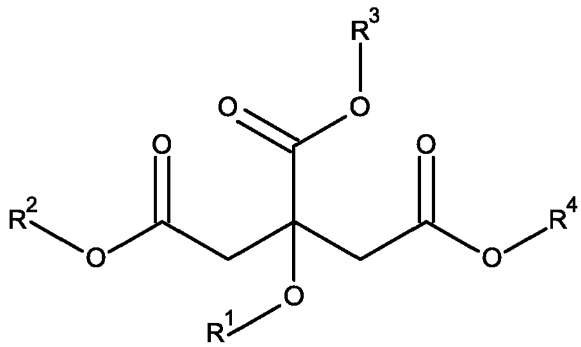

(I)

wherein the variables of formula (i) are as follows:
$R^1$ is selected from H and $C_1$-$C_{10}$ alkylcarbonyl, wherein alkyl may be linear or branched and may bear one or more hydroxyl groups;
$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H

component (b): at least one enzyme selected from the group of hydrolases (EC 3), preferably at least one enzyme selected from the group of amylases, more preferably at least one enzyme selected from the group of alpha-amylases (EC 3.2.1.1); and/or at least one enzyme is selected from proteases, preferably from subtilisin type proteases (EC 3.4.21.62);
and optionally
component (c): at least one compound selected from solvents, enzyme stabilizers different from component (a), and



Printed by Jouve, 75001 PARIS (FR)

(Cont. next page)

compounds stabilizing the liquid enzyme preparation as such.

## Description

The present invention is directed to an enzyme preparation, preferably a liquid enzyme preparation, comprising

component (a): at least one compound according to general formula (I)

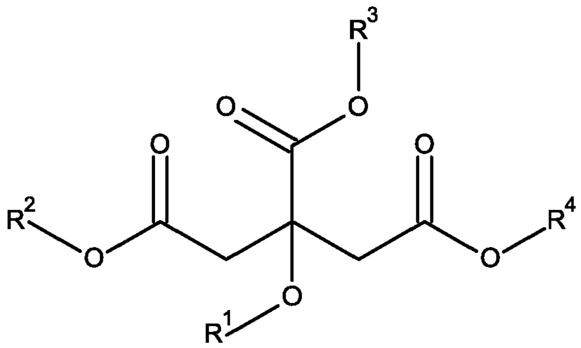

(I)

wherein the variables in formula (I) are as follows:

$R^1$ is selected from H and $C_1$-$C_{10}$ alkylcarbonyl, wherein alkyl may be linear or branched and may bear one or more hydroxyl groups,

$R^2$, $R^3$, $R^4$ are independently from each other selected from H, linear $C_1$-$C_5$ alkyl, and branched $C_3$-$C_{10}$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H;

component (b): at least one enzyme selected from the group of hydrolases (EC 3), preferably at least one enzyme selected from the group of amylases, more preferably at least one enzyme selected from the group of alpha-amylases (EC 3.2.1.1); and/or at least one enzyme is selected from proteases, preferably from subtilisin type proteases (EC 3.4.21.62);

and optionally

component (c): at least one compound selected from solvents, enzyme stabilizers different from component (a), and compounds stabilizing the liquid enzyme preparation as such.

Enzymes are usually produced commercially as a liquid concentrate, frequently derived from a fermentation broth. The enzyme tends to loose enzymatic activity if it remains in an aqueous environment and so it is conventional practice to convert it to an anhydrous form: aqueous concentrates may be lyophilized or spray-dried e.g. in the presence of a carrier material to form aggregates. Usually, solid enzyme products need to be "dissolved" prior to use. To stabilize enzymes in liquid products enzyme inhibitors are usually employed, preferably reversible enzyme inhibitors, to inhibit enzyme activity temporarily until the enzyme inhibitor is released.

The problem to be solved for the current invention relates to providing a compound helping to reduce loss of enzymatic activity during storage of liquid enzyme containing products, even if the liquid enzyme containing product comprises complexing agents such as EDTA and/or DTPA and/or MGDA and/or GLDA. It was a further objective of the present invention to provide an enzyme preparation that allows to be flexibly formulated into liquid detergent formulations or cleaning formulations with either one type of enzymes or mixtures of enzymes.

The problem was solved by providing compounds according to general formula (I):

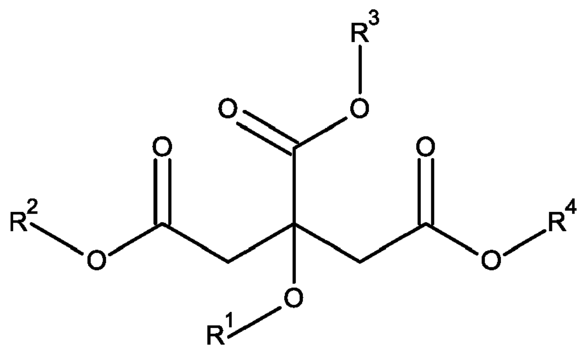

(I)

wherein the variables in formula (I) are as follows:

$R^1$ is selected from H and $C_1$-$C_{10}$ alkylcarbonyl, wherein alkyl may be linear or branched and may bear one or more hydroxyl groups,

$R^2$, $R^3$, $R^4$ are independently from each other selected from H, linear $C_1$-$C_5$ alkyl, and branched $C_3$-$C_{10}$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H; and

wherein said compound supports retention of enzymatic activity of at least one enzyme selected from the group of hydrolases (EC 3), preferably from the group of amylases; during storage of the same within liquid products.

Enzyme names are known to those skilled in the art based on the recommendations of the Nomenclature Committee of the International Union of Biochemistry and Molecular Biology (IUBMB). Enzyme names include: an EC (Enzyme Commission) number, recommended name, alternative names (if any), catalytic activity, and other factors.; see http://www.sbcs.qmul.ac.uk/iubmb/enzyme/EC3/ in the version last updated on 28 June, 2018.

In one aspect, the invention provides an enzyme preparation containing

component (a): at least one enzyme stabilizer selected from compounds according to general formula (I)

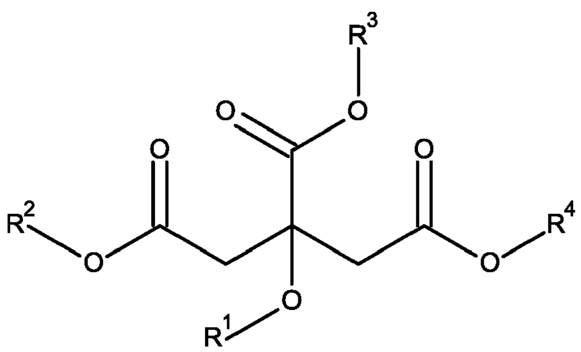

(I)

wherein the variables in formula (I) are as follows:

$R^1$ is selected from H and $C_1$-$C_{10}$ alkylcarbonyl, wherein alkyl may be linear or branched and may bear one or more hydroxyl groups,

$R^2$, $R^3$, $R^4$ are independently from each other selected from H, linear $C_1$-$C_5$ alkyl, and branched $C_3$-$C_{10}$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H, and

component (b): at least one enzyme selected from the group of hydrolases (EC 3), preferably at least one enzyme

selected from the group of amylases, more preferably at least one enzyme selected from the group of alpha-amylases (EC 3.2.1.1); and/or at least one enzyme is selected from proteases, preferably from subtilisin type proteases (EC 3.4.21.62);

and optionally

component (c): at least one compound selected from solvents, enzyme stabilizers different from component (a), and compounds stabilizing the liquid enzyme preparation as such.

The enzyme preparation of the invention may be liquid at 20°C and 101.3 kPa. Liquids include solutions, emulsions and dispersions, gels etc. as long as the liquid is fluid and pourable. In one embodiment of the present invention, liquid detergent formulations according to the present invention have a dynamic viscosity in the range of about 500 to about 20,000 mPa*s, determined at 25°C according to Brookfield, for example spindle 3 at 20 rpm with a Brookfield viscosimeter LVT-II.

In one embodiment, liquid means that the enzyme preparation does not show visible precipitate formation or turbidity after storage of the liquid enzyme preparation, preferably after at least 20 days of storage at 37°C.

**Component (a)**

More specifically, component (a) is a compound of general formula (I)

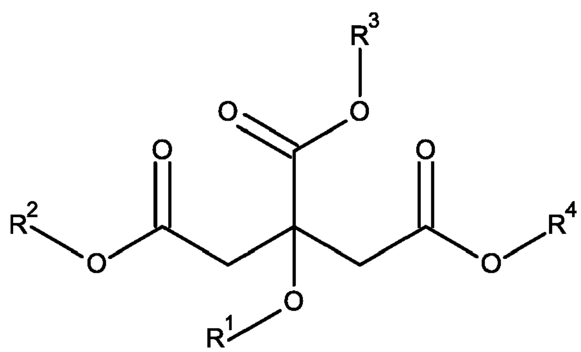

(I)

wherein the variables in formula (I) are defined as follows:

$R^1$ is selected from H and $C_1$-$C_{10}$ alkylcarbonyl, wherein alkyl may be linear or branched and may bear one or more hydroxyl groups,

$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H. Examples of linear $C_1$-$C_8$ alkyl are methyl, ethyl, n-propyl, n-butyl, n-pentyl, etc. Examples of branched $C_3$-$C_8$ alkyl re 2-propyl, 2-butyl, sec.-butyl, tert.-butyl, 2-pentyl, 3-pentyl, iso-pentyl, etc. Examples of $C_6$-$C_{10}$-aryl, non-substituted or substituted with one or more carboxylate or hydroxyl groups, are phenyl, 1-naphthyl, 2-naphthyl, ortho-phenylcarboxylic acid group, meta-phenylcarboxylic acid group, para-phenylcarboxylic acid group, ortho-hydroxyphenyl, para-hydroxyphenyl, etc.

In one embodiment, $R^1$ in the compound according to formula (I) is selected from H, acetyl and propionyl.

In one embodiment, $R^2$ in the compound according to formula (I) is H, and $R^3$, $R^4$ are independently from each other selected from linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl.

In one embodiment, $R^2$, $R^3$, $R^4$ in the compound according to formula (I) are the same, wherein $R^2$, $R^3$, $R^4$ are selected from linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-arylalkyl, wherein alkyl of the latter is selected from linear $C_1$-$C_8$ alkyl or branched $C_3$-$C_8$ alkyl.

In one embodiment, $R^1$ in the compound according to formula (I) is H, and $R^2$, $R^3$, $R^4$ are selected from linear

$C_2$-$C_4$ alkyl, phenylmethyl, and ortho-phenylcarboxylic acid group (salicyl).

In one embodiment, $R^1$, $R^2$ and $R^3$ in the compound according to formula (I) are H, and $R^4$ is selected from linear $C_2$-$C_4$ alkyl, preferably $C_2$ alkyl. In one embodiment, $R^1$, and $R^2$ in the compound according to formula (I) are H, and $R^3$ and $R^4$ are selected from linear $C_2$-$C_4$ alkyl, preferably $C_2$ alkyl.

In one embodiment, $R^1$ in the compound according to formula (I) is acetyl, and $R^2$, $R^3$, $R^4$ are selected from linear $C_2$-$C_4$ alkyl, preferably $C_2$ and $C_4$ alkyl.

Component (a) includes salts of the compound according to formula (I). Salts include alkali metal and ammonium salts e.g those of mono- and triethanolamine. Preference is given to potassium salts and sodium salts.

In one embodiment of the present invention, enzyme preparations, preferably liquid enzyme preparations, comprise component (a) in amounts in the range of 0.1% to 30% by weight, relative to the total weight of the enzyme preparation. The enzyme preparation may comprise component (a) in amounts in the range of 0.1% to 15% by weight, 0.25% to 10% by weight, 0.5% to 10% by weight, 0.5% to 6% by weight, or 1 % to 3% by weight, all relative to the total weight of the enzyme preparation.

In one embodiment of the present invention, compound (a) comprises at least one at least partially hydrolyzed derivative of compound (a) as impurity. In one embodiment of the present invention, component (a) comprises as an impurity of a fully hydrolyzed compound (a') which is as follows:

$R^3$-OH

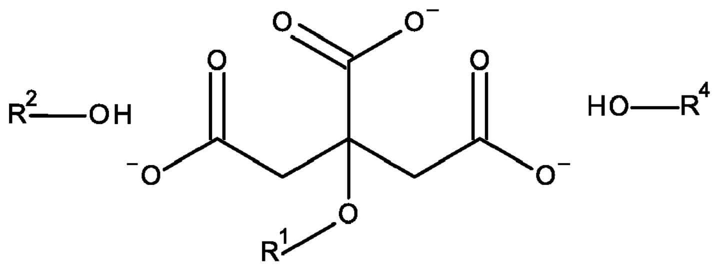

(II)

wherein the variables $R^1$, $R^2$, $R^3$, and $R^4$ are the same as described for component (a) above.

Such impurity may amount to up to 50 mol-%, preferably 0.1 to 20 mol-%, even more preferably 1 to 10 mol-% of component (a). Although the impurities may originate from the synthesis of component (a) and may be removed by purification methods it is not preferred to remove it.

**Component (b)**

In one aspect of the invention, at least one enzyme comprised in component (b) is part of a liquid enzyme concentrate. "Liquid enzyme concentrate" herein means any liquid enzyme-comprising product comprising at least one enzyme. "Liquid" in the context of enzyme concentrate is related to the physical appearance at 20°C and 101.3 kPa.

The liquid enzyme concentrate may result from dissolution of solid enzyme in solvent. The solvent may be selected from water and an organic solvent. A liquid enzyme concentrate resulting from dissolution of solid enzyme in solvent may comprise amounts of enzyme up to the saturation concentration.

Dissolution herein means, that solid compounds are liquified by contact with at least one solvent. Dissolution means complete dissolution of a solid compound until the saturation concentration is achieved in a specified solvent, wherein no phase-separation occurs.

In one aspect of the invention, component (b) of the resulting enzyme concentrate may be free of water, meaning that no significant amounts of water are present. Non-significant amounts of water herein means, that the enzyme preparation comprises less than 25%, less than 20%, less than 15%, less than 10%, less than 7%, less than 5%, less than 4%, less than 3%, less than 2% by weight water, all relative to the total weight of the enzyme concentrate, or no water. In one embodiment, enzyme concentrate free of water free of water means that the enzyme concentrate does not comprise significant amounts of water but does comprise organic solvents in amounts of 30-80% by weight, relative to the total weight of the enzyme concentrate.

Liquid enzyme concentrates comprising water may be called "aqueous enzyme concentrates". Aqueous enzyme concentrates may be enzyme-comprising solutions, wherein solid enzyme product has been dissolved in water. In one embodiment "aqueous enzyme concentrate" means enzyme-comprising products resulting from enzyme production by fermentation.

Fermentation means the process of cultivating recombinant cells which express the desired enzyme in a suitable nutrient medium allowing the recombinant host cells to grow (this process may be called fermentation) and express the desired protein. At the end of the fermentation, fermentation broth usually is collected and further processed, wherein the fermentation broth comprises a liquid fraction and a solid fraction. Depending on whether the enzyme has been secreted into the liquid fraction or not, the desired protein or enzyme may be recovered from the liquid fraction of the fermentation broth or from cell lysates. Recovery of the desired enzyme uses methods known to those skilled in the art. Suitable methods for recovery of proteins or enzymes from fermentation broth include but are not limited to collection, centrifugation, filtration, extraction, and precipitation.

Liquid enzyme concentrates, may comprise amounts of enzyme in the range of 0.1% to 40% by weight, or 0.5% to 30% by weight, or 1% to 25% by weight, or 3% to 25% by weight, or 5% to 25% by weight, all relative to the total weight of the enzyme concentrate. In one embodiment, liquid enzyme concentrates are resulting from fermentation and are aqueous.

Aqueous enzyme concentrates resulting from fermentation may comprise water in amounts of more than about 50% by weight, more than about 60% by weight, more than about 70% by weight, or more than about 80% by weight, all relative to the total weight of the enzyme concentrate. Aqueous enzyme concentrates which result from fermentation, may comprise residual components such as salts originating from the fermentation medium, cell debris originating from the production host cells, metabolites produced by the production host cells during fermentation. In one embodiment, residual components may be comprised in liquid enzyme concentrates in amounts less than 30% by weight, less than 20% by weight less, than 10% by weight, or less than 5% by weight, all relative to the total weight of the aqueous enzyme concentrate.

At least one enzyme comprised in component (b) is selected from hydrolases (EC 3), hereinafter also referred to as enzyme (component (b)). Preferred enzymes are selected from the group of enzymes acting on ester bond (E.C. 3.1), glycosylases (E.C. 3.2), and peptidases (E.C. 3.4). Enzymes acting on ester bond (E.C. 3.1), are hereinafter also referred to as lipases. Glycosylases (E.C. 3.2) are hereinafter also referred to as either amylases, cellulases, or mannanases. Peptidases are hereinafter also referred to as proteases.

Hydrolases comprised in component (b) are identified by polypeptide sequences (also called amino acid sequences herein). The polypeptide sequence specifies the three-dimensional structure including the "active site" of an enzyme which in turn determines the catalytic activity of the same. Polypeptide sequences may be identified by a SEQ ID NO. According to the World Intellectual Property Office (WIPO) Standard ST.25 (1998) the amino acids herein are represented using three-letter code with the first letter as a capital or the corresponding one letter.

Any enzyme comprised in component (b) according to the invention relates to parent enzymes and/or variant enzymes, both having enzymatic activity. Enzymes having enzymatic activity are enzymatically active or exert enzymatic conversion, meaning that enzymes act on substrates and convert these into products. The term "enzyme" herein excludes inactive variants of an enzyme.

A "parent" sequence (of a parent protein or enzyme, also called "parent enzyme") is the starting sequence for introduction of changes (e.g. by introducing one or more amino acid substitutions, insertions, deletions, or a combination thereof) to the sequence, resulting in "variants" of the parent sequences. The term parent enzyme (or parent sequence) includes wild-type enzymes (sequences) and synthetically generated sequences (enzymes) which are used as starting sequences for introduction of (further) changes.

The term "enzyme variant" or "sequence variant" or "variant enzyme" refers to an enzyme that differs from its parent enzyme in its amino acid sequence to a certain extent. If not indicated otherwise, variant enzyme "having enzymatic activity" means that this variant enzyme has the same type of enzymatic activity as the respective parent enzyme.

In describing the variants of the present invention, the nomenclature described as follows is used:
Amino acid substitutions are described by providing the original amino acid of the parent enzyme followed by the number of the position within the amino acid sequence, followed by the substituted amino acid.

Amino acid deletions are described by providing the original amino acid of the parent enzyme followed by the number of the position within the amino acid sequence, followed by *.

Amino acid insertions are described by providing the original amino acid of the parent enzyme followed by the number of the position within the amino acid sequence, followed by the original amino acid and the additional amino acid. For example, an insertion at position 180 of lysine next to glycine is designated as "Gly180GlyLys" or "G180GK".

In cases where a substitution and an insertion occur at the same position, this may be indicated as S99SD+S99A or in short S99AD. In cases where an amino acid residue identical to the existing amino acid residue is inserted, it is clear that degeneracy in the nomenclature arises. If for example a glycine is inserted after the glycine in the above example this would be indicated by G180GG.

Where different alterations can be introduced at a position, the different alterations are separated by a comma, e.g. "Arg170Tyr, Glu" represents a substitution of arginine at position 170 with tyrosine or glutamic acid. Alternatively different alterations or optional substitutions may be indicated in brackets e.g. Arg170[Tyr, Gly] or Arg170{Tyr, Gly}; or in short R170 [Y,G] or R170 {Y, G}; or in long R170Y, R170G.

Enzyme variants may be defined by their sequence identity when compared to a parent enzyme. Sequence identity usually is provided as "% sequence identity" or "% identity". For calculation of sequence identities, in a first step a sequence alignment has to be produced. According to this invention, a pairwise global alignment has to be produced, meaning that two sequences have to be aligned over their complete length, which is usually produced by using a mathematical approach, called alignment algorithm.

According to the invention, the alignment is generated by using the algorithm of Needleman and Wunsch (J. Mol. Biol. (1979) 48, p. 443-453). Preferably, the program "NEEDLE" (The European Molecular Biology Open Software Suite (EMBOSS)) is used for the purposes of the current invention, with using the programs default parameter (gap open=10.0, gap extend=0.5 and matrix=EBLOSUM62).

According to this invention, the following calculation of %-identity applies: %-identity = (identical residues / length of the alignment region which is showing the respective sequence of this invention over its complete length) *100.

According to this invention, enzyme variants may be described as an amino acid sequence which is at least n% identical to the amino acid sequence of the respective parent enzyme with "n" being an integer between 10 and 100. In one embodiment, variant enzymes are at least 70%, at least 75%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% identical when compared to the full-length amino acid sequence of the parent enzyme, wherein the enzyme variant has enzymatic activity.

Enzyme variants may be defined by their sequence similarity when compared to a parent enzyme. Sequence similarity usually is provided as "% sequence similarity" or "%-similarity". % sequence similarity takes into account that defined sets of amino acids share similar properties, e.g by their size, by their hydrophobicity, by their charge, or by other characteristics. Herein, the exchange of one amino acid with a similar amino acid may be called "conservative mutation".

For determination of %-similarity according to this invention the following applies: amino acid A is similar to amino acids S; amino acid D is similar to amino acids E and N; amino acid E is similar to amino acids D and K and Q; amino acid F is similar to amino acids W and Y; amino acid H is similar to amino acids N and Y; amino acid I is similar to amino acids L and M and V; amino acid K is similar to amino acids E and Q and R; amino acid L is similar to amino acids I and M and V; amino acid M is similar to amino acids I and L and V; amino acid N is similar to amino acids D and H and S; amino acid Q is similar to amino acids E and K and R; amino acid R is similar to amino acids K and Q; amino acid S is similar to amino acids A and N and T; amino acid T is similar to amino acids S; amino acid V is similar to amino acids I and L and M; amino acid W is similar to amino acids F and Y; amino acid Y is similar to amino acids F and H and W.

Conservative amino acid substitutions may occur over the full-length of the sequence of a polypeptide sequence of a functional protein such as an enzyme. In one embodiment, such mutations are not pertaining the functional domains of an enzyme. In one embodiment, conservative mutations are not pertaining the catalytic centers of an enzyme.

To take conservative mutations into account, a value for sequence similarity of two amino acid sequences may be calculated from the same alignment, which is used to calculate %-identity.

According to this invention, the following calculation of %-similarity applies: %-similarity = [ (identical residues + similar residues) / length of the alignment region which is showing the respective sequence(s) of this invention over its complete length]*100.

According to this invention, enzyme variants may be described as an amino acid sequence which is at least m% similar to the respective parent sequences with "m" being an integer between 10 and 100. In one embodiment, variant enzymes are at least 70%, at least 75%, at least 80%, at least 85%, at least 90%, at least 91%, at least 92%, at least 93%, at least 94%, at least 95%, at least 96%, at least 97%, at least 98%, or at least 99% similar when compared to the full-length polypeptide sequence of the parent enzyme, wherein the variant enzyme has enzymatic activity.

"Enzymatic activity" means the catalytic effect exerted by an enzyme, which usually is expressed as units per milligram of enzyme (specific activity) which relates to molecules of substrate transformed per minute per molecule of enzyme (molecular activity).

Variant enzymes may have enzymatic activity according to the present invention when said enzyme variants exhibit at least 20%, at least 25%, at least 30%, at least 35%, at least 40%, at least 45%, at 10 least 50%, at least 55%, at least 60%, at least 65%, at least 70%, at least 75%, at least 80%, at least 85%, at least 90%, at least 95%, or 100% of the enzymatic activity of the respective parent enzyme.

Component (b) preferably comprises at least one hydrolase selected from the group of amylases.

**Amylase**

"Amylases" according to the invention (alpha and/or beta) include those of bacterial or fungal origin (EC 3.2.1.1 and 3.2.1.2, respectively). Preferably, component (b) comprises at least one enzyme selected from the group of alpha-amylases (EC 3.2.1.1). Chemically modified or protein engineered mutants are included.

Amylases comprised in component (b) according to the invention have "amylolytic activity" or "amylase activity" involving (endo)hydrolysis of glucosidic linkages in polysaccharides. alpha-amylase activity may be determined by assays for measurement of alpha-amylase activity which are known to those skilled in the art. Examples for assays measuring alpha-amylase activity are:

alpha-amylase activity can be determined by a method employing Phadebas tablets as substrate (Phadebas Amylase Test, supplied by Magle Life Science). Starch is hydrolyzed by the alpha-amylase giving soluble blue fragments. The absorbance of the resulting blue solution, measured spectrophotometrically at 620 nm, is a function of the alpha-amylase activity. The measured absorbance is directly proportional to the specific activity (activity/mg of pure alpha-amylase protein) of the alpha-amylase in question under the given set of conditions.

alpha-amylase activity can also be determined by a method employing the Ethyliden-4-nitrophenyl-alpha-D-maltoheptaosid (EPS). D-maltoheptaoside is a blocked oligosaccharide which can be cleaved by an endo-amylase. Following the cleavage, the alpha-glucosidase included in the kit to digest the substrate to liberate a free PNP molecule which has a yellow color and thus can be measured by visible spectophotometry at 405nm. Kits containing EPS substrate and alpha-glucosidase is manufactured by Roche Costum Biotech (cat. No. 10880078103). The slope of the time dependent absorption-curve is directly proportional to the specific activity (activity per mg enzyme) of the alpha-amylase in question under the given set of conditions.

Amylolytic activity may be provided in units per gram enzyme. For example, 1 unit alpha-amylase may liberate 1.0 mg of maltose from starch in 3 min at pH 6.9 at 20°C.

At least one amylase comprised in component (b) may be selected from the following:

- amylases from *Bacillus licheniformis* having SEQ ID NO:2 as described in WO 95/10603. Suitable variants are described in WO 95/10603 comprising one or more substitutions in the following positions: 15, 23, 105, 106, 124, 128, 133, 154, 156, 178, 179, 181, 188, 190, 197, 201, 202, 207, 208, 209, 211, 243, 264, 304, 305, 391, 408, and 444 which have amylolytic activity. Variants are described in WO 94/02597, WO 94/018314, WO 97/043424 and SEQ ID NO:4 of WO 99/019467.
- amylases from *B. stearothermophilus* having SEQ ID NO:6 as disclosed in WO 02/10355 or an amylase with optionally having a C-terminal truncation over the wildtype sequence. Suitable variants of SEQ ID NO:6 include those comprising a deletion in positions 181 and/or 182 and/or a substitution in position 193.
- amylases from *Bacillus sp.707* having SEQ ID NO:6 as disclosed in WO 99/19467. Preferred variants of SEQ NO: 6 are those having a substitution, a deletion or an insertion in one or more of the following positions: R181, G182, H183, G184, N195, I206, E212, E216 and K269.
- amylases from *Bacillus halmapalus* having SEQ ID NO:2 or SEQ ID NO:7 as described in WO 96/23872, also described herein as SP-722. Preferred variants are described in WO 97/3296, WO 99/194671 and WO 2013/001078.
- amylases from *Bacillus sp.* DSM 12649 having SEQ ID NO:4 as disclosed in WO 00/22103.
- amylases from *Bacillus* strain TS-23 having SEQ ID NO:2 as disclosed in WO 2009/061380.
- amylases from *Cytophaga sp.* having SEQ ID NO:1 as disclosed in WO 2013/184577.
- amylases from *Bacillus megaterium* DSM 90 having SEQ ID NO:1 as disclosed in WO 2010/104675.
- amylases from *Bacillus sp.* comprising amino acids 1 to 485 of SEQ ID NO:2 as described in WO 00/60060.
- amylases from *Bacillus amyloliquefaciens* or variants thereof, preferably selected from amylases according to SEQ ID NO: 3 as described in WO 2016/092009.
- amylases having SEQ ID NO:12 as described in WO 2006/002643 or amylase variants comprising the substitutions Y295F and M202LITV within said SEQ ID NO:12.
- amylases having SEQ ID NO:6 as described in WO 2011/098531 or amylase variants comprising a substitution at one or more positions selected from the group consisting of 193 [G,A,S,T or M], 195 [F,W,Y,L,I or V], 197 [F,W,Y,L,I or V], 198 [Q or N], 200 [F,W,Y,L,I or V], 203 [F,W,Y,L,I or V], 206 [F,W,Y,N,L,I,V,H,Q,D or E], 210 [F,W,Y,L,I or V], 212 [F,W,Y,L,I or V], 213 [G,A,S,T or M] and 243 [F,W,Y,L,I or V] within said SEQ ID NO:6.
- amylases having SEQ ID NO:1 as described in WO 2013/001078 or amylase variants comprising an alteration at two or more (several) positions corresponding to positions G304, W140, W189, D134, E260, F262, W284, W347, W439, W469, G476, and G477 within said SEQ ID NO:1.
- amylases having SEQ ID NO:2 as described in WO 2013/001087 or amylase variants comprising a deletion of positions 181+182, or 182+183, or 183+184, within said SEQ ID NO:2, optionally comprising one or two or more modifications in any of positions corresponding to W140, W159, W167, Q169, W189, E194, N260, F262, W284, F289, G304, G305, R320, W347, W439, W469, G476 and G477 within said SEQ ID NO:2.
- amylases which are hybrid alpha-amylases from above mentioned amylases as for example as described in WO 2006/066594;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

Suitable amylases comprised in component (b) include amylase variants of the amylases disclosed herein having amylase activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% identical when compared to the full-length polypeptide sequence of the parent enzyme as disclosed above.

Suitable amylases comprised in component (b) include amylase variants of the amylases disclosed herein having amylase activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% similar when compared to the full-length polypeptide sequence of the parent enzyme.

In one embodiment, at least one amylase is selected from commercially available amylases which include but are not limited to products sold under the trade names Duramyl™, Termamyl™, Fungamyl™, Stainzyme™, Stainzyme Plus™, Natalase™, Liquozyme X and BAN™, Amplify™, Amplify Prime™ (from Novozymes A/S), and Rapidase™, Purastar™, Powerase™, Effectenz™ (M100 from DuPont), Preferenz™ (S1000, S110 and F1000; from DuPont), Prima-Green™ (ALL; DuPont), Optisize™ (DuPont).

According to the present invention, component (b) may comprise a combination of at least two amylases as disclosed above, wherein said combination comprises one or more amylases selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

In one embodiment, component (b) comprises a combination of at least one amylase, preferably selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those

having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

and at least one further enzyme preferably selected from proteases, lipases, cellulases, and mannanases.

**Protease**

At least one enzyme comprised in component (b) may be selected from the group of proteases, preferably selected from the group of serine endopeptidases (EC 3.4.21), most preferably selected from the group of subtilisin type proteases (EC 3.4.21.62).

Proteases are members of class EC 3.4. Proteases include aminopeptidases (EC 3.4.11), dipeptidases (EC 3.4.13), dipeptidyl-peptidases and tripeptidyl-peptidases (EC 3.4.14), peptidyl-dipeptidases (EC 3.4.15), serine-type carboxypeptidases (EC 3.4.16), metallocarboxypeptidases (EC 3.4.17), cysteine-type carboxypeptidases (EC 3.4.18), omega peptidases (EC 3.4.19), serine endopeptidases (EC 3.4.21), cysteine endopeptidases (EC 3.4.22), aspartic endopeptidases (EC 3.4.23), metallo-endopeptidases (EC 3.4.24), threonine endopeptidases (EC 3.4.25), or endopeptidases of unknown catalytic mechanism (EC 3.4.99).

In one embodiment, component (b) comprises at least one protease selected from serine proteases (EC 3.4.21). Serine proteases or serine peptidases are characterized by having a serine in the catalytically active site, which forms a covalent adduct with the substrate during the catalytic reaction. A serine protease in the context of the present invention may be selected from the group consisting of chymotrypsin (e.g., EC 3.4.21.1), elastase (e.g., EC 3.4.21.36), elastase (e.g., EC 3.4.21.37 or EC 3.4.21.71), granzyme (e.g., EC 3.4.21.78 or EC 3.4.21.79), kallikrein (e.g., EC 3.4.21.34, EC 3.4.21.35, EC 3.4.21.118, or EC 3.4.21.119,) plasmin (e.g., EC 3.4.21.7), trypsin (e.g., EC 3.4.21.4), thrombin (e.g., EC 3.4.21.5), and subtilisin. Subtilisin is also known as subtilopeptidase, e.g., EC 3.4.21.62, the latter hereinafter also being referred to as "subtilisin".

A sub-group of the serine proteases tentatively designated as subtilases has been proposed by Siezen et al. (1991), Protein Eng. 4:719-737 and Siezen et al. (1997), Protein Science 6:501-523. Subtilases includes the subtilisin family, thermitase family, the proteinase K family, the lantibiotic peptidase family, the kexin family and the pyrolysin family.

A subgroup of the subtilases are the subtilisins which are serine proteases from the family S8 as defined by the MEROPS database (http://merops.sanger.ac.uk). Peptidase family S8 comprises the serine endopeptidase subtilisin and its homologues. In subfamily S8A, the active site residues frequently occur in the motifs Asp-Thr/Ser-Gly (which is similar to the sequence motif in families of aspartic endopeptidases in clan AA), His-Gly-Thr-His and Gly-Thr-Ser-Met-Ala-Xaa-Pro.

The subtilisin related class of serine proteases shares a common amino acid sequence defining a catalytic triad which distinguishes them from the chymotrypsin related class of serine proteases. Subtilisins and chymotrypsin related serine proteases both have a catalytic triad comprising aspartate, histidine and serine.

Examples include the subtilisins as described in WO 89/06276 and EP 0283075, WO 89/06279, WO 89/09830, WO 89/09819, WO 91/06637 and WO 91/02792.

Proteases are active proteins exerting "protease activity" or "proteolytic activity". Proteolytic activity is related to the rate of degradation of protein by a protease or proteolytic enzyme in a defined course of time.

The methods for analyzing proteolytic activity are well-known in the literature (see e.g. Gupta et al. (2002), Appl. Microbiol. Biotechnol. 60: 381-395). Proteolytic activity may be determined by using Succinyl-Ala-Ala-Pro-Phe-p-nitroanilide (Suc-AAPF-pNA, short AAPF; see e.g. DelMar et al. (1979), Analytical Biochem 99, 316-320) as substrate. pNA is cleaved from the substrate molecule by proteolytic cleavage, resulting in release of yellow color of free pNA which can be quantified by measuring $OD_{405}$.

Proteolytic activity may be provided in units per gram enzyme. For example, 1 U protease may correspond to the amount of protease which sets free 1 μmol folin-positive amino acids and peptides (as tyrosine) per minute at pH 8.0 and 37°C (casein as substrate).

Proteases of the subtilisin type (EC 3.4.21.62) may be bacterial proteases originating from a microorganism selected from *Bacillus, Clostridium, Enterococcus, Geobacillus, Lactobacillus, Lactococcus, Oceanobacillus, Staphylococcus, Streptococcus,* or *Streptomyces* protease, or a Gram-negative bacterial polypeptide such as a *Campylobacter, E. coli, Flavobacterium, Fusobacterium, Helicobacter, Ilyobacter, Neisseria, Pseudomonas, Salmonella,* and *Ureaplasma.*

In one aspect of the invention, at least one protease is selected from *Bacillus alcalophilus, Bacillus amyloliquefaciens, Bacillus brevis, Bacillus circulans, Bacillus clausii, Bacillus coagulans, Bacillus firmus, Bacillus gibsonii, Bacillus lautus, Bacillus lentus, Bacillus licheniformis, Bacillus megaterium, Bacillus pumilus, Bacillus sphaericus, Bacillus stearothermophilus, Bacillus subtilis,* or *Bacillus thuringiensis* protease.

In one embodiment of the present invention, component (b) comprises at least one protease selected from the following: subtilisin from *Bacillus amyloliquefaciens* BPN' (described by Vasantha et al. (1984) J. Bacteriol. Volume 159, p. 811-819 and JA Wells et al. (1983) in Nucleic Acids Research, Volume 11, p. 7911-7925); subtilisin from *Bacillus licheniformis* (subtilisin Carlsberg; disclosed in EL Smith et al. (1968) in J. Biol Chem, Volume 243, pp. 2184-2191, and Jacobs et al. (1985) in Nucl. Acids Res, Vol 13, p. 8913-8926); subtilisin PB92 (original sequence of the alkaline protease PB92 is described in EP 283075 A2); subtilisin 147 and/or 309 (Esperase®, Savinase®, respectively) as disclosed in WO 89/06279; subtilisin from *Bacillus lentus* as disclosed in WO 91/02792, such as from *Bacillus lentus* DSM 5483 or the variants of *Bacillus lentus* DSM 5483 as described in WO 95/23221; subtilisin from *Bacillus alcalophilus* (DSM 11233) disclosed in DE 10064983; subtilisin from *Bacillus gibsonii* (DSM 14391) as disclosed in WO 2003/054184; subtilisin from *Bacillus sp.* (DSM 14390) disclosed in WO 2003/056017; subtilisin from *Bacillus sp.* (DSM 14392) disclosed in WO 2003/055974; subtilisin from *Bacillus gibsonii* (DSM 14393) disclosed in WO 2003/054184; subtilisin having SEQ ID NO: 4 as described in WO 2005/063974; subtilisin having SEQ ID NO: 4 as described in WO 2005/103244; subtilisin having SEQ ID NO: 7 as described in WO 2005/103244; and subtilisin having SEQ ID NO: 2 as described in application DE 102005028295.4.

In one embodiment, component (b) comprises at least subtilisin 309 (which might be called Savinase herein) as disclosed as sequence a) in Table I of WO 89/06279 or a variant thereof which is at least 80% identical thereto and has proteolytic activity.

Examples of useful proteases in accordance with the present invention comprise the variants described in: WO 92/19729, WO 95/23221, WO 96/34946, WO 98/20115, WO 98/20116, WO 99/11768, WO 01/44452, WO 02/088340, WO 03/006602, WO 2004/03186, WO 2004/041979, WO 2007/006305, WO 2011/036263, WO 2011/036264, and WO 2011/072099. Suitable examples comprise especially variants of subtilisin protease derived from SEQ ID NO:22 as described in EP 1921147 (which is the sequence of mature alkaline protease from *Bacillus lentus* DSM 5483) with amino acid substitutions in one or more of the following positions: 3, 4, 9, 15, 24, 27, 33, 36, 57, 68, 76, 77, 87, 95, 96, 97, 98, 99, 100, 101 , 102, 103, 104, 106, 118, 120, 123, 128, 129, 130, 131, 154, 160, 167, 170, 194, 195, 199, 205, 206, 217, 218, 222, 224, 232, 235, 236, 245, 248, 252 and 274 (according to the BPN' numbering), which have proteolytic activity. In one embodiment, such a protease is not mutated at positions Asp32, His64 and Ser221 (according to BPN' numbering).

Component (b) may comprise a protease variant having proteolytic activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% identical when compared to the full-length polypeptide sequence of the parent enzyme as disclosed above.

Component (b) may comprise a protease variant having proteolytic activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% similar when compared to the full-length polypeptide sequence of the parent enzyme.

In one embodiment, at least one protease comprised in component (b) has SEQ ID NO:22 as described in EP 1921147, or a protease which is at least 80% identical thereto and has proteolytic activity. In one embodiment, said protease is characterized by having amino acid glutamic acid (E), or aspartic acid (D), or asparagine (N), or glutamine (Q), or alanine (A), or glycine (G), or serine (S) at position 101 (according to BPN' numbering) and has proteolytic activity. In one embodiment, said protease comprises one or more further substitutions: (a) threonine at position 3 (3T), (b) isoleucine at position 4 (4I), (c) alanine, threonine or arginine at position 63 (63A, 63T, or 63R), (d) aspartic acid or glutamic acid at position 156 (156D or 156E), (e) proline at position 194 (194P), (f) methionine at position 199 (199M), (g) isoleucine at position 205 (205I), (h) aspartic acid, glutamic acid or glycine at position 217 (217D, 217E or 217G), (i) combinations of two or more amino acids according to (a) to (h). At least one protease may be at least 80% identical to

SEQ ID NO:22 as described in EP 1921147 and is characterized by comprising one amino acid (according to (a)-(h)) or combinations according to (i) together with the amino acid 101E, 101D, 101N, 101Q, 101A, 101G, or 101S (according to BPN' numbering) and having proteolytic activity. In one embodiment, said protease is characterized by comprising the mutation (according to BPN' numbering) R101E, or S3T + V4I + V205I, or R101E and S3T, V4I, and V205I, or S3T + V4I + V199M + V205I + L217D, and having proteolytic activity.

In one embodiment, protease according to SEQ ID NO:22 as described in EP 1921147 is characterized by comprising the mutation (according to BPN' numbering) S3T + V4I + S9R + A15T + V68A + D99S + R101S + A103S + I104V + N218D, and having proteolytic activity.

In one embodiment, at least one protease is selected from commercially available protease enzymes which include but are not limited to products sold under the trade names Alcalase®, Blaze®, Duralase™, Durazym™, Relase®, Relase® Ultra, Savinase®, Savinase® Ultra, Primase®, Polarzyme®, Kannase®, Liquanase®, Liquanase® Ultra, Ovozyme®, Coronase®, Coronase® Ultra, Neutrase®, Everlase® and Esperase® (Novozymes A/S), those sold under the tradename Maxatase®, Maxacal®, Maxapem®, Purafect®, Purafect® Prime, Purafect MA®, Purafect Ox®, Purafect OxP®, Puramax®, Properase®, FN2®, FN3®, FN4®, Excellase®, Eraser®, Ultimase®, Opticlean®, Effectenz®, Preferenz® and Optimase® (Danisco/DuPont), Axapem™ (Gist-Brocases N.V.), *Bacillus lentus* Alkaline Protease (BLAP; sequence shown in Figure 29 of US 5,352,604) and variants thereof and KAP (*Bacillus alkalophilus* subtilisin) from Kao Corp.

According to the present invention, component (b) may comprise a combination of at least two proteases, preferably selected from the group of serine endopeptidases (EC 3.4.21), more preferably selected from the group of subtilisin type proteases (EC 3.4.21.62) - all as disclosed above.

In one embodiment, component (b) comprises at least one protease selected from proteases according to SEQ ID NO:22 as described in EP 1921147 or variants thereof having proteolytic activity, as disclosed above.

In one embodiment, component (b) comprises at least one protease selected from subtilisin 309 as disclosed in Table I a) of WO 89/06279 or variants thereof having proteolytic activity, as disclosed above.

In one embodiment, component (b) comprises a combination of at least one alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity;

and at least one protease as disclosed above, preferably selected from the group of serine endopeptidases (EC 3.4.21), more preferably selected from the group of subtilisin type proteases (EC 3.4.21.62).

In one embodiment, component (b) comprises a combination of at least one alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity;

at least one protease as disclosed above, preferably selected from the group of serine endopeptidases (EC 3.4.21), more preferably selected from the group of subtilisin type proteases (EC 3.4.21.62), and
at least one further enzyme preferably selected from lipase, cellulase, and mannanase.

**Lipase**

At least one enzyme comprised in component (b) may be selected from the group of lipases. "Lipases", "lipolytic enzyme", "lipid esterase", all refer to an enzyme of EC class 3.1.1 ("carboxylic ester hydrolase"). Lipase means active protein having lipase activity (or lipolytic activity; triacylglycerol lipase, EC 3.1.1.3), cutinase activity (EC 3.1.1.74; enzymes having cutinase activity may be called cutinase herein), sterol esterase activity (EC 3.1.1.13) and/or wax-ester hydrolase activity (EC 3.1.1.50).

The methods for determining lipolytic activity are well-known in the literature (see e.g. Gupta et al. (2003), Biotechnol. Appl. Biochem. 37, p. 63-71). E.g. the lipase activity may be measured by ester bond hydrolysis in the substrate para-nitrophenyl palmitate (pNP-Palmitate, C:16) and releases pNP which is yellow and can be detected at 405 nm.

"Lipolytic activity" means the catalytic effect exerted by a lipase, which may be provided in lipolytic units (LU). For example, 1LU may correspond to the amount of lipase which produces 1 $\mu$mol of titratable fatty acid per minute in a pH stat. under the following conditions: temperature 30°C.; pH=9.0; substrate may be an emulsion of 3.3 wt.% of olive oil and 3.3% gum arabic, in the presence of 13 mmol/l $Ca^{2+}$ and 20 mmol/l NaCl in 5 mmol/l Tris-buffer.

Lipases which may be comprised in component (b) include those of bacterial or fungal origin. In one aspect of the invention, a suitable lipase (component (b)) is selected from the following: lipases from *Humicola* (synonym *Thermomyces*), e.g. from *H. lanuginosa* (*T. lanuginosus*) as described in EP 258068, EP 305216, WO 92/05249 and WO 2009/109500 or from *H. insolens* as described in WO 96/13580; lipases derived from *Rhizomucor miehei* as described in WO 92/05249; lipase from strains of *Pseudomonas* (some of these now renamed to *Burkholderia*), e.g. from *P. alcaligenes* or *P. pseudoalcaligenes* (EP 218272, WO 94/25578, WO 95/30744, WO 95/35381, WO 96/00292), *P. cepacia* (EP 331376), *P. stutzeri* (GB 1372034), *P. fluorescens, Pseudomonas sp.* strain SD705 (WO 95/06720 and WO 96/27002), *P. wisconsinensis* (WO 96/12012), *Pseudomonas mendocina* (WO 95/14783), *P. glumae* (WO 95/35381, WO 96/00292); lipase from *Streptomyces griseus* (WO 2011/150157) and *S. pristinaespiralis* (WO 2012/137147), GDSL-type *Streptomyces* lipases (WO 2010/065455); lipase from *Thermobifida fusca* as disclosed in WO 2011/084412; lipase from *Geobacillus stearothermophilus* as disclosed in WO 2011/084417; *Bacillus* lipases, e.g. as disclosed in WO 00/60063, lipases from *B. subtilis* as disclosed in Dartois et al. (1992), Biochemica et Biophysica Acta, 1131, 253-360 or WO 2011/084599, *B. stearothermophilus* (JP S64-074992) or *B. pumilus* (WO 91/16422); lipase from *Candida antarctica* as disclosed in WO 94/01541; cutinase from *Pseudomonas mendocina* (US 5389536, WO 88/09367); cutinase from *Magnaporthe grisea* (WO 2010/107560); cutinase from *Fusarum solani pisi* as disclosed in WO 90/09446, WO 00/34450 and WO 01/92502; and cutinase from *Humicola lanuginosa* as disclosed in WO 00/34450 and WO 01/92502.

Suitable lipases also include those referred to as acyltransferases or perhydrolases, e.g. acyltransferases with homology to *Candida antarctica* lipase A (WO 2010/111143), acyltransferase from *Mycobacterium smegmatis* (WO

2005/056782), perhydrolases from the CE7 family (WO 2009/67279), and variants of the *M. smegmatis* perhydrolase in particular the S54V variant (WO 2010/100028).

Component (b) may comprise lipase variants of the above described lipases which have lipolytic activity. Such suitable lipase variants are e.g. those which are developed by methods as disclosed in WO 95/22615, WO 97/04079, WO 97/07202, WO 00/60063, WO 2007/087508, EP 407225 and EP 260105.

Component (b) may comprise lipase variants having lipolytic activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% identical when compared to the full-length polypeptide sequence of the parent enzyme as disclosed above.

Component (b) may comprise lipase variants having lipolytic activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% similar when compared to the full-length polypeptide sequence of the parent enzyme as disclosed above.

In one embodiment, component (b) comprises at least one lipase selected from fungal triacylglycerol lipase (EC class 3.1.1.3). Fungal triacylglycerol lipase may be selected from *Thermomyces lanuginose* lipase. In one embodiment, *Thermomyces lanuginosa* lipase is selected from triacylglycerol lipase according to amino acids 1-269 of SEQ ID NO:2 of US 5869438 and variants thereof having lipolytic activity. Triacylglycerol lipase according to amino acids 1-269 of SEQ ID NO:2 of US 5869438 may be called Lipolase herein.

*Thermomyces lanuginosa* lipase may be selected from variants having lipolytic activity which are at least 90%, at least 91%, at least 92%, at least 93%, at least 94%, at least 95%, at least 96%, at least 97%, at least 98% or at least 99% identical when compared to the full-length polypeptide sequence of amino acids 1-269 of SEQ ID NO:2 of US 5869438.

*Thermomyces lanuginosa* lipase may be selected from variants having lipolytic activity comprising conservative mutations only, which do however not pertain the functional domain of amino acids 1-269 of SEQ ID NO:2 of US 5869438. Lipase variants of this embodiment having lipolytic activity may be at least 95%, at least 96%, at least 97%, at least 98% or at least 99% similar when compared to the full-length polypeptide sequence of amino acids 1-269 of SEQ ID NO:2 of US 5869438.

*Thermomyces lanuginosa* lipase may be at least 80% identical to SEQ ID NO:2 of US 5869438 characterized by having amino acid T231R and N233R. Said *Thermomyces lanuginosa* lipase may further comprise one or more of the following amino acid exchanges: Q4V, V60S, A150G, L227G, P256K.

In one embodiment, at least one lipase is selected from commercially available lipases which include but are not limited to products sold under the trade names Lipolase™, Lipex™, Lipolex™ and Lipoclean™ (Novozymes A/S), Lumafast (originally from Genencor) and Lipomax (Gist-Brocades/ now DSM).

According to the present invention, component (b) may comprise a combination of at least two lipases, preferably selected from triacylglycerol lipase according to amino acids 1-269 of SEQ ID NO:2 of US 5869438 and variants thereof having lipolytic activity as disclosed above.

In one embodiment, component (b) comprises a combination of at least one alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO:

2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity;

and at least one lipase as disclosed above, preferably selected from triacylglycerol lipase according to amino acids 1-269 of SEQ ID NO:2 of US 5869438 and variants thereof having lipolytic activity.

In one embodiment, component (b) comprises a combination of at least one alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity;

at least one lipase as disclosed above, preferably selected from triacylglycerol lipase according to amino acids 1-269 of SEQ ID NO:2 of US 5869438 and variants thereof having lipolytic activity;
at least one protease as disclosed above, preferably selected from the group of serine endopeptidases (EC 3.4.21), more preferably selected from the group of subtilisin type proteases (EC 3.4.21.62); and
at least one further enzyme preferably selected from cellulase, and mannanase.

### Cellulase

At least one enzyme comprised in component (b) may be selected from the group of cellulases. At least one cellulase may be selected from cellobiohydrolase (1,4-P-D-glucan cellobiohydrolase, EC 3.2.1.91), endo-ss-1,4-glucanase (endo-1,4-P-D-glucan 4-glucanohydrolase, EC 3.2.1.4) and ss-glucosidase (EC 3.2.1.21). Preferably, component (b) comprises at least one cellulase of the glycosyl hydrolase family 7 (GH7, pfam00840), preferably selected from endoglucanases (EC 3.2.1.4).

"Cellulases", "cellulase enzymes" or "cellulolytic enzymes" (component (b)) are enzymes involved in hydrolysis of cellulose. Assays for measurement of "cellulase activity" or "cellulolytic activity" are known to those skilled in the art. For example, cellulolytic activity may be determined by virtue of the fact that cellulase hydrolyses carboxymethyl cellulose to reducing carbohydrates, the reducing ability of which is determined colorimetrically by means of the ferricyanide reaction, according to Hoffman, W. S., J. Biol. Chem. 120, 51 (1937).

Cellulolytic activity may be provided in units per gram enzyme. For example, 1 unit may liberate 1.0 μmole of glucose from cellulose in one hour at pH 5.0 at 37 °C (2 hour incubation time).

Cellulases according to the invention include those of bacterial or fungal origin. In one embodiment, at least one cellulase is selected from cellulases comprising a cellulose binding domain. In one embodiment, at least one cellulase is selected from cellulases comprising a catalytic domain only, meaning that the cellulase lacks cellulose binding domain.

In one embodiment, at least one cellulase comprised in component (b) is selected from commercially available

cellulases which include but are not limited to Celluzyme™, Endolase™, Carezyme™, Cellusoft™, Renozyme™, Celluclean™ (from Novozymes A/S), Ecostone™, Biotouch™, Econase™, Ecopulp™ (from AB Enzymes Finland), Clazinase™, and Puradax HA™, Genencor detergent cellulase L, IndiAge™ Neutra (from Genencor International Inc./DuPont), Revitalenz™ (2000 from DuPont), Primafast™ (DuPont) and KAC-500™ (from Kao Corporation).

According to the present invention, component (b) may comprise a combination of at least two cellulases, preferably selected from endoglucanases (EC 3.2.1.4) as disclosed above.

In one embodiment, component (b) comprises a combination of at least one alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity;

and at least one cellulase of the GH7 family, preferably selected from endoglucanases (EC 3.2.1.4) as disclosed above.

In one embodiment, component (b) comprises a combination of at least one alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO

2014/183921, wherein the hybrid amylase has amylolytic activity; preferably, the hybrid alpha-amylase is at least 95% identical to SEQ ID NO: 30 as disclosed in WO 2014/183921 and having amylolytic activity;

at least one cellulase of the GH7 family, preferably selected from endoglucanases (EC 3.2.1.4) as disclosed above; and one or more further enzymes, preferably selected from

- lipase as disclosed above, preferably selected from triacylglycerol lipase according to amino acids 1-269 of SEQ ID NO:2 of US 5869438 and variants thereof having lipolytic activity;
- protease as disclosed above, preferably selected from the group of serine endopeptidases (EC 3.4.21), more preferably selected from the group of subtilisin type proteases (EC 3.4.21.62); and
- mannanase.

**Mannan degrading enzyme**

At least one enzyme comprised in component (b) may be selected from the group of mannan degrading enzymes. At least one mannan degrading enzyme may be selected from β-mannosidase (EC 3.2.1.25), endo-1,4-β-mannosidase (EC 3.2.1.78), and 1,4-β-mannobiosidase (EC 3.2.1.100). Preferably, at least one mannan degrading enzyme is selected from the group of endo-1,4-β-mannosidase (EC 3.2.1.78), a group of enzymes which may be called endo-β-1,4-D-mannanase, β-mannanase, or mannanase herein.

A polypeptide having mannanase activity may be tested for mannanase activity according to standard test procedures known in the art, such as by applying a solution to be tested to 4 mm diameter holes punched out in agar plates containing 0.2% AZCL galactomannan (carob), i. e. substrate for the assay of endo-1,4-beta-D-mannanase available as CatNo. I-AZGMA from the company Megazyme (Megazyme's Internet address: http://www. megazyme. com/Purchase/index. html).

Component (b) may comprise at least one mannanase selected from alkaline mannanase of Family 5 or 26. The term "alkaline mannanase" is meant to encompass mannanases having an enzymatic activity of at least 40% of its maximum activity at a given pH ranging from 7 to 12, preferably 7.5 to 10.5.

At least one mannanase comprised in component (b) may be selected from mannanases originating from Bacillus organisms, such as described in JP-0304706 [beta-mannanase from Bacillus sp.], JP-63056289 [alkaline, thermostable beta-mannanase], JP-63036774 [Bacillus microorganism FERM P-8856 producing beta-mannanase and beta-mannosidase at an alkaline pH], JP-08051975 [alkaline beta-mannanases from alkalophilic Bacillus sp. AM-001], WO 97/11164 [mannanase from Bacillus amyloliquefaciens], WO 91/18974 [mannanase active at an extreme pH and temperature], WO 97/11164 [mannanase from Bacillus amyloliquefaciens], WO2014100018 [endo-(3-mannanase1 cloned from a *Bacillus circulans* or *Bacillus lentus* strain CMG1240 (Bleman1; see U.S. 5,476,775)]. Suitable mannanases are described in WO 99/064619].

At least one mannanase comprised in component (b) may be selected from mannanases originating from Trichoderma organisms, such as disclosed in WO 93/24622 or in WO 2011/085747.

Component (b) may comprise mannanase variants having mannanase activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% identical when compared to the full-length polypeptide sequence of the corresponding parent enzyme as disclosed above.

Component (b) may comprise mannanase variants having mannanase activity which are at least 40%, at least 45%, at least 50%, at least 55%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98% or at least 99% similar when compared to the full-length polypeptide sequence of the corresponding parent enzyme as disclosed above.

Component (b) may comprise a commercially available mannanase such as Mannaway® (Novozymes AIS).

In one embodiment, component (b) comprises a combination of at least one alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity;

and at least one alkaline mannanase, preferably selected from the group of endo-1,4-β-mannosidase (EC 3.2.1.78) as disclosed above.

In one embodiment, component (b) comprises a combination of at least one alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity;

at least one alkaline mannanase, preferably selected from the group of endo-1,4-β-mannosidase (EC 3.2.1.78) as disclosed above;
and one or more further enzymes, preferably selected from

- cellulase as disclosed above, preferably of the GH7 family, more preferably selected from endoglucanases (EC 3.2.1.4) as disclosed above;

- lipase as disclosed above, preferably selected from triacylglycerol lipase according to amino acids 1-269 of SEQ ID NO:2 of US 5869438 and variants thereof having lipolytic activity; and

- protease as disclosed above, preferably selected from the group of serine endopeptidases (EC 3.4.21), more preferably selected from the group of subtilisin type proteases (EC 3.4.21.62).

**Component (c)**

In one embodiment, the liquid enzyme preparation of the invention comprises component (c) which comprises at least one compound selected from solvents, enzyme stabilizers different from component (a), and compounds stabilizing the liquid enzyme preparation as such.

**Enzyme stabilizers different from component (a):**

The liquid enzyme preparation of the invention may comprise at least one enzyme stabilizer different from component (a). Said enzyme stabilizer (component (c)) may be selected from boron-containing compounds, polyols, peptide aldehydes, other stabilizers, and mixtures thereof.

*Boron-containing compounds:*

Boron-containing compounds (component (c)) may be selected from boric acid or its derivatives and from boronic acid or its derivatives such as aryl boronic acids or its derivatives, from salts thereof, and from mixtures thereof. Boric acid herein may be called orthoboric acid.

In one embodiment, boron-containing compound (component (c)) is selected from the group consisting of aryl boronic acids and its derivatives. In one embodiment, boron-containing compound is selected from the group consisting of benzene boronic acid (BBA) which is also called phenyl boronic acid (PBA), derivatives thereof, and mixtures thereof. In one embodiment, phenyl boronic acid derivatives are selected from the group consisting of the derivatives of formula (IIIa) and (IIIb) formula:

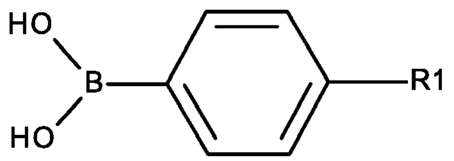
(IIIa)

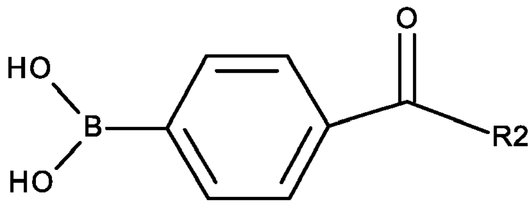
(IIIb)

wherein

R1 is selected from the group consisting of hydrogen, hydroxy, non-substituted or substituted $C_1$-$C_6$ alkyl, and non-substituted or substituted $C_1$-$C_6$ alkenyl; in a preferred embodiment, R is selected from the group consisting of hydroxy, and non-substituted $C_1$ alkyl;

R2 is selected from the group consisting of hydrogen, hydroxy, non-substituted or substituted $C_1$-$C_6$ alkyl, and non-substituted or substituted $C_1$-$C_6$ alkenyl; in a preferred embodiment, R is selected from the group consisting of H, hydroxy, and substituted $C_1$ alkyl.

In one embodiment phenyl-boronic acid derivatives (component (c)) are selected from the group consisting of 4-formyl phenyl boronic acid (4-FPBA), 4-carboxy phenyl boronic acid (4-CPBA), 4-(hydroxymethyl) phenyl boronic acid (4-HMPBA), and p-tolylboronic acid (p-TBA).

Other suitable derivatives (component (c)) include: 2-thienyl boronic acid, 3-thienyl boronic acid, (2-acetamidophenyl) boronic acid, 2-benzofuranyl boronic acid, 1-naphthyl boronic acid, 2-naphthyl boronic acid, 2-FPBA, 3-FBPA, 1-thianthrenyl boronic acid, 4-dibenzofuran boronic acid, 5-methyl-2-thienyl boronic acid, 1-benzothiophene-2 boronic acid, 2-furanyl boronic acid, 3-furanyl boronic acid, 4,4 biphenyl-diboronic acid, 6-hydroxy-2-naphthaleneboronic acid, 4-(methylthio) phenyl boronic acid, 4-(trimethylsilyl) phenyl boronic acid, 3-bromothiophene boronic acid, 4-methylthiophene boronic acid, 2-naphthyl boronic acid, 5-bromothiophene boronic acid, 5-chlorothiophene boronic acid, dimethylthiophene boronic acid, 2-bromophenyl boronic acid, 3-chlorophenyl boronic acid, 3-methoxy-2-thiophene boronic acid, p-methyl-phenylethyl boronic acid, 2-thianthrenyl boronic acid, di-benzothiophene boronic acid, 9-anthracene boronic acid, 3,5 dichlorophenyl boronic, acid, diphenyl boronic acid anhydride, o-chlorophenyl boronic acid, p-chlorophenyl boronic acid, m-bromophenyl boronic acid, p-bromophenyl boronic acid, p-fluorophenyl boronic acid, octyl boronic acid, 1,3,5 trimethylphenyl boronic acid, 3-chloro-4-fluorophenyl boronic acid, 3-aminophenyl boronic acid, 3,5-bis-(trifluoromethyl) phenyl boronic acid, 2,4 dichlorophenyl boronic acid, 4-methoxyphenyl boronic acid, and mixtures thereof.

*Polyols:*

Polyols (component (c)) may be selected from polyols containing from 2 to 6 hydroxyl groups. Suitable examples include glycol, propylene glycol, 1,2-propane diol, 1,2-butane diol, ethylene glycol, hexylene glycol, glycerol, sorbitol, mannitol, erythriol, glucose, fructose, lactore, and erythritan.

*Peptide aldehydes:*

Peptide aldehydes (component (c)) may be selected from di-, tri- or tetrapeptide aldehydes and aldehyde analogues (either of the form B1-BO-R wherein, R is H, $CH_3$, $CX_3$, $CHX_2$, or $CH_2X$ (X=halogen), BO is a single amino acid residue (in one embodiment with an optionally substituted aliphatic or aromatic side chain); and B1 consists of one or more amino acid residues (in one embodiment one, two or three), optionally comprising an N-terminal protection group, or as described in WO 09/118375 and WO 98/13459, or a protease inhibitor of the protein type such as RASI, BASI, WASI (bifunctional alpha-amylase/subtilisin inhibitors of rice, barley and wheat) or $CI_2$ or SSI.

*Other stabilizers:*

Other stabilizers (component (c)) may be selected from salts like NaCl or KCl, and alkali salts of lactic acid and formic acid.

Other stabilizers (component (c)) may be selected from water-soluble sources of zinc (II), calcium (II) and/or magnesium (II) ions in the finished compositions that provide such ions to the enzymes, as well as other metal ions (e.g. barium (II), scandium (II), iron (II), manganese (II), aluminum (III), Tin (II), cobalt (II), copper (II), Nickel (II), and oxovanadium (IV)).

**Compounds stabilizing the liquid enzyme preparation as such**

Compounds stabilizing the liquid enzyme preparation as such means any compound except enzyme stabilizers needed to establish storage stability of a liquid preparation in amounts effective to ensure the storage stability.

Storage stability in the context of liquid preparations to those skilled in the art usually includes aspects of appearance of the product and uniformity of dosage.

Appearance of the product is influenced by the pH of the product and by the presence of compounds such as preservatives, antioxidants, viscosity modifiers, emulsifiers etc.

Uniformity of dosage is usually related to the homogeneity of a product.

Inventive enzyme preparations may be alkaline or exhibit a neutral or slightly acidic pH value, for example 6 to 14, 6.5 to 13, 8 to 10.5, or 8.5 to 9.0.

The liquid enzyme preparation of the invention may comprise at least one preservative. Preservatives are added in amounts effective in preventing microbial contamination of the liquid enzyme preparation, preferably the aqueous enzyme preparation.

Non-limiting examples of suitable preservatives include (quaternary) ammonium compounds, isothiazolinones, organic acids, and formaldehyde releasing agents. Non-limiting examples of suitable (quaternary) ammonium compounds include benzalkonium chlorides, polyhexamethylene biguanide (PHMB), Didecyldimethylammonium chloride(DDAC), and N-(3-aminopropyl)-N-dodecylpropane-1,3-diamine (Diamine). Non-limiting examples of suitable isothiazolinones include 1,2-benzisothiazolin-3-one (BIT), 2-methyl-2H-isothiazol-3-one (MIT), 5-chloro-2-methyl-2H-isothiazol-3-one (CIT), 2-octyl-2H-isothiazol-3-one (OIT), and 2-butyl-benzo[d]isothiazol-3-one (BBIT). Non-limiting examples of suitable organic acids include benzoic acid, sorbic acid, L-(+)-lactic acid, formic acid, and salicylic acid. Non-limiting examples of suitable formaldehyde releasing agent include N,N'-methylenebismorpholine (MBM), 2,2',2''-(hexahydro-1,3,5-triazine-1,3,5- triyl)triethanol (HHT), (ethylenedioxy)dimethanol, .alpha.,.alpha.',.alpha.''-trimethyl-1,3,5-triazine-1,3,5(2H,4H,6H)-triethanol (HPT), 3,3'-methylenebis[5-methyloxazolidine] (MBO), and cis-1-(3-chloroallyl)-3,5,7-triaza-1- azoniaadamantane chloride (CTAC).

Further useful preservatives include iodopropynyl butylcarbamate (IPBC), halogen releasing compounds such as dichloro-dimethyl-hydantoine (DCDMH), bromo-chloro-dimethyl-hydantoine (BCDMH), and dibromo-dimethyl-hydantoine (DBDMH); bromo-nitro compounds such as Bronopol (2-bromo-2-nitropropane-1,3-diol), 2,2-dibromo-2-cyanoacetamide (DBNPA); aldehydes such as glutaraldehyde; phenoxyethanol; Biphenyl-2-ol; and zinc or sodium pyrithione.

**Solvents**

In one embodiment, the inventive enzyme preparation is aqueous, comprising water in amounts in the range of 5% to 95 % by weight, in the range of 5% to 30% by weight, in the range of 5% to 25% by weight, or in the range of 20% to 70% by weight, all relative to the total weight of the enzyme preparation.

In one embodiment, the enzyme preparation of the invention comprises at least one organic solvent selected from ethanol, n-propanol, iso-propanol, n-butanol, iso-butanol, sec.-butanol, ethylene glycol, propylene glycol, 1,3-propane diol, butane diol, glycerol, diglycol, propyl diglycol, butyl diglycol, hexylene glycol, ethylene glycol methyl ether, ethylene glycol ethyl ether, ethylene glycol propyl ether, and phenoxyethanol, preferred are ethanol, isopropanol or propylene glycol. Further, the enzyme preparation of the invention may comprise at least one organic solvent selected from compounds such as 2-butoxyethanol, isopropyl alcohol, and d-limonene.

Said enzyme preparation may comprise organic solvents in amounts in the range of 0% to 20% by weight relative to the total weight of the enzyme preparation. In one embodiment, the enzyme preparation comprises water in amounts in the range of 5% to 15% by weight and no significant amounts of organic solvent, for example 1% by weight or less, all relative to the total weight of the enzyme preparation.

In one embodiment, the enzyme preparation of the invention comprises at least

component (a): at least one enzyme stabilizer selected from compounds according to general formula (I)

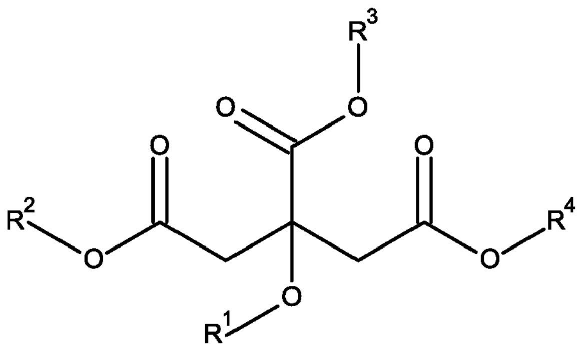

(I)

wherein the variables in formula (I) are as follows:

$R^1$ is selected from H and $C_1$-$C_{10}$ alkylcarbonyl, wherein alkyl may be linear or branched and may bear one or more hydroxyl groups,

$R^2$, $R^3$, $R^4$ are independently from each other selected from H, linear $C_1$-$C_5$ alkyl, and branched $C_3$-$C_{10}$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H, and

component (b): at least one enzyme selected from the group of hydrolases (EC 3), preferably at least one enzyme selected from the group of amylases, more preferably at least one enzyme selected from the group of alpha-amylases (EC 3.2.1.1); and/or at least one enzyme is selected from proteases, preferably from subtilisin type proteases (EC 3.4.21.62);

and

component (c): at least one enzyme stabilizer different from component (a) as disclosed above, preferably selected from polyols, peptide aldehydes and other stabilizers as disclosed above;

wherein at least one amylase is selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity;

and wherein at least one protease is selected from the group of subtilisin type proteases (EC 3.4.21.62), preferably from

- a protease according to SEQ ID NO:22 as described in EP 1921147 or variants thereof having proteolytic activity; and

- a protease selected from subtilisin 309 as disclosed in Table I a) of WO 89/06279 or variants thereof having proteolytic activity.

**Preparation of enzyme preparation:**

The invention relates to a process for making an enzyme preparation, said process comprising the step of mixing at least component (a) as disclosed above and component (b) as disclosed above.

In one embodiment the invention relates to a process for making an enzyme preparation, said process comprising the step of mixing components (a), (b), and (c) as disclosed above, wherein component (b) preferably comprises at least one amylase; and optionally at least one enzyme selected from the group of proteases, lipases, cellulases, and mannanases. The amylase is preferably selected from the group of alpha-amylases (EC 3.2.1.1) as disclosed above, more preferably at least one amylase is selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

In one embodiment component (c) comprises at least one solvent as disclosed above. In one embodiment, component (c) comprises at least one enzyme stabilizer different from component (a) as disclosed above.

Component (b) may be solid. Solid component (b) may be added to solid component (a) prior to contact of both with at least one solvent (component (c)). At least one solvent is as disclosed above. Contact with at least one solvent (component (c)) may result in solubilizing of at least one molecule component (a) and at least one molecule component (b), resulting in stabilization of at least one molecule component (b). In one embodiment, solid components (a) and (b) are completely dissolved in at least one solvent (component (c)) without phase separation.

Solid component (a) may be dissolved in at least one solvent (component (c)) prior to mixing with solid or liquid component (b). In one embodiment, component (a) is completely dissolved in at least one solvent (component (c)) prior to mixing with component (b). At least one solvent is as disclosed above.

Component (b) may be liquid, wherein at least one enzyme may be comprised in a liquid enzyme concentrate as disclosed above. Liquid component (b) may be supplemented with solid component (a), wherein solid component (a) dissolves in liquid component (b). In one embodiment, liquid component (b) is aqueous, preferably resulting from fermentation. In one embodiment, when solid component (a) dissolves in liquid component (b), no additional solvent may be added.

In one embodiment, component (c) as disclosed above is mixed with components (a) and (b), wherein the mixing is characterized in being done in one or more steps.

## Enzyme stabilization

The invention relates to a method of stabilizing at least one hydrolase comprised in component (b) by the step of adding component (a), wherein components (a) and (b) are those disclosed above. In one embodiment, component (b) is liquid. In one embodiment, the invention relates to a method of stabilizing component (b) by the step of adding component (a), wherein component (b) comprises at least one amylase and/or at least one protease.

At least one amylase may be selected from alpha-amylases (EC 3.2.1.1) as disclosed above, more preferably at least one amylase is selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

At least one protease may be selected from the group of subtilisin type proteases (EC 3.4.21.62), preferably from

- a protease according to SEQ ID NO:22 as described in EP 1921147 or variants thereof having proteolytic activity; and
- a protease selected from subtilisin 309 as disclosed in Table I a) of WO 89/06279 or variants thereof having proteolytic activity.

In one embodiment, the invention relates to a method of stabilizing component (b) by the step of adding component (a) and at least one enzyme stabilizer different from component (a) (component (c)) as disclosed above, preferably selected from polyols, peptide aldehydes, and other stabilizers as disclosed above.

In one embodiment, the invention relates to a method of stabilizing component (b) in the presence of at least one surfactant by the step of adding component (a) and optionally at least one enzyme stabilizer different from component (a) as disclosed above, wherein components (a) and (b) are those disclosed above and at least one surfactant is selected from non-ionic surfactants, amphoteric surfactants, anionic surfactants, and cationic surfactants, all as described below. In one embodiment, liquid formulations are detergent formulations.

In one embodiment, the invention relates to the use of a compound according to formula (I) - component (a) as disclosed above:

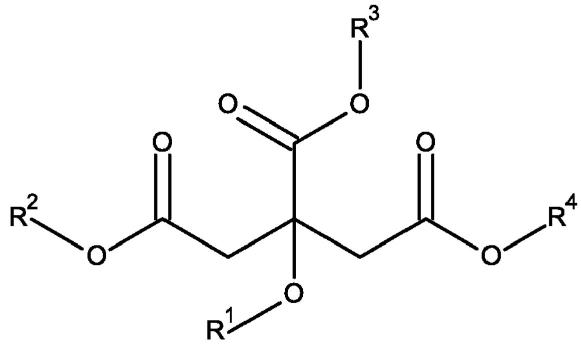

(I)

wherein the variables in formula (I) are defined as follows:

$R^1$ is selected from H and $C_1$-$C_{10}$ alkylcarbonyl, wherein alkyl may be linear or branched and may bear one or more hydroxyl groups;

$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H

as additive for at least one hydrolase (component (b)), wherein the compound according to formula (I) and the hydrolase are solid, and wherein enzymatic activity of the hydrolase is stabilized when the compound according to formula (I) and the hydrolase are contacted with at least one solvent [component (c)].

Contact with at least one solvent (component (c)) may result in solubilizing of at least one molecule component (a) and at least one molecule component (b), resulting in stabilization of at least one molecule component (b). In one embodiment, solid components (a) and (b) are completely dissolved in at least one solvent (component (c)) without phase separation.

In one embodiment of the present invention, component (a) is added in amounts in the range of 0.1% to 30% by weight, relative to the total weight of the enzyme preparation. The enzyme preparation may comprise component (a) in amounts in the range of 0.1% to 15% by weight, 0.25% to 10% by weight, 0.5% to 10% by weight, 0.5% to 6% by weight, or 1 % to 3% by weight, all relative to the total weight of the enzyme preparation.

In one embodiment, at least one enzyme stabilizer different from component (a) is added in amounts effective to reversibly inhibit the proteolytic activity of at least one protease comprised in component (b).

In one embodiment, said compound according to formula (I) (component (a)) is used as an additive for component (b), wherein component (b) comprises at least one amylase preferably selected from alpha-amylases (EC 3.2.1.1) as disclosed above, wherein the compound according to formula (I) and the amylase are solid, and wherein amylolytic activity of the amylase is stabilized when the compound according to formula (I) and the amylase are contacted with at least one solvent [component (c)]. The amylase may be selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

  - hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

  - hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

In one embodiment, component (b) comprises at least one amylase and at least one protease, wherein at least one protease may be selected from the group of subtilisin type proteases (EC 3.4.21.62), preferably from

- a protease according to SEQ ID NO:22 as described in EP 1921147 or variants thereof having proteolytic activity; and

- a protease selected from subtilisin 309 as disclosed in Table I a) of WO 89/06279 or variants thereof having proteolytic activity.

In one embodiment, said compound according to formula (I) (component (a)) is used as an additive for compositions comprising component (b) and at least one complexing agent selected from EDTA, DTPA, MGDA and GLDA as disclosed herein. Component (b) in this context comprises at least one amylase preferably selected from alpha-amylases (EC 3.2.1.1) as disclosed above, wherein the compound according to formula (I) and the amylase are solid, and wherein amylolytic activity of the amylase is stabilized when the compound according to formula (I) and the amylase are contacted with at least one solvent [component (c)]. The amylase may be selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

In one embodiment, component (b) comprises at least one amylase and at least one protease, wherein at least

one protease may be selected from the group of subtilisin type proteases (EC 3.4.21.62), preferably from

- a protease according to SEQ ID NO:22 as described in EP 1921147 or variants thereof having proteolytic activity; and
- a protease selected from subtilisin 309 as disclosed in Table I a) of WO 89/06279 or variants thereof having proteolytic activity.

In one embodiment, said compound according to formula (I) is used together with at least one enzyme stabilizer different from component (a), as additive for component (b), wherein component (b) comprises at least one amylase preferably selected from alpha-amylase(EC 3.2.1.1) as disclosed above, wherein the compound according to formula (I), the enzyme stabilizer different from component (a) and the amylase are solid, and wherein amylolytic activity of the amylase is stabilized when the solid components are contacted with at least one solvent [component (c)]. The amylase may be selected

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

In one embodiment, component (b) comprises at least one amylase and at least one further enzyme selected from

- protease selected from the group of serine endopeptidases (EC 3.4.21), preferably selected from the group of subtilisin type proteases (EC 3.4.21.62), more preferably selected from proteases according to SEQ ID NO:22 as described in EP 1921147 or variants thereof having proteolytic activity as disclosed above and from subtilisin 309 as disclosed in Table I a) of WO 89/06279 or variants thereof having proteolytic activity as disclosed above, and

- lipase selected from the group of triacylglycerol lipase, preferably from triacylglycerol lipase according to amino acids 1-269 of SEQ ID NO:2 of US 5869438 or a variant thereof having lipolytic activity as disclosed above

and optionally at least one enzyme stabilizer different from component (a), preferably selected from boron-containing compounds, polyols, peptide aldehydes and other stabilizers as disclosed above,
wherein all components are solid, and wherein amylolytic activity of the amylase and/or proteolytic activity of the protease and/or the lipolytic activity of the lipase are stabilized when the components are contacted with at least one solvent [component (c)].

Stabilization of an enzyme may relate to stability in the course of time (e.g. storage stability), thermal stability, pH stability, and chemical stability. The term "enzyme stability" herein preferably relates to the retention of enzymatic activity as a function of time e.g. during storage or operation. The term "storage" herein means to indicate the fact of products or compositions being stored from the time of being manufactured to the point in time of being used in final application. Retention of enzymatic activity as a function of time during storage is called "storage stability". In one

embodiment, storage means storage for at least 20 days at 37°C. Storage may mean storage for 21, 28, or 42 days at 37°C.

To determine changes in enzymatic activity over time, the "initial enzymatic activity" of an enzyme may be measured under defined conditions at time zero (i.e. before storage) and the "enzymatic activity after storage" may be measured at a certain point in time later (i.e. after storage).

The enzymatic activity after storage divided by the initial enzymatic activity multiplied by 100 gives the "residual enzymatic activity" (a%).

An enzyme is stable according to the invention, when its residual enzymatic activity is at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% or 100% when compared to the initial enzymatic activity before storage.

Subtracting a% from 100% gives the "loss of enzymatic activity during storage" when compared to the initial enzymatic activity before storage. In one embodiment, an enzyme is stable according to the invention when essentially no loss of enzymatic activity occurs during storage, i.e. loss in enzymatic activity equals 0% when compared to the initial enzymatic activity before storage. Essentially no loss of enzymatic activity within this invention may mean that the loss of enzymatic activity is less than 30%, less than 25%, less than 20%, less than 15%, less than 10%, less than 9%, less than 8%, less than 7%, less than 6%, less than 5%, less than 4%, less than 3%, less than 2%, or less than 1% when compared to the initial enzymatic activity before storage.

In one aspect of the invention component (a) is used to reduce loss of enzymatic activity during storage of component (b). Calculation of % reduced loss of enzymatic activity is done as follows: (% loss of enzymatic activity of stabilized enzyme) - (% loss of enzymatic activity of non-stabilized enzyme). The value for reduced loss indicates the reduced loss of enzymatic activity of at least one enzyme comprised in component (b) in the presence of component (a) when compared to the loss of enzymatic activity of the same enzyme(s) in the absence of component (a) at a certain point in time.

Reduced loss of enzymatic activity within this invention may mean that the loss of enzymatic activity is reduced in the presence of component (a) by at least 5%, by at least 10%, by at least 15%, by at least 20%, by at least 25%, by at least 30%, by at least 40%, by at least 50%, by least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 99.5%, when compared to the loss of enzymatic activity in the absence of component (a).

In one aspect, the invention relates to a method of reducing loss of amylolytic activity of at least one amylase preferably selected from alpha-amylases comprised in component (b) which is comprised in a liquid during storage by the step of adding a compound according to formula (I):

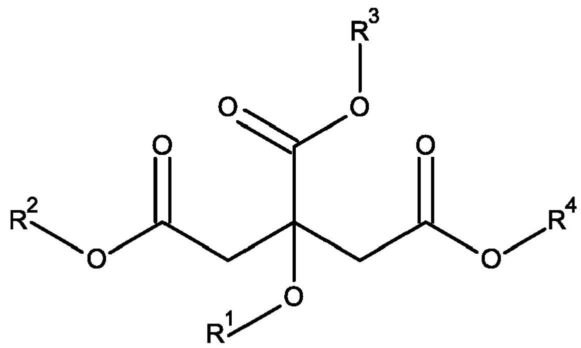

(I)

wherein the variables in formula (I) are defined as follows:

$R^1$ is selected from H and $C_1$-$C_{10}$ alkylcarbonyl, wherein alkyl may be linear or branched and may bear one or more hydroxyl groups;

$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H.

In one embodiment, the method of reducing loss of amylolytic activity of at least one amylase (component (b)) comprised in a liquid during storage comprises the step of adding a compound according to formula (I) and the step of

adding at least one enzyme stabilizer different from component (a), preferably selected from polyols, peptide aldehydes and other stabilizers as disclosed above.

In one embodiment, the amylase (component (b)) is comprised in a liquid enzyme preparation, or the amylase is comprised in a liquid composition comprising at least one surfactant such as a liquid detergent formulation, preferably further comprising at least one complexing agent selected from EDTA, DTPA, MGDA and GLDA as disclosed herein. The amylase may be selected from alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity.In one embodiment, component (b) comprises at least one amylase preferably selected from alpha-amylase (EC 3.2.1.1) as disclosed above, preferably selected from

In one embodiment, component (b) comprises at least one amylase and at least one protease selected from the group of serine endopeptidases (EC 3.4.21), preferably selected from the group of subtilisin type proteases (EC 3.4.21.62), more preferably selected from proteases according to SEQ ID NO:22 as described in EP 1921147 or variants thereof having proteolytic activity as disclosed above and from subtilisin 309 as disclosed in Table I a) of WO 89/06279 or variants thereof having proteolytic activity as disclosed above.

In one aspect of the invention, component (a) stabilizes at least one amylase comprised in component (b). At least one amylase comprised in component (b), preferably selected from alpha-amylase (EC 3.2.1.1) as disclosed above. In one embodiment, component (a) is used to stabilize amylase [component (b)] within a liquid enzyme preparation. In one embodiment, component (a) is used to stabilize amylase [component (b)] within a liquid composition comprising at least one surfactant, preferably within a liquid detergent composition. Stabilization in this context may mean stabilization during storage at 37°C for 21, 28 and/or 42 days.

In one embodiment, the addition of component (a) to component (b) stabilizes amylase during storage, wherein stabilization is characterized by

(a) residual amylolytic activity after storage at 37°C for 21 days being ≥60%, ≥70%, ≥80%, or ≥90% when compared to the initial amylolytic activity before storage and/or

(b) residual amylolytic activity after storage at 37°C for 28 days being ≥55%, ≥60%, ≥70%, or ≥80% when compared to the initial amylolytic activity before storage and/or

(c) residual amylolytic activity after storage at 37°C for 42 days being ≥35%, ≥45%, ≥50%, or ≥60% when compared to the initial amylolytic activity before storage.

The addition of component (a) to component (b) may stabilize amylase during storage preferably in the presence of a complexing agent such as EDTA and/or DTPA and/or MGDA and/or GLDA, wherein component (a) is preferably

comprised in amounts in the range of 1% to 5% by weight, more preferably in the range of 1.5% to 2% by weight, both relative to the total weight of the composition, and/or wherein amylase is preferably comprised in amounts in the range of 0.2% to 2% by weight, more preferably in about 0.5% by weight, both relative to the total weight of the composition, and/or wherein optionally

- EDTA and/or DTPA and/or
- MGDA and/or GLDA are comprised in amounts in the range of 10% to 30% by weight, preferably in the range of 15% to 25%, all relative to the total weight of the composition. MGDA (methyl glycine diacetic acid) and GLDA (glutamic acid diacetic acid) which are known as sequestrants for alkaline earth metal ions such as $Ca^{2+}$ and $Mg^{2+}$, are those as disclosed herein below.

In one embodiment, the addition of component (a) to component (b) stabilizes amylase during storage, wherein component (a) is characterized by $R^1$ in the compound according to formula (I) is H, and $R^2$, $R^3$, $R^4$ are selected from linear $C_2$-$C_4$ alkyl, and wherein stabilization is characterized by

(a) residual amylolytic activity after storage at 37°C for 21 days being ≥75%, ≥80%, or ≥85% when compared to the initial amylolytic activity before storage and/or

(b) residual amylolytic activity after storage at 37°C for 28 days being ≥65%, ≥70%, or ≥80% when compared to the initial amylolytic activity before storage and/or

(c) residual amylolytic activity after storage at 37°C for 42 days being ≥55%, ≥60%, or ≥65% when compared to the initial amylolytic activity before storage.

The addition of component (a) to component (b) may stabilize amylase during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from linear $C_2$-$C_4$ alkyl, and
wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and wherein stabilization is characterized by

(a) residual amylolytic activity after storage at 37°C for 21 days being ≥65%, ≥70%, or ≥80% when compared to the initial amylolytic activity before storage and/or

(b) residual amylolytic activity after storage at 37°C for 28 days being ≥55%, ≥65%, or ≥70% when compared to the initial amylolytic activity before storage and/or

(c) residual amylolytic activity after storage at 37°C for 42 days being ≥35%, ≥50% or ≥60% when compared to the initial amylolytic activity before storage.

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and
wherein component (a) is preferably comprised in amounts in the range of 1% to 5% by weight, more preferably in the range of 1.5% to 2% by weight, both relative to the total weight of the composition, and/or

wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and wherein stabilization is characterized by

(a) residual amylolytic activity after storage at 37°C for 21 days being ≥60%, ≥70%, or ≥75% when compared to the initial amylolytic activity before storage and/or

(b) residual amylolytic activity after storage at 37°C for 28 days being ≥55%, ≥60%, or ≥65% when compared to the initial amylolytic activity before storage and/or

(c) residual amylolytic activity after storage at 37°C for 42 days being ≥45%, ≥50%, or ≥60% when compared to the initial amylolytic activity before storage.

The addition of component (a) to component (b) may stabilize protease during storage in the presence of a complexing agent such as EDTA and/or DTPA and/or MGDA and/or GLDA,
wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and
wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ in the compound according to formula (I) is H, and $R^2$, $R^3$, $R^4$ are selected from phenylmethyl, and salicyl, and wherein stabilization is characterized by

(a) residual amylolytic activity after storage at 37°C for 21 days being ≥65%, ≥75%, or ≥80% when compared to the initial amylolytic activity before storage and/or

(b) residual amylolytic activity after storage at 37°C for 28 days being ≥60%, ≥65%, ≥70%, or ≥80% when compared to the initial amylolytic activity before storage and/or

(c) residual amylolytic activity after storage at 37°C for 42 days being ≥55%, ≥60%, or ≥70% when compared to the initial amylolytic activity before storage.

The addition of component (a) to component (b) may stabilize amylase during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from phenylmethyl, and salicyl, and wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein stabilization is characterized by

(a) loss of amylolytic activity during storage at 37°C for 21 days being ≤35%, ≤30% or ≤25% when compared to the initial amylolytic activity before storage and/or

(b) loss of amylolytic activity during storage at 37°C for 28 days being ≤40%, ≤35% or ≤30% when compared to the initial amylolytic activity before storage and/or

(c) loss of amylolytic activity during storage at 37°C for 42 days being ≤60%, ≤50%, or ≤45% when compared to the initial amylolytic activity before storage.

The addition of component (a) to component (b) may stabilize amylase during storage preferably in the presence of a complexing agent such as EDTA and/or DTPA and/or MGDA and/or GLDA, wherein component (a) is preferably comprised in amounts in the range of 1% to 5% by weight, more preferably in the range of 1.5% to 2% by weight, both relative to the total weight of the composition, and/or wherein amylase is preferably comprised in amounts in the range of 0.2% to 2% by weight, more preferably in about 0.5% by weight, both relative to the total weight of the composition, and/or wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ in the compound according to formula (I) is H, and $R^2$, $R^3$, $R^4$ are selected from linear $C_2$-$C_4$ alkyl, and wherein stabilization is characterized by

(a) loss of amylolytic activity during storage at 37°C for 21 days being ≤20%, or ≤5% when compared to the initial amylolytic activity before storage and/or

(b) loss of amylolytic activity during storage at 37°C for 28 days being ≤31%, or ≤25% when compared to the initial amylolytic activity before storage and/or

(c) loss of amylolytic activity during storage at 37°C for 42 days being ≤43%, or ≤35%, when compared to the initial amylolytic activity before storage.

The addition of component (a) to component (b) may stabilize amylase during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from linear $C_2$-$C_4$ alkyl, and wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ in the compound according to formula (I) is acetyl, and $R^2$, $R^3$, $R^4$ are selected

from linear $C_2$-$C_4$ alkyl, preferably $C_2$ and $C_4$ alkyl, and wherein stabilization is characterized by

(a) loss of amylolytic activity during storage at 37°C for 21 days being ≤32%, or ≤5% when compared to the initial amylolytic activity before storage and/or

(b) loss of amylolytic activity during storage at 37°C for 28 days being ≤40%, or ≤25% when compared to the initial amylolytic activity before storage and/or

(c) loss of amylolytic activity during storage at 37°C for 42 days being ≤60%, ≤50%, or ≤40% when compared to the initial amylolytic activity before storage.

The addition of component (a) to component (b) may stabilize amylase during storage preferably in the presence of a complexing agent such as EDTA and/or DTPA and/or MGDA and/or GLDA, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and
wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and wherein stabilization is characterized by

(a) loss of amylolytic activity during storage at 37°C for 21 days being ≤34%, ≤27% or ≤5% when compared to the initial amylolytic activity before storage and/or

(b) loss of amylolytic activity during storage at 37°C for 28 days being ≤42%, or ≤36 when compared to the initial amylolytic activity before storage and/or

(c) loss of amylolytic activity during storage at 37°C for 42 days being ≤50%, or ≤46% when compared to the initial amylolytic activity before storage.

The addition of component (a) to component (b) may stabilize amylase during storage preferably in the presence of a complexing agent such as EDTA and/or DTPA and/or MGDA and/or GLDA, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and
wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ in the compound according to formula (I) is H, and $R^2$, $R^3$, $R^4$ are selected from phenylmethyl, and salicyl, and wherein stabilization is characterized by

(a) loss of amylolytic activity during storage at 37°C for 21 days being ≤31%, ≤20%, ≤15% or ≤5% when compared to the initial amylolytic activity before storage and/or

(b) loss of amylolytic activity during storage at 37°C for 28 days being ≤37%, ≤30%, or ≤25%, or ≤ 20% when compared to the initial amylolytic activity before storage and/or

(c) loss of amylolytic activity during storage at 37°C for 42 days being ≤43%, ≤35%, or ≤30% when compared to the initial amylolytic activity before storage.

The addition of component (a) to component (b) may stabilize amylase during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from phenylmethyl, and salicyl, and wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein stabilization is characterized by

(a) reduced loss of amylolytic activity during storage at 37°C for 21 days being ≥14% when compared to the loss of amylolytic activity in the absence of component (a) and/or

(b) reduced loss of amylolytic activity during storage at 37°C for 28 days being ≥29% when compared to the loss of amylolytic activity in the absence of component (a) and/or

(c) reduced loss of amylolytic activity during storage at 37°C for 42 days being ≥24% when compared to the loss of amylolytic activity in the absence of component (a).

The addition of component (a) to component (b) may stabilize amylase during storage preferably in the presence of a complexing agent such as EDTA and/or DTPA and/or MGDA and/or GLDA, wherein component (a) is preferably comprised in amounts in the range of 1% to 5% by weight, more preferably in the range of 1.5% to 2% by weight, both relative to the total weight of the composition, and/or wherein amylase is preferably comprised in amounts in the range of 0.2% to 2% by weight, more preferably in about 0.5% by weight, both relative to the total weight of the composition, and/or wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ in the compound according to formula (I) is H, and $R^2$, $R^3$, $R^4$ are selected from linear $C_2$-$C_4$ alkyl, and wherein stabilization is characterized by

(a) reduced loss of amylolytic activity during storage at 37°C for 21 days being ≥14%, ≥25%, or ≥40% when compared to the loss of amylolytic activity in the absence of component (a) and/or

(b) reduced loss of amylolytic activity during storage at 37°C for 28 days being ≥40% when compared to the loss of amylolytic activity in the absence of component (a) and/or

(c) reduced loss of amylolytic activity during storage at 37°C for 42 days being ≥37% when compared to the loss of

amylolytic activity in the absence of component (a).

The addition of component (a) to component (b) may stabilize amylase during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from linear $C_2$-$C_4$ alkyl, and wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or

- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and wherein stabilization is characterized by

(a) reduced loss of amylolytic activity during storage at 37°C for 21 days being ≥18%, or ≥30% when compared to the loss of amylolytic activity in the absence of component (a) and/or

(b) reduced loss of amylolytic activity during storage at 37°C for 28 days being ≥33%, or ≥40% when compared to the loss of amylolytic activity in the absence of component (a) and/or

(c) reduced loss of amylolytic activity during storage at 37°C for 42 days being ≥23%, or ≥30% when compared to the loss of amylolytic activity in the absence of component (a).

The addition of component (a) to component (b) may stabilize amylase during storage preferably in the presence of a complexing agent such as EDTA and/or DTPA and/or MGDA and/or GLDA, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and
wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and wherein stabilization is characterized by

(a) reduced loss of amylolytic activity during storage at 37°C for 21 days being ≥16%, or ≥25% when compared to the loss of amylolytic activity in the absence of component (a) and/or

(b) reduced loss of amylolytic activity during storage at 37°C for 28 days being ≥29% when compared to the loss of amylolytic activity in the absence of component (a) and/or

(c) reduced loss of amylolytic activity during storage at 37°C for 42 days being ≥24% when compared to the loss of amylolytic activity in the absence of component (a).

The addition of component (a) to component (b) may stabilize amylase during storage preferably in the presence of a complexing agent such as EDTA and/or DTPA and/or MGDA and/or GLDA, wherein component (a) is characterized

by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and
wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes amylase during storage, wherein component (a) is characterized by $R^1$ in the compound according to formula (I) is H, and $R^2$, $R^3$, $R^4$ are selected from phenylmethyl, and salicyl, and wherein stabilization is characterized by

(a) reduced loss of amylolytic activity during storage at 37°C for 21 days being ≥29%, or ≥40% when compared to the loss of amylolytic activity in the absence of component (a) and/or

(b) reduced loss of amylolytic activity during storage at 37°C for 28 days being ≥34%, or ≥40% when compared to the loss of amylolytic activity in the absence of component (a) and/or

(c) reduced loss of amylolytic activity during storage at 37°C for 42 days being ≥38% when compared to the loss of amylolytic activity in the absence of component (a).

The addition of component (a) to component (b) may stabilize amylase during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from phenylmethyl, and salicyl, and wherein component (a) is preferably comprised in amounts in the range of 1% to 5% by weight, more preferably in the range of 1.5% to 2% by weight, both relative to the total weight of the composition, and/or
wherein amylase is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In embodiments of the above embodiments, component (a) as disclosed above is used to stabilize amylase [component (b)] within a liquid enzyme preparation. Further, in embodiments of the above embodiments the amylase which is stabilized by component (a) is alpha-amylase (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2

of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

In one aspect of the invention, component (a) is used to stabilize component (b) comprising at least one amylase, preferably alpha-amylase (EC 3.2.1.1) as disclosed above, and at least one protease, preferably selected from serine endopeptidases (EC 3.4.21), more preferably selected from the group of subtilisin type proteases (EC 3.4.21.62), within a liquid composition preferably comprising at least one surfactant and/or at least one complexing agent selected from EDTA, DTPA, MGDA and GLDA as disclosed herein, wherein at least one amylase is selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity;

and wherein at least one protease is selected from the group of subtilisin type proteases (EC 3.4.21.62); preferably selected from protease according to SEQ ID NO:22 as described in EP 1921147 or variants thereof having proteolytic activity as disclosed above and from subtilisin 309 as disclosed in Table I a) of WO 89/06279 or variants thereof having proteolytic activity as disclosed above.

In one aspect of the invention, component (a) stabilizes at least one protease comprised in component (b). At least one protease comprised in component (b), preferably selected from subtilisin type proteases (EC 3.4.21.62) as disclosed above. In one embodiment, component (a) is used to stabilize protease [component (b)] within a liquid enzyme preparation. In one embodiment, component (a) is used to stabilize protease [component (b)] within a liquid composition comprising at least one surfactant and/or at least one complexing agent selected from EDTA, DTPA, MGDA and GLDA as disclosed herein. Stabilization in this context may mean stabilization during storage at 37°C for 14, 21, 28 and/or 42 days.

In one embodiment, the addition of component (a) to component (b) stabilizes protease during storage, wherein stabilization is characterized by

(a) residual proteolytic activity after storage at 37°C for 21 days being ≥70%, or ≥80% when compared to the initial proteolytic activity before storage and/or

(b) residual proteolytic activity after storage at 37°C for 28 days being ≥65%, ≥70%, or ≥75% when compared to

the initial proteolytic activity before storage and/or

(c) residual proteolytic activity after storage at 37°C for 42 days being ≥55%, ≥60%, or ≥65% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is preferably comprised in amounts in the range of 1% to 5% by weight, more preferably in the range of 1.5% to 2% by weight, both relative to the total weight of the composition, and/or wherein protease is preferably comprised in amounts in the range of 0.2% to 2% by weight, more preferably in about 0.5% by weight, both relative to the total weight of the composition, and/or wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition. MGDA (methyl glycine diacetic acid) and GLDA (glutamic acid diacetic acid) which are known as sequestrants for alkaline earth metal ions such as $Ca^{2+}$ and $Mg^{2+}$, are those as disclosed herein below.

In one embodiment, the addition of component (a) to component (b) stabilizes protease during storage, wherein component (a) is characterized by $R^1$ in the compound according to formula (I) is H, and $R^2$, $R^3$, $R^4$ are selected from linear $C_2$-$C_4$ alkyl, and wherein stabilization is characterized by

(a) residual proteolytic activity after storage at 37°C for 21 days being ≥75%, or ≥80% when compared to the initial proteolytic activity before storage and/or

(b) residual proteolytic activity after storage at 37°C for 28 days being ≥70%, or ≥75% when compared to the initial proteolytic activity before storage and/or

(c) residual proteolytic activity after storage at 37°C for 42 days being ≥65% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from linear $C_2$-$C_4$ alkyl, and wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and wherein stabilization is characterized by

(a) residual proteolytic activity after storage at 37°C for 21 days being ≥75%, or ≥80% when compared to the initial proteolytic activity before storage and/or

(b) residual proteolytic activity after storage at 37°C for 28 days being ≥70%, or ≥75% when compared to the initial proteolytic activity before storage and/or

(c) residual proteolytic activity after storage at 37°C for 42 days being ≥60%, or ≥65% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and
wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1/R^2$ or $R^4$, and wherein stabilization is characterized by

(a) residual proteolytic activity after storage at 37°C for 21 days being ≥70%, or ≥75% when compared to the initial proteolytic activity before storage and/or

(b) residual proteolytic activity after storage at 37°C for 28 days being ≥65%, or ≥70% when compared to the initial proteolytic activity before storage and/or

(c) residual proteolytic activity after storage at 37°C for 42 days being ≥55%, or ≥60% when compared to the initial proteolytic activity before storage.

The addition of component (a) to component (b) may stabilize protease during storage in the presence of a complexing agent such as EDTA and/or DTPA and/or MGDA and/or GLDA,
wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1/R^2$ or $R^4$, and
wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ in the compound according to formula (I) is H, and $R^2$, $R^3$, $R^4$ are selected from phenylmethyl, and salicyl, and wherein stabilization is characterized by

(a) residual proteolytic activity after storage at 37°C for 21 days being ≥75%, or ≥80% when compared to the initial proteolytic activity before storage and/or

(b) residual proteolytic activity after storage at 37°C for 28 days being ≥70%, or ≥75% when compared to the initial proteolytic activity before storage and/or

(c) residual proteolytic activity after storage at 37°C for 42 days being ≥60%, or ≥65% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is characterized

by $R^1$ in the compound according to formula (I) is H, and $R^2$, $R^3$, $R^4$ are selected from phenylmethyl, and salicyl, and wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein stabilization is characterized by

(a) loss of proteolytic activity during storage at 37°C for 21 days being ≤27%, or ≤20% when compared to the initial proteolytic activity before storage and/or

(b) loss of proteolytic activity during storage at 37°C for 28 days being ≤33%, or ≤23% when compared to the initial proteolytic activity before storage and/or

(c) loss of proteolytic activity during storage at 37°C for 42 days being ≤44%, or ≤38% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is preferably comprised in amounts in the range of 1% to 5% by weight, more preferably in the range of 1.5% to 2% by weight, both relative to the total weight of the composition, and/or wherein protease is preferably comprised in amounts in the range of 0.2% to 2% by weight, more preferably in about 0.5% by weight, both relative to the total weight of the composition, and/or wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ in the compound according to formula (I) is H, and $R^2$, $R^3$, $R^4$ are selected from linear $C_2$-$C_4$ alkyl, and wherein stabilization is characterized by

(a) loss of proteolytic activity during storage at 37°C for 21 days being ≤20% when compared to the initial proteolytic activity before storage and/or

(b) loss of proteolytic activity during storage at 37°C for 28 days being ≤28% when compared to the initial proteolytic activity before storage and/or

(c) loss of proteolytic activity during storage at 37°C for 42 days being ≤33% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from linear $C_2$-$C_4$ alkyl, and wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and
wherein stabilization is characterized by

(a) loss of proteolytic activity during storage at 37°C for 21 days being ≤21 % when compared to the initial proteolytic activity before storage and/or

(b) loss of proteolytic activity during storage at 37°C for 28 days being ≤29%, or ≤25% when compared to the initial proteolytic activity before storage and/or

(c) loss of proteolytic activity during storage at 37°C for 42 days being ≤35% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and
wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and wherein stabilization is characterized by

(a) loss of proteolytic activity during storage at 37°C for 21 days being ≤27%, or ≤23% when compared to the initial proteolytic activity before storage and/or

(b) loss of proteolytic activity during storage at 37°C for 28 days being ≤33% when compared to the initial proteolytic activity before storage and/or

(c) loss of proteolytic activity during storage at 37°C for 42 days being ≤45%, or ≤40% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and
wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or

- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ in the compound according to formula (I) is H, and $R^2$, $R^3$, $R^4$ are selected from phenylmethyl, and salicyl, and wherein stabilization is characterized by

(a) loss of proteolytic activity during storage at 37°C for 21 days being ≤21 %, or ≤15% when compared to the initial proteolytic activity before storage and/or

(b) loss of proteolytic activity during storage at 37°C for 28 days being ≤30%, or ≤25%, when compared to the initial proteolytic activity before storage and/or

(c) loss of proteolytic activity during storage at 37°C for 42 days being ≤36%, or ≤30% when compared to the initial proteolytic activity before storage.

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from phenylmethyl, and salicyl, and wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein stabilization is characterized by

(a) reduced loss of proteolytic activity during storage at 37°C for 21 days being ≥5%, or ≥10% when compared to the loss of proteolytic activity in the absence of component (a) and/or

(b) reduced loss of proteolytic activity during storage at 37°C for 28 days being ≥5%, or ≥10% when compared to the loss of proteolytic activity in the absence of component (a) and/or

(c) reduced loss of proteolytic activity during storage at 37°C for 42 days being ≥5%, or ≥10% when compared to the loss of proteolytic activity in the absence of component (a).

The addition of component (a) to component (b) may stabilize protease during storage preferably in the presence of a complexing agent such as EDTA and/or DTPA and/or MGDA and/or GLDA, wherein component (a) is preferably comprised in amounts in the range of 1% to 5% by weight, more preferably in the range of 1.5% to 2% by weight, both relative to the total weight of the composition, and/or wherein protease is preferably comprised in amounts in the range of 0.2% to 2% by weight, more preferably in about 0.5% by weight, both relative to the total weight of the composition, and/or wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ in the compound according to formula (I) is H, and $R^2$, $R^3$, $R^4$ are selected from linear $C_2$-$C_4$ alkyl, and wherein stabilization is characterized by

(a) reduced loss of proteolytic activity during storage at 37°C for 21 days being ≥5%, ≥10%, or ≥14% when compared to the loss of proteolytic activity in the absence of component (a) and/or

(b) reduced loss of proteolytic activity during storage at 37°C for 28 days being ≥2%, ≥5%, ≥10%, or ≥20% when compared to the loss of proteolytic activity in the absence of component (a) and/or

(c) reduced loss of proteolytic activity during storage at 37°C for 42 days being ≥10%, ≥15%, or ≥ 20% when compared to the loss of proteolytic activity in the absence of component (a).

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from linear $C_2$-$C_4$ alkyl, and wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition..

In one embodiment, the addition of component (a) to component (b) stabilizes protease during storage, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and wherein stabilization is characterized by

(a) reduced loss of proteolytic activity during storage at 37°C for 21 days being ≥5%, ≥10%, or ≥ 15% when compared to the loss of proteolytic activity in the absence of component (a) and/or

(b) reduced loss of proteolytic activity during storage at 37°C for 28 days being ≥5%, ≥10%, or ≥ 20% when compared to the loss of proteolytic activity in the absence of component (a) and/or

(c) reduced loss of proteolytic activity during storage at 37°C for 42 days being ≥9%, ≥ 15% or ≥20% when compared to the loss of proteolytic activity in the absence of component (a).

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is acetyl, and $R^2$, $R^3$, $R^4$ are selected from linear $C_2$-$C_4$ alkyl, preferably $C_2$ and $C_4$ alkyl, and
wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1/R^2$ or $R^4$, and wherein stabilization is characterized by

(a) reduced loss of proteolytic activity during storage at 37°C for 21 days being ≥5%, or ≥10% when compared to the loss of proteolytic activity in the absence of component (a) and/or

(b) reduced loss of proteolytic activity during storage at 37°C for 28 days being ≥2%, ≥5%, or ≥10% when compared to the loss of proteolytic activity in the absence of component (a) and/or

(c) reduced loss of proteolytic activity during storage at 37°C for 42 days being ≥5%, or ≥10% when compared to the loss of proteolytic activity in the absence of component (a).

The addition of component (a) to component (b) may stabilize protease during storage preferably in the presence of a complexing agent such as EDTA and/or DTPA and/or MGDA and/or GLDA, wherein component (a) is characterized by $R^1$ and $R^2$ in the compound according to formula (I) are H, $R^4$ is selected from linear $C_2$-$C_4$ alkyl, preferably $C_2$ alkyl, and $R^3$ equals either $R^1$/$R^2$ or $R^4$, and
wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In one embodiment, the addition of component (a) to component (b) stabilizes protease during storage, wherein component (a) is characterized by $R^1$ in the compound according to formula (I) is H, and $R^2$, $R^3$, $R^4$ are selected from phenylmethyl, and salicyl, and wherein stabilization is characterized by

(a) reduced loss of proteolytic activity during storage at 37°C for 21 days being ≥4%, or ≥10% when compared to the loss of proteolytic activity in the absence of component (a) and/or

(b) reduced loss of proteolytic activity during storage at 37°C for 28 days being ≥5%, or ≥10% when compared to the loss of proteolytic activity in the absence of component (a) and/or

(c) reduced loss of proteolytic activity during storage at 37°C for 42 days being ≥10%, ≥15%, or ≥20% when compared to the loss of proteolytic activity in the absence of component (a).

The addition of component (a) to component (b) may stabilize protease during storage preferably in the presence of a complexing agent such as EDTA and/or DTPA and/or MGDA and/or GLDA, wherein component (a) is characterized by $R^1$ in the compound according to formula (I) is H, and $R^2$, $R^3$, $R^4$ are selected from phenylmethyl, and salicyl, and
wherein component (a) is preferably comprised in amounts in the range of 1% to 5% by weight, more preferably in the range of 1.5% to 2% by weight, both relative to the total weight of the composition, and/or
wherein protease is preferably comprised in amounts in the range of 0.2% to 2% by weight, more preferably in about 0.5% by weight, both relative to the total weight of the composition, and/or
wherein optionally

- EDTA and/or DTPA are comprised in amounts up to 3% by weight, preferably up to 2.5% and/or
- MGDA and/or GLDA are comprised in amounts in the range of 10% to 30% by weight, preferably in the range of 15% to 25%, all relative to the total weight of the composition.

In embodiments, the subtilisin type protease (EC 3.4.21.62) of the above embodiments may be selected from protease according to SEQ ID NO:22 as described in EP 1921147 or variants thereof having proteolytic activity as disclosed above and from subtilisin 309 as disclosed in Table I a) of WO 89/06279 or variants thereof having proteolytic activity as disclosed above.

**Use of enzyme preparation for formulation processes**

The invention in one aspect relates to the use of the liquid enzyme preparation of the invention to be formulated into detergent formulations such as I&I and homecare formulations for laundry and hard surface cleaning, wherein at least components (a) and (b) are mixed in no specified order in one or more steps with one or more detergent components.

In one embodiment, at least components (a), (b) and (c) as disclosed above are mixed in no specified order in one or more steps with one or more detergent components.

In one aspect of the invention relates to a detergent formulation comprising the liquid enzyme preparation of the invention and one or more detergent components.

Detergent components vary in type and/or amount in a detergent formulation depending on the desired application such as laundering white textiles, colored textiles, and wool. The component(s) chosen further depend on physical form of a detergent formulation (liquid, solid, gel, provided in pouches or as a tablet, etc). The component(s) chosen e.g. for laundering formulations further depend on regional conventions which themselves are related to aspects like washing temperatures used, mechanics of laundry machine (vertical vs. horizontal axis machines), water consumption per wash cycle etc. and geographical characteristics like average hardness of water.

Individual detergent components and usage in detergent formulations are known to those skilled in the art. Suitable detergent components comprise inter alia surfactants, builders, polymers, alkaline, bleaching systems, fluorescent whitening agents, suds suppressors and stabilizers, hydrotropes, and corrosion inhibitors. Further examples are described e.g. in "complete Technology Book on Detergents with Formulations (Detergent Cake, Dishwashing Detergents, Liquid & Paste Detergents, Enzyme Detergents, Cleaning Powder & Spray Dried Washing Powder)", Engineers India Research Institute (EIRI), 6th edition (2015). Another reference book for those skilled in the art may be "Detergent Formulations Encyclopedia", Solverchem Publications, 2016.

It is understood that the detergent components are in addition to the components comprised in the enzyme preparation of the invention. If a component comprised in the enzyme preparation of the invention is also a detergent component, it might be the concentrations that need to be adjusted that the component is effective for the purpose desired in the detergent formulation.

Detergent components may have more than one function in the final application of a detergent formulation, therefore any detergent component mentioned in the context of a specific function herein, may also have another function in the final application of a detergent formulation. The function of a specific detergent component in the final application of a detergent formulation usually depends on its amount within the detergent formulation, i.e. the effective amount of a detergent component.

The term "effective amount" includes amounts of individual components to provide effective stain removal and/or effective cleaning conditions (e.g. pH, quantity of foaming), amounts of certain components to effectively provide optical benefits (e.g. optical brightening, dye transfer inhibition), and/or amounts of certain components to effectively aid the processing (maintain physical characteristics during processing, storage and use; e.g. viscosity modifiers, hydrotropes, desiccants).

In one embodiment, a detergent formulation is a formulation of more than two detergent components, wherein at least one component is effective in stain-removal, at least one component is effective in providing the optimal cleaning conditions, and at least one component is effective in maintaining the physical characteristics of the detergent.

Detergent formulations of the invention may comprise component (a) and component (b) being dissolved in solvent. Dissolved may mean being dissolved in the overall detergent formulation. Dissolved may mean component (a) and component (b) being part of the liquid enzyme preparation of the invention which may be encapsulated. Encapsulated liquid enzyme preparation may be part of a liquid detergent formulation or part of a solid detergent formulation.

In one embodiment of the present invention, detergent formulations, preferably liquid detergent formulations, comprise component (a) in amounts in the range of 0.1% to 30% by weight, relative to the total weight of the detergent formulation. The enzyme preparation may comprise component (a) in amounts in the range of 0.1% to 15% by weight, 0.25% to 10% by weight, 0.5% to 10% by weight, 0.5% to 6% by weight, or 1% to 3% by weight, all relative to the total weight of the detergent formulation.

In one embodiment of the present invention, detergent formulations, preferably liquid detergent formulations, comprise 0.5 to 20% by weight, particularly 1-10% by weight component (b) and 0.01% to 10% of component (a), more particularly 0.05 to 5% by weight and most particularly 0.1% to 2% by weight of component (a), all relative to the total weight of the detergent formulation.

In one embodiment, the detergent formulation of the invention is liquid at 20°C and 101.3 kPa. The liquid detergent formulation may comprise water or may be essentially free of water, meaning that no significant amounts of water are present. Non-significant amounts of water herein means, that the liquid detergent formulation comprises less than 15%, less than 10%, less than 7%, less than 5%, less than 4%, less than 3%, less than 2% by weight water, all relative to the total weight of the liquid detergent formulation, or no water. In one embodiment, enzyme concentrate free of water free of water means that the liquid detergent formulation does not comprise significant amounts of water but does comprise organic solvents in amounts of 30-80% by weight, relative to the total weight of the enzyme concentrate.

Water-comprising liquid detergent formulations may comprise water as sole solvent. In embodiments, mixtures of water with one or more water-miscible solvents are used as aqueous medium. The term water-miscible solvent refers to organic solvents that are miscible with water at ambient temperature without phase-separation. Examples are ethylene glycol, 1,2-propylene glycol, isopropanol, and diethylene glycol. Preferably, at least 50% by volume of the respective

aqueous medium is water, referring to the solvent.

Detergent formulations of the invention comprise at least one compound selected from surfactants, builders, polymers, fragrances and dyestuffs.

The detergent formulation of the invention comprises at least one surfactant selected from non-ionic surfactants, amphoteric surfactants, anionic surfactants, and cationic surfactants.

The detergent formulation may comprise 0.1 to 60% by weight relative to the total weight of the detergent formulation of surfactant. The detergent formulation may comprise at least one compound selected from anionic surfactants, non-ionic surfactants, amphoteric surfactants, and amine oxide surfactants as well as combinations of at least two of the foregoing. In one embodiment, the detergent formulation of the invention comprises 5 to 30 % by weight of anionic surfactant and at least one non-ionic surfactant, for example in the range of from 3 to 20% by weight, all relative to the total weight of the detergent formulation, wherein the detergent formulation may be liquid.

At least one non-ionic surfactant may be selected from alkoxylated alcohols, di- and multiblock copolymers of ethylene oxide and propylene oxide and reaction products of sorbitan with ethylene oxide or propylene oxide, alkyl polyglycosides (APG), hydroxyalkyl mixed ethers and amine oxides.

Preferred examples of alkoxylated alcohols and alkoxylated fatty alcohols are, for example, compounds of the general formula (II)

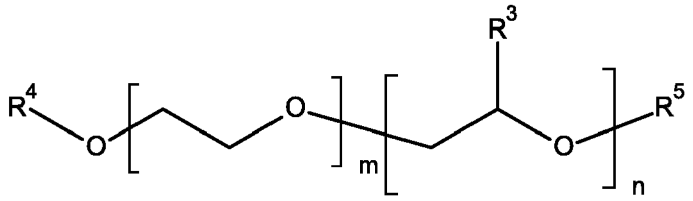

(II)

wherein

$R^3$ is identical or different and selected from hydrogen and linear $C_1$-$C_{10}$-alkyl, preferably in each case identical and ethyl and particularly preferably hydrogen or methyl,

$R^4$ is selected from $C_8$-$C_{22}$-alkyl, branched or linear, for example n-$C_8H_{17}$, n-$C_{10}H_{21}$, n-$C_{12}H_{25}$, n-$C_{14}H_{29}$, n-$C_{16}H_{33}$ or n-$C_{18}H_{37}$,

$R^5$ is selected from $C_1$-$C_{10}$-alkyl, methyl, ethyl, n-propyl, isopropyl, n-butyl, isobutyl, sec-butyl, tert-butyl, n-pentyl, isopentyl, sec-pentyl, neopentyl, 1,2-dimethylpropyl, isoamyl, n-hexyl, isohexyl, sec-hexyl, n-heptyl, n-octyl, 2-ethylhexyl, n-nonyl, n-decyl or isodecyl.

The variables m and n are in the range from zero to 300, where the sum of n and m is at least one, preferably in the range of from 3 to 50. Preferably, m is in the range from 1 to 100 and n is in the range from 0 to 30.

In one embodiment, compounds of the general formula (II) may be block copolymers or random copolymers, preference being given to block copolymers.

Other preferred examples of alkoxylated alcohols are, for example, compounds of the general formula (III):

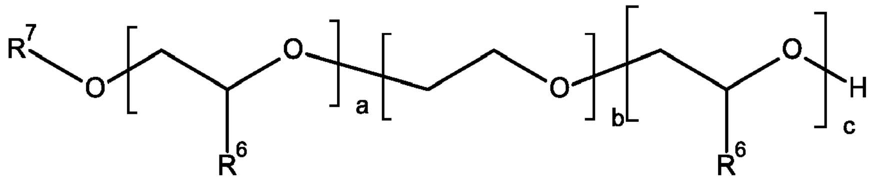

(III)

wherein

$R^6$ is identical or different and selected from hydrogen and linear $C_1$-$C_{10}$-alkyl, preferably identical in each case and

ethyl and particularly preferably hydrogen or methyl,

$R^7$ is selected from $C_6$-$C_{20}$-alkyl, branched or linear, in particular n-$C_8H_{17}$, n-$C_{10}H_{21}$, n-$C_{12}H_{25}$, n-$C_{13}H_{27}$, n-$C_{15}H_{31}$, n-$C_{14}H_{29}$, n-$C_{16}H_{33}$, n-$C_{18}H_{37}$,

a is a number in the range from zero to 10, preferably from 1 to 6,

b is a number in the range from 1 to 80, preferably from 4 to 20,

c is a number in the range from zero to 50, preferably 4 to 25.

The sum a + b + c is preferably in the range of from 5 to 100, even more preferably in the range of from 9 to 50.

In one embodiment, an alkoxylated alcohol is selected from those according to formula (III), wherein there is no $R^6$ and $R^7$ is selected from n-$C_8H_{17}$, n-$C_{10}H_{21}$, n-$C_{12}H_{25}$, n-$C_{13}H_{27}$, n-$C_{15}H_{31}$, n-$C_{14}H_{29}$, n-$C_{16}H_{33}$, n-$C_{18}H_{37}$; a and c are zero, b is in the range from 4 to 20, preferably 9.

Preferred examples for hydroxyalkyl mixed ethers are compounds of the general formula (IV)

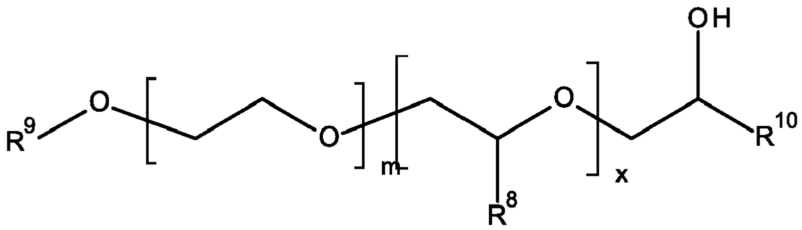

(IV)

in which the variables are defined as follows:

$R^8$ is identical or different and selected from hydrogen and linear $C_1$-$C_{10}$-alkyl, preferably in each case identical and ethyl, and particularly preferably hydrogen or methyl,

$R^9$ is selected from linear or branched $C_8$-$C_{22}$-alkyl and $C_8$-$C_{22}$-alkenyl; example include iso-$C_{11}H_{23}$, iso-$C_{13}H_{27}$, n-$C_8H_{17}$, n-$C_{10}H_{21}$, n-$C_{12}H_{25}$,n-$C_{14}H_{29}$, n-$C_{16}H_{33}$ or n-$C_{18}H_{37}$,

$R^{10}$ is selected from linear or branched $C_1$-$C_{18}$-alkyl and $C_2$-$C_{18}$ alkenyl; examples include methyl, ethyl, n-propyl, isopropyl, n-butyl, isobutyl, sec-butyl, tert-butyl, n-pentyl, isopentyl, sec-pentyl, neopentyl, 1,2-dimethylpropyl, isoamyl, n-hexyl, isohexyl, sec-hexyl, n-heptyl, n-octyl, 2-ethylhexyl, n-nonyl, n-decyl, isodecyl, n-dodecyl, n-tetradecyl, n-hexadecyl, and n-octadecyl.

The variables m and x are in the range from zero to 300, preferably in the range from zero to 100; the sum of m and x is at least one, preferably in the range of from 5 to 50.

Compounds of the general formulae (III) and (IV) may be block copolymers or random copolymers, preference being given to block copolymers.

Further suitable nonionic surfactants are selected from di- and multiblock copolymers, composed of ethylene oxide and propylene oxide. Further suitable nonionic surfactants are selected from ethoxylated or propoxylated sorbitan esters. Amine oxides or alkyl polyglycosides, especially linear $C_4$-$C_{18}$-alkyl polyglucosides and branched $C_8$-$C_{18}$-alkyl polyglycosides such as compounds of general average formula (V) are likewise suitable.

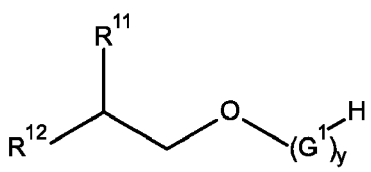

(V)

wherein:

$R^{11}$ is $C_1$-$C_4$-alkyl, in particular ethyl, n-propyl or isopropyl,

$R^{12}$ is -$(CH_2)_2$-$R^{11}$,

$G^1$ is selected from monosaccharides with 4 to 6 carbon atoms, especially from glucose and xylose,

y in the range of from 1.1 to 4, y being an average number.

Further examples of non-ionic surfactants are compounds of general formula (VIa) and (VIb)

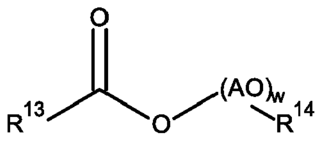

(VIa)

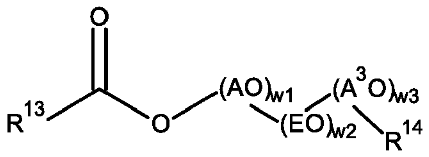

(VIb)

wherein

| | |
|---|---|
| AO | is selected from ethylene oxide, propylene oxide and butylene oxide, |
| EO | is ethylene oxide, $CH_2CH_2$-O, |
| $R^{13}$ | is $C_1$-$C_4$-alkyl, in particular ethyl, n-propyl or isopropyl, |
| $R^{14}$ | selected from $C_8$-$C_{18}$-alkyl, branched or linear |
| $A^3O$ | is selected from propylene oxide and butylene oxide, |
| w | is a number in the range of from 15 to 70, preferably 30 to 50, |
| w1 and w3 | are numbers in the range of from 1 to 5, and |
| w2 | is a number in the range of from 13 to 35. |

An overview of suitable further nonionic surfactants can be found in EP-A 0 851 023 and in DE-A 198 19 187.

In one embodiment, the detergent formulation comprises mixtures of two or more different nonionic surfactants.

At least one amphoteric surfactant may be selected from surfactants that bear a positive and a negative charge in the same molecule under use conditions. Preferred examples of amphoteric surfactants are so-called betaine-surfactants. Many examples of betaine-surfactants bear one quaternized nitrogen atom and one carboxylic acid group per molecule. A particularly preferred example of amphoteric surfactants is cocamidopropyl betaine (lauramidopropyl betaine).

Examples of amine oxide surfactants are compounds of the general formula (VII)

$$R^{13}R^{14}R^{15}N \rightarrow O \qquad (VII)$$

wherein $R^{13}$, $R^{14}$ and $R^{15}$ are selected independently from each other from aliphatic, cycloaliphatic or $C_2$-$C_4$-alkylene $C_{10}$-$C_{20}$-alkylamid0 moieties. Preferably, $R^{12}$ is selected from $C_8$-$C_{20}$-alkyl or $C_2$-$C_4$-alkylene $C_{10}$-$C_{20}$-alkylamido and $R^{13}$ and $R^{14}$ are both methyl.

A particularly preferred example is lauryl dimethyl aminoxide, sometimes also called lauramine oxide. A further particularly preferred example is cocamidylpropyl dimethylaminoxide, sometimes also called cocamidopropylamine oxide.

At least one anionic surfactant may be selected from alkali metal and ammonium salts of $C_8$-$C_{18}$-alkyl sulfates, of $C_8$-$C_{18}$-fatty alcohol polyether sulfates, of sulfuric acid half-esters of ethoxylated $C_4$-$C_{12}$-alkylphenols (ethoxylation: 1 to 50 mol of ethylene oxide/mol), $C_{12}$-$C_{18}$ sulfo fatty acid alkyl esters, for example of $C_{12}$-$C_{18}$ sulfo fatty acid methyl

esters, furthermore of $C_{12}$-$C_{18}$-alkylsulfonic acids and of $C_{10}$-$C_{18}$-alkylarylsulfonic acids. Preference is given to the alkali metal salts of the aforementioned compounds, particularly preferably the sodium salts.

Specific examples of anionic surfactants are compounds according to general formula (VIII)

$$C_sH_{2s+1}\text{-}O(CH_2CH_2O)_t\text{-}SO_3M \quad \text{(VIII)}$$

wherein

s being a number in the range of from 10 to 18, preferably 12 to 14, and even more preferably s = 12,

t being a number in the range of from 1 to 5, preferably 2 to 4 and even more preferably 3.

M being selected from alkali metals, preferably potassium and even more preferably sodium.

The variables s and t may be average numbers and therefore they are not necessarily whole numbers, while in individual molecules according to formula (VIII), both s and t denote whole numbers.

Further examples for suitable anionic surfactants are soaps, for example the sodium or potassium salts of stearic acid, oleic acid, palmitic acid, ether carboxylates, and alkylether phosphates.

Detergent formulations of the invention may comprise one or more compounds selected from complexing agents (chelating agents, sequestrating agents), precipitating agents, and ion exchange compounds, which may form water-soluble complexes with calcium and magnesium. Such compounds may be called "builders" or "building agents" herein, without meaning to limit such compounds to this function in the final application of a detergent formulation.

Non-phosphate based builders according to the invention include sodium gluconate, citrate(s), silicate(s), carbonate(s), phosphonate(s), amino carboxylate(s), polycarboxylate(s), polysulfonate(s), and polyphosphonate(s).

Detergent formulations of the invention may comprise one or more citrates. The term "citrate(s)" includes the mono- and the dialkali metal salts and in particular the mono- and preferably the trisodium salt of citric acid, ammonium or substituted ammonium salts of citric acid as well as citric acid as such. Citrate can be used as the anhydrous compound or as the hydrate, for example as sodium citrate dihydrate. The detergent formulation of the invention may comprise citric acid in amounts in the range of 0.1% to 10.0% by weight, in the range of 0.5% to 8.0% by weight, in the range of 1.0% to 5.0% by weight, or in the range of 2.0 to 4.0% by weight, all relative to the total weight of the detergent formulation. The citric acid may be provided as a mixture with formiate, e.g. Na-citrate:Na-formiate=9:1.

Detergent formulations of the invention may comprise one or more silicates. "Silicate(s)" in the context of the present invention include in particular sodium disilicate and sodium metasilicate, aluminosilicates such as sodium aluminosilicates like zeolith A (i.e. $Na_{12}(AlO_2)_{12}(SiO_2)_{12}*27H_2O$), and sheet silicates, in particular those of the formula alpha-$Na_2Si_2O_5$, beta-$Na_2Si_2O_5$, and delta-$Na_2Si_2O_5$.

Detergent formulations of the invention may comprise one or more carbonates. The term "carbonate(s)" includes alkali metal carbonates and alkali metal hydrogen carbonates, preferred are the sodium salts. Particularly suitable is sodium carbonate ($Na_2CO_3$).

Detergent formulations of the invention may comprise one or more phosphonates. "Phosphonates" include, but are not limited to 2-phosphinobutane-1,2,4-tricarboxylic acid (PBTC); ethylenediaminetetra(methylenephosphonic acid) (EDTMPA); 1-hydroxyethane-1,1-diphosphonic acid (HEDP), $CH_2C(OH)[PO(OH)_2]_2$; aminotris(methylenephosphonic acid) (ATMP), $N[CH_2PO(OH)_2]_3$; aminotris(methylenephosphonate), sodium salt (ATMP), $N[CH_2PO(ONa)_2]_3$; 2-hydroxyethyliminobis(methylenephosphonic acid), $HOCH_2CH_2N[CH_2PO(OH)_2]_2$; diethylenetriaminepenta(methylenephosphonic acid) (DTPMP), $(HO)_2POCH_2N[CH_2CH_2N[CH_2PO(OH)_2]_2]_2$; diethylenetriaminepenta(methylenephosphonate), sodium salt, $C_9H_{(28-x)}N_3Na_xO_{15}P_5$ (x=7); hexamethylenediamine(tetramethylenephosphonate), potassium salt, $C_{10}H_{(28-x)}N_2K_xO_{12}P_4$ (x=6); and bis(hexamethylene)triamine(pentamethylenephosphonic acid), $(HO_2)POCH_2N[(CH_2)_2N[CH_2PO(OH)_2]_2]_2$. Salts thereof may be suitable, too.

The detergent formulation of the invention may comprise at least one phosphonate, preferably selected from derivatives polyphosphonic acids such as of diphosphonic acid such as sodium salt of HEDP, derivatives of aminopolyphosphonic acid such as aminoalkylene phosphonic acids such as DTPMP in amounts in the range of 0.1 % to 5.0% by weight, in the range of 0.5% to 3.0% by weight, or in the range of 1.0% to 2.0% by weight, all relative to the total weight of the detergent formulation.

Detergent formulations of the invention may comprise one or more aminocarboxylates. Non-limiting examples of suitable "amino carboxylates" include, but are not limited to: diethanol glycine (DEG), dimethylglycine (DMG), nitrilitriacetic acid (NTA), N-hydroxyethylaminodiacetic acid, ethylenediaminetetraacetic acid (EDTA), N-(2hydroxyethyl)iminodiacetic acid (HEIDA), hydroxyethylenediaminetriacetic acid, N-hydroxyethyl-ethylenediaminetriacetic acid (HEDTA), hydroxyethylenediaminetetraacetic acid, diethylenetriaminepentaacetic acid (DTPA), and methylglycinediacetic acid (MGDA), glutamic acid-diacetic acid (GLDA), iminodisuccinic acid (IDS), hydroxyiminodisuccinic acid, ethylenediaminedisuccinic acid (EDDS), aspartic acid-diacetic acid, and alkali metal salts or ammonium salts thereof. Further suitable are aspartic acid-N-monoacetic acid (ASMA), aspartic acid-N,N-diacetic acid (ASDA), aspartic acid-N- monopropionic acid (ASMP), N-(2-sulfomethyl) aspartic acid (SMAS), N-(2-sulfoethyl) aspartic acid (SEAS), N-(2-sulfomethyl) glutamic acid (SMGL), N-(2-sulfoethyl) glutamic acid (SEGL), N-methyliminodiacetic acid (MIDA), alpha-alanine-N,N-diacetic acid (alpha-ALDA), serine-N,N-diacetic acid (SEDA), isoserine-N,N-diacetic acid (ISDA), phenylalanine-N,N-diacetic acid (PHDA), anthranilic acid-N ,N-diacetic acid (ANDA), sulfanilic acid-N,N-diacetic acid (SLDA), taurine-N,N-diacetic acid (TUDA) and sulfomethyl-N,N-diacetic acid (SMDA) and alkali metal salts or ammonium salts thereof. The term "ammonium salts" as used in in this context refers to salts with at least one cation that bears a nitrogen atom that is permanently or temporarily quaternized. Examples of cations that bear at least one nitrogen atom that is permanently quaternized include tetramethylammonium, tetraethylammonium, dimethyldiethyl ammonium, and n-$C_{10}$-$C_{20}$-alkyl trimethyl ammonium. Examples of cations that bear at least one nitrogen atom that is temporarily quaternized include protonated amines and ammonia, such as monomethyl ammonium, dimethyl ammonium, trimethyl ammonium, monoethyl ammonium, diethyl ammonium, triethyl ammonium, n-$C_{10}$-$C_{20}$-alkyl dimethyl ammonium 2-hydroxyethylammonium, bis(2-hydroxyethyl) ammonium, tris(2-hydroxyethyl)ammonium, N-methyl 2-hydroxyethyl ammonium, N,N-dimethyl-2-hydroxyethylammonium, and especially $NH_4^+$.

In one embodiment, detergent formulations of the invention comprise more than one builder. Preferably, inventive detergent formulations contain less than 0.2% by weight of nitrilotriacetic acid (NTA), or 0.01 to 0.1% NTA by weight relative to the total weight of the detergent formulation.

In one embodiment, the detergent formulation of the invention comprises at least one aminocarboxylate selected from ethylenediaminetetraacetic acid (EDTA), diethylenetriaminepentaacetic acid (DTPA), methylglycine diacetate (MGDA), and glutamic acid diacetate (GLDA), which all may be (partially) neutralized with alkali, in amounts in the range of 0.1% to 25.0% by weight, in the range of 1.0% to 15.0% by weight, in the range of 2.0% to 12.0% by weight, or in the range of 2.5% to 10.0% by weight, all relative to the total weight of the detergent formulation.

The term alkali refers to alkali metal cations, same or different, for example cations of lithium, sodium, potassium, rubidium, cesium, and combinations of at least two of the foregoing. Preferred examples of alkali metal cations are sodium and potassium and combinations of sodium and potassium.

In an embodiment, the detergent formulation of the invention comprises at least:

- one alkali metal salt of methyl glycine diacetic acid (MGDA), with an average of more than two and less than three of the carboxyl groups being neutralized with alkali, and/or
- one alkali metal salt of L- and D-enantiomers of glutamic acid diacetic acid (GLDA) or of enantiomerically pure L-GLDA, with an average of more than three of the carboxyl groups being neutralized with alkali, preferably an average of more than three and less than four of the carboxyl groups are neutralized with alkali.

In one embodiment of the present invention, alkali metal salts of MGDA are selected from compounds of the general formula (XIII):

$$[CH_3\text{-}CH(COO)\text{-}N(CH_2\text{-}COO)_2]M_{3\text{-}x1\text{-}y1}(NH_4)_{z1}H_{x1} \qquad \text{(XIII)}$$

The variables of formula (XIII) are defined as follows:

M is selected from alkali metal cations, same or different, for example cations of lithium, sodium, potassium, rubidium, cesium, and combinations of at least two of the foregoing. Preferred examples of alkali metal cations are sodium and potassium and combinations of sodium and potassium.
x1 is selected from 0.0 to 1.0, preferably 0.1 to 0.5, more preferably up to 0.1 to 0.3;
z1 is selected from 0.0 to 1.0, preferably 0.0005 to 0.5;
however, the sum of x1+z1 in formula (I) is greater than zero, for example 0.05 to 0.6.

Examples of $M_{3\text{-}x1\text{-}z1}(NH_4)_{z1}H_{x1}$ are $Na_{3\text{-}x1}H_{x1}$, $[Na_{0.7}(NH4)_{0.3}]_{3\text{-}x1}H_{x1}$, $[(NH_4)_{0.7}Na_{0.3}]_{3\text{-}x1}H_{x1}$, $(K_{0.7}Na_{0.3})_{3\text{-}x1}H_{x1}$, $(Na_{0.7}K_{0.3})_{3\text{-}x1}H_{x1}$, $(K_{0.22}Na_{0.78})_{3\text{-}x1}H_{x1}$, $(Na_{0.22}K_{0.78})_{3\text{-}x1}H_{x1}$, and $K_{3\text{-}x1}H_{x1}$. Preferred examples of $M_{3\text{-}x1}(NH_4)_{z1}H_{x1}$ are selected from $Na_{3\text{-}x1}H_{x1}$, $(K_{0.85}Na_{0.15})_{3\text{-}x1}H_{x1}$, and $(Na_{0.85}K_{0.15})_{3\text{-}x1}H_{x1}$.

In one embodiment of the present invention, MGDA is selected from at least one alkali metal salt of racemic MGDA and from alkali metal salts of mixtures of L- and D-enantiomers according to formula (XIII), said mixture containing predominantly the respective L-isomer with an enantiomeric excess (ee) in the range of from 5 to 99%, preferably 5 to 95 %, more preferably from 10 to 75% and even more preferably from 10 to 66%.

In one embodiment of the present invention, the total degree of alkali neutralization of MGDA is in the range of from 0.80 to 0.98 mol-%, preferred are 0.90 to 0.97%. The total degree of alkali neutralization does not take into

account any neutralization with ammonium.

In one embodiment of the present invention, alkali metal salts of GLDA are selected from compounds of the general formula (XIV)

$$[OOC\text{-}(CH_2)_2\text{-}CH(COO)\text{-}N(CH_2\text{-}COO)_2]M_{4\text{-}x2\text{-}z2}(NH_4)_{z2}H_{x2} \qquad (XIV)$$

The variables of formula (XIV) are defined as follows:

M is selected from alkali metal cations, same or different, as defined above for compounds of general formula (XIII)
x2 is selected from 0.0 to 2.0, preferably 0.02 to 0.5, more preferably up to 0.1 to 0.3;
z2 is selected from 0.0 to 1.0, preferably 0.0005 to 0.5;
however, the sum of x2+z2 in formula (I) is greater than zero, for example 0.05 to 0.6.

Examples of $M_{3\text{-}x2\text{-}z2}(NH_4)_{z2}H_{x1}$ are $Na_{3\text{-}x2}H_{x2}$, $[Na_{0.7}(NH_4)_{0.3}]_{3\text{-}x2}H_{x2}$, $[(NH_4)_{0.7}Na_{0.3}]_{3\text{-}x2}H_{x2}$, $(K_{0.7}Na_{0.3})_{3\text{-}x2}H_{x2}$, $(Na_{0.7}K_{0.3})_{3\text{-}x2}H_{x2}$, $(K_{0.22}Na_{0.78})_{3\text{-}x2}H_{x2}$, $(Na_{0.22}K_{0.78})_{3\text{-}x2}H_{x2}$, and $K_{3\text{-}x2}H_{x2}$. Preferred examples of $M_{3\text{-}x2}(NH_4)_{z2}H_{x2}$ are selected from $Na_{3\text{-}x2}H_{x2,}$ $(K_{0.85}Na_{0.15})_{3\text{-}x2}H_{x2}$, and $(Na_{0.85}K_{0.15})_{3\text{-}x2}H_{x2}$.

In one embodiment of the present invention, alkali metal salts of GLDA may be selected from alkali metal salts of the L- and D- enantiomers according to formula (XIV), said mixture containing the racemic mixture or preferably predominantly the respective L-isomer, for example with an enantiomeric excess (ee) in the range of from 5 to 99%, preferably 5 to 95%.

The enantiomeric excess can be determined, e.g., by measuring the polarization (polarimetry) or preferably by chromatography, for example by HPLC with a chiral column, for example with one or more cyclodextrins as immobilized phase or with a ligand exchange (Pirkle-brush) concept chiral stationary phase. Preferred is determination of the enantiomeric excess by HPLC with an immobilized optically active ammonium salt such as D-penicillamine.

Generally, in the context of the present invention, small amounts of MGDA and/or GLDA may also bear a cation other than alkali metal. It is thus possible that small amounts of builder, such as 0.01 % to 5 mol-% of total builder may bear alkali earth metal cations such as, e.g., $Mg^{2+}$ or $Ca^{2+}$, or a transition metal cation such as, e.g., a $Fe^{2+}$ or $Fe^{3+}$ cation. "Small amounts" of MGDA and/or GLDA herein refer to a total of 0.1% to 1 w/w%, relative to the respective builder.

In one embodiment of the present invention, MGDA and/or GLDA comprised in detergent formulations may contain in the range of 0.1% to 10% by weight relative to the respective builder of one or more optically inactive impurities, at least one of the impurities being at least one of the impurities being selected from iminodiacetic acid, formic acid, glycolic acid, propionic acid, acetic acid and their respective alkali metal or mono-, di- or triammonium salts.

Further examples of detergent builders are polymers with complexing groups like, for example, polyethylenimine in which 20 to 90 mole-% of the N-atoms bear at least one $CH_2COO$- group, and the respective alkali metal salts of the above sequestrants, especially their sodium salts.

Further examples of suitable polymers are polyalkylenimines, for example polyethylenimines and polypropylene imines. Polyalkylenimines may be used as such or as polyalkoxylated derivatives, for examples ethoxylated or propoxylated. Polyalkylenimines comprise at least three alkylenimine units per molecule.

In one embodiment of the present invention, said alkylenimine unit is a $C_2$-$C_{10}$-alkylendiamine unit, for example a 1,2-propylendiamine, preferably an $\alpha,\omega$-$C_2$-$C_{10}$-alkylendiamine, for example 1,2-ethylendiamine, 1,3-propylendiamine, 1,4-butylendiamine, 1,5-pentylendiaminne, 1,6-hexandiamine (also being referred to as 1,6-hexylendiamine), 1,8-diamine or 1,10-decandiamine, even more preferred are 1,2-ethylendiamine, 1,3-propylendiamine, 1,4-butylendiamine, and 1,6-hexandiamine.

In another embodiment of the present invention, said polyalkylenimine is selected from polyalkylenimine unit, preferably a polyethylenimine or polypropylenimine unit.

The term "polyethylenimine" in the context of the present invention does not only refer to polyethylenimine homopolymers but also to polyalkylenimines comprising $NH\text{-}CH_2\text{-}CH_2\text{-}NH$ structural elements together with other alkylene diamine structural elements, for example $NH\text{-}CH_2\text{-}CH_2\text{-}CH_2\text{-}NH$ structural elements, $NH\text{-}CH_2\text{-}CH(CH_3)\text{-}NH$ structural elements, $NH\text{-}(CH_2)_4\text{-}NH$ structural elements, $NH\text{-}(CH_2)_6\text{-}NH$ structural elements or $(NH\text{-}(CH_2)_8\text{-}NH$ structural elements but the $NH\text{-}CH_2\text{-}CH_2\text{-}$ NH structural elements being in the majority with respect to the molar share. Preferred polyethylenimines comprise $NH\text{-}CH_2\text{-}CH_2\text{-}NH$ structural elements being in the majority with respect to the molar share, for example amounting to 60 mol-% or more, more preferably amounting to at least 70 mol-%, referring to all alkylenimine structural elements. In a special embodiment, the term polyethylenimine refers to those polyalkylenimines that bear only one or zero alkylenimine structural element per polyethylenimine unit that is different from $NH\text{-}CH_2\text{-}CH_2\text{-}NH$.

The term "polypropylenimine" in the context of the present invention does not only refer to polypropylenimine homopolymers but also to polyalkylenimines comprising $NH\text{-}CH_2\text{-}CH(CH_3)\text{-}NH$ structural elements together with other alkylene diamine structural elements, for example $NH\text{-}CH_2\text{-}CH_2\text{-}CH_2\text{-}NH$ structural elements, $NH\text{-}CH_2\text{-}CH_2\text{-}NH$ structural elements, $NH\text{-}(CH_2)_4\text{-}NH$ structural elements, $NH\text{-}(CH_2)_6\text{-}NH$ structural elements or $(NH\text{-}(CH_2)_8\text{-}NH$ structural elements

but the $NH-CH_2-CH(CH_3)-NH$ structural elements being in the majority with respect to the molar share. Preferred polypropylenimines comprise $NH-CH_2-CH(CH_3)-NH$ structural elements being in the majority with respect to the molar share, for example amounting to 60 mol-% or more, more preferably amounting to at least 70 mol-%, referring to all alkylenimine structural elements. In a special embodiment, the term polypropylenimine refers to those polyalkylenimines that bear only one or zero alkylenimine structural element per polypropylenimine unit that is different from $NH-CH_2-CH(CH_3)-NH$.

Branches may be alkylenamino groups such as, but not limited to $-CH_2-CH_2-NH_2$ groups or $(CH_2)_3-NH_2$-groups. Longer branches may be, for examples, $-(CH_2)_3-N(CH_2CH_2CH_2NH_2)_2$ or $-(CH_2)_2-N(CH_2CH_2NH_2)_2$ groups. Highly branched polyethylenimines are, e.g., polyethylenimine dendrimers or related molecules with a degree of branching in the range from 0.25 to 0.95, preferably in the range from 0.30 to 0.80 and particularly preferably at least 0.5. The degree of branching can be determined for example by $^{13}C$-NMR or $^{15}N$-NMR spectroscopy, preferably in $D_2O$, and is defined as follows:

$$DB = D+T/D+T+L$$

with D (dendritic) corresponding to the fraction of tertiary amino groups, L (linear) corresponding to the fraction of secondary amino groups and T (terminal) corresponding to the fraction of primary amino groups.

Within the context of the present invention, branched polyethylenimine units are polyethylenimine units with DB in the range from 0.25 to 0.95, particularly preferably in the range from 0.30 to 0.90% and very particularly preferably at least 0.5. Preferred polyethylenimine units are those that exhibit little or no branching, thus predominantly linear or linear polyethylenimine units.

In the context of the present invention, $CH_3$-groups are not being considered as branches.

In one embodiment of the present invention polyalkylenimine may have a primary amine value in the range of from 1 to 1000 mg KOH/g, preferably from 10 to 500 mg KOH/g, most preferred from 50 to 300 mg KOH/g. The primary amine value can be determined according to ASTM D2074-07.

In one embodiment of the present invention polyalkylenimine may have a secondary amine value in the range of from 10 to 1000 mg KOH/g, preferably from 50 to 500 mg KOH/g, most preferred from 50 to 500 mg KOH/g. The secondary amine value can be determined according to ASTM D2074-07.

In one embodiment of the present invention polyalkylenimine may have a tertiary amine value in the range of from 1 to 300 mg KOH/g, preferably from 5 to 200 mg KOH/g, most preferred from 10 to 100 mg KOH/g. The tertiary amine value can be determined according to ASTM D2074-07.

In one embodiment of the present invention, the molar share of tertiary N atoms is determined by $^{15}N$-NMR spectroscopy. In cases that tertiary amine value and result according to $^{13}C$-NMR spectroscopy are inconsistent, the results obtained by $^{13}C$-NMR spectroscopy will be given preference.

In one embodiment of the present invention, the average molecular weight $M_w$ of said polyalkylenimine is in the range of from 250 to 100,000 g/mol, preferably up to 50,000 g/mol and more preferably from 800 up to 25,000 g/mol. The average molecular weight $M_w$ of polyalkylenimine may be determined by gel permeation chromatography (GPC) of the intermediate respective polyalkylenimine, with 1.5 % by weight aqueous formic acid as eluent and cross-linked polyhydroxyethyl methacrylate as stationary phase.

Said polyalkylenimine may be free or alkoxylated, said alkoxylation being selected from ethoxylation, propoxylation, butoxylation and combinations of at least two of the foregoing. Preference is given to ethylene oxide, 1,2-propylene oxide and mixtures of ethylene oxide and 1,2-propylene oxide. If mixtures of at least two alkylene oxides are applied, they can be reacted step-wise or simultaneously.

In one embodiment of the present invention, an alkoxylated polyalkylenimine bears at least 6 nitrogen atoms per unit.

In one embodiment of the present invention, polyalkylenimine is alkoxylated with 2 to 50 moles of alkylene oxide per NH group, preferably 5 to 30 moles of alkylene oxide per NH group, even more preferred 5 to 25 moles of ethylene oxide or 1,2-propylene oxide or combinations therefrom per NH group. In the context of the present invention, an $NH_2$ unit is counted as two NH groups. Preferably, all - or almost all - NH groups are alkoxylated, and there are no detectable amounts of NH groups left.

Depending on the manufacture of such alkoxylated polyalkylenimine, the molecular weight distribution may be narrow or broad. For example, the polydispersity $Q = M_w/M_n$ in the range of from 1 to 3, preferably at least 2, or it may be greater than 3 and up to 20, for example 3.5 to 15 and even more preferred in the range of from 4 to 5.5.

In one embodiment of the present invention, the polydispersity Q of alkoxylated polyalkylenimine is in the range of from 2 to 10.

In one embodiment of the present invention alkoxylated polyalkylenimine is selected from polyethoxylated polyethylenimine, ethoxylated polypropylenimine, ethoxylated $\alpha,\omega$-hexandiamines, ethoxylated and propoxylated polyethylenimine, ethoxylated and propoxylated polypropylenimine, and ethoxylated and poly-propoxylated $\alpha,\omega$-hexandi-

amines.

In one embodiment of the present invention the average molecular weight $M_n$ (number average) of alkoxylated polyethylenimine is in the range of from 2,500 to 1,500,000 g/mol, determined by GPC, preferably up to 500,000 g/mol.

In one embodiment of the present invention, the average alkoxylated polyalkylenimine are selected from ethoxylated $\alpha,\omega$-hexanediamines and ethoxylated and poly-propoxylated $\alpha,\omega$-hexanediamines, each with an average molecular weight $M_n$ (number average) in the range of from 800 to 500,000 g/mol, preferably 1,000 to 30,000 g/mol.

Detergent formulations of the invention may comprise one or more complexing agent other than EDTA, DTPA, MGDA and GLDA, e.g. citrate, phosphonic acid derivatives, for example the disodium salt of hydroxyethane-1,1-diphosphonic acid ("HEDP"), for example trisodium citrate, and phosphates such as STPP (sodium tripolyphosphate).

In one embodiment, the detergent formulation of the invention comprises a builder system comprising

- ethylenediaminetetraacetic acid (EDTA) and/or diethylenetriaminepentaacetic acid (DTPA) and/or methylglycine diacetate (MGDA) and/or glutamic acid diacetate (GLDA), as disclosed above in amounts in the range of 0.1 % to 25.0% by weight, in the range of 1.0% to 15.0% by weight, or in the range of 3.0% to 10.0% by weight, all relative to the total weight of the detergent formulation;

- optionally citric acid in amounts in the range of 0.1% to 10.0% by weight, in the range of 0.5% to 8.0% by weight, in the range of 1.0% to 5.0% by weight, or in the range of 2.0% to 4% by weight, all relative to the total weight of the detergent formulation; the citric acid may be provided as a mixture with formiate, e.g. Na-citrate:Na-formiate=9:1;

- optionally at least one phosphonate, preferably selected from derivatives polyphosphonic acids such as of diphosphonic acid such as sodium salt of HEDP, and derivatives of aminopolyphosphonic acid such as aminoalkylene phosphonic acids such as DTPMP in amounts in the range of 0.1% to 5.0% by weight, in the range of 0.5% to 3.0% by weight, or in the range of 1.0% to 2.0% by weight, all relative to the total weight of the detergent formulation;

- optionally at least one polycarboxylate selected from homopolymers with the repeating monomer being the same unsaturated carboxylic acid, such as polyacrylic acid (PAA) and copolymers with the repeating monomers being at least two different unsaturated carboxylic acids, such as copolymers of acrylic acid with methacrylic acid, copolymers of acrylic acid or methacrylic acid and maleic acid and/or fumaric acid, in amounts in the range of 0.1% to 10% by weight, 0.25% to 5% by weight, or 0.3% to 2.5% by weight, all relative to the total weight of the detergent formulation;

In one embodiment of the present invention, the formulation according to the invention is free from phosphates and polyphosphates, with hydrogenphosphates being subsumed, for example free from trisodiumphosphate, pentasodiumtripolyphosphate and hexasodiummetaphosphate. In connection with phosphates and polyphosphates, in the context of the present invention, "free from" is to be understood as meaning that the content of phosphate and polyphosphate is in total in the range from 10 ppm to 0.2% by weight, determined by gravimetry and relative to the total weight of the detergent formulation.

Liquid detergent formulations of the invention may comprise one or more corrosion inhibitors. Non-limiting examples of suitable corrosion inhibitors include sodium silicate, triazoles such as benzotriazoles, bisbenzotriazoles, aminotriazoles, alkylaminotriazoles, phenol derivatives such as hydroquinone, pyrocatechol, hydroxyhydroquinone, gallic acid, phloroglucinol and pyrogallol, further polyethylenimine and salts of bismuth or zinc. Corrosion inhibitors may be formulated into liquid detergent formulations of the invention in amounts of 0.1 to 1.5 % w/w relative to the overall weight of the liquid detergent formulation.

Liquid detergent formulations of the invention may comprise at least one graft copolymer composed of

(a) at least one graft base selected from nonionic monosaccharides, disaccharides, oligosaccharides and polysaccharides,
and side chains obtained by grafting on of
(b) at least one ethylenically unsaturated mono- or dicarboxylic acid and
(c) at least one compound of the general formula (XI),

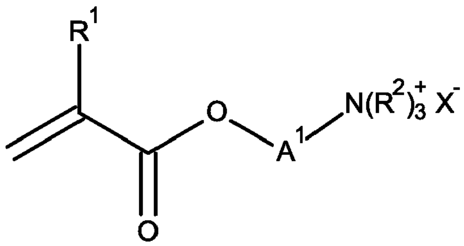

(XI)

wherein the variables are defined as follows:

$R^1$ is selected from methyl and hydrogen,

$A^1$ is selected from $C_2$-$C_4$-alkylene,

$R^2$ are identical or different and selected from $C_1$-$C_4$-alkyl,

$X^-$ is selected from halide, mono-$C_1$-$C_4$-alkyl sulfate and sulfate.

Liquid detergent formulations of the invention may comprise one or more buffers such as monoethanolamine and N,N,N-triethanolamine.

Liquid detergent formulations of the invention may be adapted in sudsing characteristics for satisfying various purposes. Hand dishwashing detergents usually request stable suds. Automatic dishwasher detergents are usually requested to be low sudsing. Laundry detergents may range from high sudsing through a moderate or intermediate range to low. Low sudsing laundry detergents are usually recommended for front-loading, tumbler-type washers and washer-dryer combinations. Those skilled in the art are familiar with using suds stabilizers or suds suppressors as detergent components in detergent formulations which are suitable for specific applications. Examples of suds stabilizers include but are not limited to alkanolamides and alkylamine oxides. Examples of suds suppressors include but are not limited to alkyl phosphates, silicones and soaps.

Liquid detergent formulations of the invention may comprise one or more fragrances such as benzyl salicylate, 2-(4-tert.-butylphenyl) 2-methylpropional, commercially available as Lilial®, and hexyl cinnamaldehyde.

Liquid detergent formulations of the invention may comprise one or more dyestuffs such as Acid Blue 9, Acid Yellow 3, Acid Yellow 23, Acid Yellow 73, Pigment Yellow 101, Acid Green 1, Solvent Green 7, and Acid Green 25.

Liquid detergent formulations may comprise at least one compound selected from organic solvents, preservatives, viscosity modifiers, and hydrotropes.

In one embodiment of the present invention, liquid detergent formulations comprise amounts of organic solvents are 0.5 to 25% by weight, relative to the total weight of the liquid detergent formulation. Especially when inventive liquid detergent formulations are provided in pouches or the like, 8 to 25% by weight of organic solvent(s) relative to the total weight of the liquid detergent formulation may be comprised. Organic solvents are those disclosed above.

Inventive liquid detergent formulations may comprise one or more preservatives selected from those disclosed above in amounts effective in avoiding microbial contamination of the liquid detergent formulation.

In one embodiment of the present invention, liquid detergent formulations comprise one or more viscosity modifiers. Non-limiting examples of suitable viscosity modifiers include agar-agar, carragene, tragacanth, gum arabic, xanthan gum, alginates, pectins, hydroxyethyl cellulose, hydroxypropyl cellulose, starch, gelatin, locust bean gum, cross-linked poly(meth)acrlyates, for example polyacrlyic acid cross-linked with bis-(meth)acrylamide, furthermore silicic acid, clay such as - but not limited to - montmorrilionite, zeolite, dextrin, and casein. Viscosity modifiers may be comprised in amounts effective in providing the desired viscosity.

In one embodiment of the present invention, liquid detergent formulations comprise one or more hydrotropes which may be organic solvents such as ethanol, isopropanol, ethylene glycol, 1,2-propylene glycol, and further organic solvents that are water-miscible under normal conditions without limitation. Further examples of suitable hydrotropes are the sodium salts of toluene sulfonic acid, of xylene sulfonic acid, and of cumene sulfonic acid. Hydrotropes may be comprised in amounts that facilitate or enables the dissolution of compounds that exhibit limited solubilty in water.

In one embodiment of the present invention, the formulation according to the invention is free from those heavy metal compounds which do not act as bleach catalysts, in particular from compounds of iron. In connection with heavy metal compounds in the context of the present invention, "free from" is to be understood as meaning that the content of heavy metal compounds which do not act as bleach catalysts is in total in the range from 0 to 100 ppm, preferably 1 to 30 ppm, determined by the Leach method. In the context of the present invention, "heavy metals" are all metals with a

specific density of at least 6 $g/cm^3$, with the exception of zinc and bismuth. In particular, heavy metals are precious metals, and also iron, copper, lead, tin, nickel, cadmium and chromium.

When inventive liquid detergent formulations are provided in compartmented pouches or the like, the compartment comprising the liquid enzyme preparation of the invention is provided separated from the compartment comprising bleaches, such as inorganic peroxide compounds or chlorine bleaches such as sodium hypochlorite. In one embodiment, the compartment comprising the liquid enzyme preparation also comprises at least one complexing agent such as EDTA and/or DTPA and/or MGDA and/or GLDA, wherein MGDA and GLDA are as disclosed above.

In one embodiment, liquid detergent formulations of the invention are free from bleaches, for example free from inorganic peroxide compounds or chlorine bleaches such as sodium hypochlorite, meaning that liquid detergent formulations according to the invention comprise in total 0.01% by weight or less of inorganic peroxide compound and chlorine bleach, relative in each case on total weight of the liquid detergent formulation.

Liquid detergent formulation may be called aqueous herein when the solvent comprised in the detergent formulation is essentially water. In one embodiment, water is the sole solvent. In other embodiments, mixtures of water with one or more water-miscible solvents are used. The term water-miscible solvent refers to organic solvents that are miscible with water at ambient temperature without phase-separation. Examples are ethylene glycol, 1,2-propylene glycol, isopropanol, and diethylene glycol. Preferably, at least 50% by volume referring to the whole solvent comprised in the aqueous detergent formulation is water.

"Detergent formulation" or "cleaning formulation" herein means formulations designated for cleaning soiled material. Cleaning may mean laundering or hard surface cleaning. Soiled material according to the invention includes textiles and/or hard surfaces.

The term "laundering" relates to both household laundering and industrial laundering and means the process of treating textiles with a solution comprising a detergent formulation of the present invention. The laundering process may be carried out by using technical devices such as a household or an industrial washing machine. Alternatively, the laundering process may be done by hand.

The term "textile" means any textile material including yarns (thread made of natural or synthetic fibers used for knitting or weaving), yarn intermediates, fibers, non-woven materials, natural materials, synthetic materials, as well as fabrics (a textile made by weaving, knitting or felting fibers) made of these materials such as garments (any article of clothing made of textile), cloths and other articles.

The term "fibers" includes natural fibers, synthetic fibers, and mixtures thereof. Examples of natural fibers are of plant (such as flax, jute and cotton) or animal origin, comprising proteins like collagen, keratin and fibroin (e.g. silk, sheeps wool, angora, mohair, cashmere). Examples for fibers of synthetic origin are polyurethane fibers such as Spandex® or Lycra®, polyester fibers, polyolefins such as elastofin, or polyamide fibers such as nylon. Fibers may be single fibers or parts of textiles such as knitwear, wovens, or nonwovens.

The term "hard surface cleaning" is defined herein as cleaning of hard surfaces wherein hard surfaces may include any hard surfaces in the household, such as floors, furnishing, walls, sanitary ceramics, glass, metallic surfaces including cutlery or dishes. The term "hard surface cleaning" may therefore may mean "dish washing" which refers to all forms of washing dishes, e.g. by hand or automatic dish wash (ADW). Dish washing includes, but is not limited to, the cleaning of all forms of crockery such as plates, cups, glasses, bowls, all forms of cutlery such as spoons, knives, forks and serving utensils as well as ceramics, plastics such as melamine, metals, china, glass and acrylics.

In one aspect, the invention relates to the providing a liquid detergent formulation comprising at least the enzyme preparation of the invention and at least one detergent component.

In one embodiment, the invention provides a liquid detergent formulation comprising at least components (a) and (b) as disclosed above and at least one detergent component, wherein component (b) comprises
at least one amylase selected from alpha-amylases (EC 3.2.1.1) as disclosed above, preferably selected from

- amylase from *Bacillus sp.707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity;

and optionally one or more further enzymes, selected from proteases, lipases, cellulases and mannanases - all as disclosed above.

In embodiments of the above embodiments, the liquid detergent formulation has increased storage stability when compared to a liquid detergent formulation lacking component (a). Increased storage stability in this context may mean that there is no significant loss in wash performance towards at least one enzyme-sensitive stain type, preferably towards at least amylase-sensitive stains, after storage of the detergent at 37°C formulation for 1, 2, 4, 6 or 8 weeks.

Wash performance towards specified enzyme sensitive-stain type means that the respective enzyme is acting on the enzyme-sensitive parts of a specific stain. Different enzymes are able to breakdown different types of stains. For example, proteases are acting on proteinaceous material and thereby degrade proteins into smaller peptides. Amylase-sensitive stains are usually starch-based stains wherein the carbohydrates may be degraded into oligo- or monosaccharides by amylases. Lipase sensitive stains are usually comprising fatty compounds. Mannanase sensitive stains usually comprise mannan. Cellulases may clean indirectly by hydrolyzing certain glycosidic bonds in cotton fibers. In this way, particulate soils attached to microfibrils are removed.

No significant loss in wash performance after storage may mean that the detergent has

i. at least 90% wash performance after 4 weeks of storage at 37°C when compared to the wash performance of the same detergent before storage; and/or

ii. at least 85% wash performance after 6 weeks of storage at 37°C when compared to the wash performance of the same detergent before storage; and/or

iii. at least 80% wash performance after 8 weeks of storage at 37°C when compared to the wash performance of the same detergent before storage.

In one embodiment, the liquid detergent formulation comprising at least components (a) and (b) and at least one detergent component has increased storage stability when compared to a liquid detergent formulation lacking component (a), wherein component (b) comprises at least one amylase, preferably selected from alpha-amylases (EC 3.2.1.1) as disclosed above, preferably selected from

- amylase from *Bacillus sp. 707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921 , wherein the hybrid amylase has amylolytic activity; preferably, the hybrid alpha-amylase is at least 95% identical to SEQ ID NO: 30 as disclosed in WO 2014/183921 and having amylolytic activity.

Increased storage stability in one embodiment means that the wash performance of a liquid detergent formulation after 4 to 8 weeks of storage at 37°C is increased by at least 5%, at least 6%, at least 7%, at least 8%, at least 9%, or at least 10% when compared to a liquid detergent formulation lacking component (a) stored for the same time at the same temperature. Increased storage stability may mean that the wash performance of a liquid detergent formulation after 8 weeks of storage at 37°C is increased by at least 5%, at least 6%, at least 7%, at least 8%, at least 9%, or at least 10% when compared to a liquid detergent formulation lacking component (a) stored for the same time at the same temperature.

In one embodiment, the liquid detergent formulation comprising at least components (a) and (b) and at least one detergent component has increased storage stability when compared to a liquid detergent formulation lacking component (a), wherein component (b) comprises in addition to at least one alpha amylase as disclosed above, at least one protease as disclosed above, preferably selected from the group of serine endopeptidases (EC 3.4.21), more preferably selected from the group of subtilisin type proteases (EC 3.4.21.62). The protease may be selected from protease according to SEQ ID NO:22 as described in EP 1921147 or variants thereof having proteolytic activity as disclosed above and from subtilisin 309 as disclosed in Table I a) of WO 89/06279 or variants thereof having proteolytic activity as disclosed above.

In one embodiment, the liquid detergent formulation comprising at least components (a) and (b) and at least one detergent component has increased storage stability when compared to a liquid detergent formulation lacking component (a), wherein component (b) comprises in addition to at least one alpha amylase as disclosed above, at least one lipase as disclosed above, preferably selected from the group of fungal triacylglycerol lipase (EC class 3.1.1.3). Fungal triacylglycerol lipase may be selected from *Thermomyces lanuginose* lipase. In one embodiment, *Thermomyces lanuginosa* lipase is selected from triacylglycerol lipase according to amino acids 1-269 of SEQ ID NO:2 of US 5869438 and variants thereof having lipolytic activity.

In one aspect, the invention relates to the use of component (a) to stabilize component (b) within a liquid detergent formulation, preferably comprising at least one complexing agent, preferably
EDTA and/or DTPA in amounts up to 3% by weight, preferably up to 2.5% and/or
MGDA and/or GLDA in amounts in the range of 10% to 30% by weight, preferably in the range of 15% to 25%, all relative to the total weight of the liquid detergent formulation,
wherein component (b) comprises
at least one amylase as disclosed above, preferably selected from alpha-amylases (EC 3.2.1.1) as disclosed above, more preferably selected from

- amylase from *Bacillus sp. 707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO

2014/183921 , wherein the hybrid amylase has amylolytic activity; preferably, the hybrid alpha-amylase is at least 95% identical to SEQ ID NO: 30 as disclosed in WO 2014/183921 and having amylolytic activity;

and optionally at least one further enzyme, preferably selected from the group of proteases, lipases, cellulases, and mannannases - all as disclosed above.

In one embodiment, at least one protease is selected from the group of serine endopeptidases (EC 3.4.21), more preferably selected from the group of subtilisin type proteases (EC 3.4.21.62). The protease may be selected from subtilisin 147 and/or 309 as disclosed in WO 89/06279 or variants thereof having proteolytic activity, subtilisin from *Bacillus lentus* as disclosed in WO 91/02792 or variants thereof having proteolytic activity, and subtilisin according to SEQ ID NO:22 as described in EP 1921147 or variants thereof having proteolytic activity.

In one embodiment, at least one lipase is selected from the group of fungal triacylglycerol lipase (EC class 3.1.1.3). Fungal triacylglycerol lipase may be selected from *Thermomyces lanuginose* lipase. In one embodiment, *Thermomyces lanuginosa* lipase is selected from triacylglycerol lipase according to amino acids 1-269 of SEQ ID NO:2 of US 5869438 and variants thereof having lipolytic activity.

In one aspect, the invention relates to the method to stabilize component (b) within a liquid detergent formulation, preferably comprising

EDTA and/or DTPA in amounts up to 3% by weight, preferably up to 2.5% and/or

MGDA and/or GLDA in amounts in the range of 10% to 30% by weight, preferably in the range of 15% to 25%, all relative to the total weight of the liquid detergent formulation,

wherein component (b) comprises

at least one amylase as disclosed above, preferably selected from alpha-amylases (EC 3.2.1.1) as disclosed above, more preferably selected from

- amylase from *Bacillus sp. 707* or variants thereof having amylolytic activity, preferably selected from amylases having SEQ ID NO:6 as disclosed in WO 99/19467 and variants thereof having amylolytic activity;

- amylase selected from those comprising amino acids 1 to 485 of SEQ ID NO:2 as described in WO 00/60060 those having SEQ ID NO: 12 as described in WO 2006/002643, and variants thereof having amylolytic activity;

- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;

- amylase from *Bacillus amyloliquefaciens* or variants thereof having amylolytic activity, preferably selected from amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity;

and optionally at least one further enzyme, preferably selected from the group of proteases, lipases, cellulases, and mannanases - all as disclosed above,

by adding a compound according to formula (I) as disclosed above, which is also called component (a) herein.

In one embodiment, at least one protease is selected from the group of serine endopeptidases (EC 3.4.21), more preferably selected from the group of subtilisin type proteases (EC 3.4.21.62). The protease may be selected from subtilisin 147 and/or 309 as disclosed in WO 89/06279 or variants thereof having proteolytic activity, subtilisin from *Bacillus lentus* as disclosed in WO 91/02792 or variants thereof having proteolytic activity, and subtilisin according to SEQ ID NO:22 as described in EP 1921147 or variants thereof having proteolytic activity.

In one embodiment, at least one lipase is selected from the group of fungal triacylglycerol lipase (EC class 3.1.1.3). Fungal triacylglycerol lipase may be selected from *Thermomyces lanuginose* lipase. In one embodiment, *Thermomyces lanuginosa* lipase is selected from triacylglycerol lipase according to amino acids 1-269 of SEQ ID NO:2 of

US 5869438 and variants thereof having lipolytic activity.

Stabilized component (b) in this context means that the wash performance towards at least one enzyme-sensitive stain, preferably towards at least amylase-sensitive stain, of a liquid detergent formulation comprising component (b) after 4 to 8 weeks of storage at 37°C is increased by at least 5%, at least 6%, at least 7%, at least 8%, at least 9%, or at least 10% when compared to a liquid detergent formulation lacking component (a) stored for the same time at the same temperature. Stabilized component (b) may mean that the wash performance of a liquid detergent formulation comprising component (b) after 8 weeks of storage at 37°C is increased by at least 5%, at least 6%, at least 7%, at least 8%, at least 9%, or at least 10% when compared to a liquid detergent formulation lacking component (a) stored for the same time at the same temperature.

In one aspect, the invention relates to the use of component (a) to reduce loss of amylolytic activity during storage, preferably at 37°C for 21, 28 and/or 42 days, of component (b) within a liquid detergent formulation, preferably comprising

EDTA and/or DTPA in amounts up to 3% by weight, preferably up to 2.5% and/or

MGDA and/or GLDA in amounts in the range of 10% to 30% by weight, preferably in the range of 15% to 25%, all relative to the total weight of the liquid detergent formulation,

wherein component (b) comprises

at least one amylase as disclosed above, preferably selected from alpha-amylases (EC 3.2.1.1) as disclosed above, more preferably selected from

- amylase from *Bacillus sp. 707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity;

and optionally at least one further enzyme, preferably selected from the group of proteases, lipases, cellulases, and mannanases - all as disclosed above.

In one embodiment, at least one protease is selected from the group of serine endopeptidases (EC 3.4.21), more preferably selected from the group of subtilisin type proteases (EC 3.4.21.62). The protease may be selected from subtilisin 147 and/or 309 as disclosed in WO 89/06279 or variants thereof having proteolytic activity, subtilisin from *Bacillus lentus* as disclosed in WO 91/02792 or variants thereof having proteolytic activity, and subtilisin according to SEQ ID NO:22 as described in EP 1921147 or variants thereof having proteolytic activity.

In one embodiment, at least one lipase is selected from the group of fungal triacylglycerol lipase (EC class 3.1.1.3). Fungal triacylglycerol lipase may be selected from *Thermomyces lanuginose* lipase. In one embodiment, *Thermomyces lanuginosa* lipase is selected from triacylglycerol lipase according to amino acids 1-269 of SEQ ID NO:2 of US 5869438 and variants thereof having lipolytic activity.

In one aspect, the invention relates to the method to reduce loss of amylolytic activity during storage, preferably at 37°C for 21, 28 and/or 42 days, of component (b) within a liquid detergent formulation, preferably comprising

EDTA and/or DTPA in amounts up to 3% by weight, preferably up to 2.5% and/or

MGDA and/or GLDA in amounts in the range of 10% to 30% by weight, preferably in the range of 15% to 25%, all relative

to the total weight of the liquid detergent formulation,
wherein component (b) comprises
at least one amylase as disclosed above, preferably selected from alpha-amylases (EC 3.2.1.1) as disclosed above, more preferably selected from

- amylase from *Bacillus sp. 707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from amylases according to SEQ ID NO: 3 of WO 2016/092009;
- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;
- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity;

and optionally at least one further enzyme, preferably selected from the group of proteases, lipases, cellulases, and mannanases - all as disclosed above;
by adding a compound according to formula (I) as disclosed above, which is also called component (a) herein.

In one embodiment, at least one protease is selected from the group of serine endopeptidases (EC 3.4.21), more preferably selected from the group of subtilisin type proteases (EC 3.4.21.62). The protease may be selected from subtilisin 147 and/or 309 as disclosed in WO 89/06279 or variants thereof having proteolytic activity, subtilisin from *Bacillus lentus* as disclosed in WO 91/02792 or variants thereof having proteolytic activity, and subtilisin according to SEQ ID NO:22 as described in EP 1921147 or variants thereof having proteolytic activity.

In one embodiment, at least one lipase is selected from the group of fungal triacylglycerol lipase (EC class 3.1.1.3). Fungal triacylglycerol lipase may be selected from *Thermomyces lanuginose* lipase. In one embodiment, *Thermomyces lanuginosa* lipase is selected from triacylglycerol lipase according to amino acids 1-269 of SEQ ID NO:2 of US 5869438 and variants thereof having lipolytic activity.

In one aspect, the invention relates to a method to increase storage stability of a liquid detergent formulation comprising at least one amylase and optionally comprising
EDTA and/or DTPA in amounts up to 3% by weight, preferably up to 2.5% and/or
MGDA and/or GLDA in amounts in the range of 10% to 30% by weight, preferably in the range of 15% to 25%, all relative to the total weight of the liquid detergent formulation, as disclosed above,
by adding at least one compound according to formula (I) to the detergent formulation:

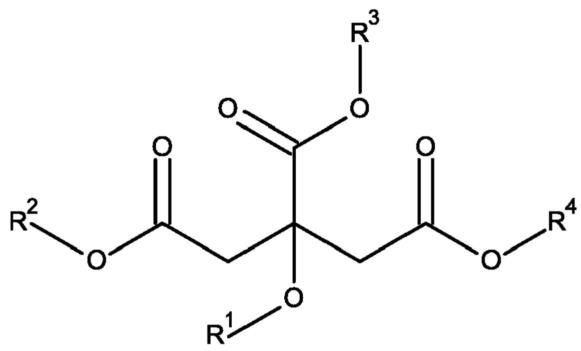

(I)

wherein the variables of formula (I) are as follows:

$R^1$ is selected from H and $C_1$-$C_{10}$ alkylcarbonyl, wherein alkyl may be linear or branched and may bear one or more hydroxyl groups;

$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H.

In one embodiment, storage stability of said liquid detergent formulation is increased during storage at 37°C for 21, 28 and/or 42 days when compared to a liquid detergent formulation lacking the compound according to formula (I) stored under the same conditions. Increased storage stability within this invention may mean that the increase in amylolytic stability in the presence of component (a) is at least 5%, at least 10%, at least 15%, at least 20%, at least 25%, at least 30%, at least 40%, at least 50%, least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or at least 99.5%, when compared to the amylolytic activity in the absence of component (a).

## Further use

The invention relates to a method for removing enzyme-sensitive stains comprising the steps of contacting a stain with a detergent formulation of the invention comprising components (a) and (b) and one or more detergent components - all as disclosed above. In one embodiment, the method for removing stains includes steps performed by an automatic device such as a laundry machine or an automatic dishwasher.
In one embodiment, the detergent formulation comprises the enzyme preparation of the invention.
In one aspect, the method relates to the removal of stains comprising starch. In one embodiment, removing of stains comprising starch may be done at cleaning temperatures ≤ 40°C, at cleaning temperatures ≤ 30°C, at cleaning temperatures ≤ 25°C, or at cleaning temperatures ≤ 20°C.
In one embodiment, the invention relates to a method for removing stains comprising starch at a cleaning temperature of temperature ≤ 30°C, wherein the method comprises the steps of contacting the stain with a detergent formulation of the invention comprising components (a) and (b) and one or more detergent components. Components (a) and (b) are those as disclosed above. Component (b), in one embodiment comprises at least one amylase as disclosed above, preferably selected from alpha-amylases (EC 3.2.1.1), more preferably selected from

- amylase from *Bacillus sp. 707* or variants thereof having amylolytic activity, preferably selected from amylases having SEQ ID NO:6 as disclosed in WO 99/19467 and variants thereof having amylolytic activity;
- amylase selected from those comprising amino acids 1 to 485 of SEQ ID NO:2 as described in WO 00/60060 those having SEQ ID NO: 12 as described in WO 2006/002643, and variants thereof having amylolytic activity;
- amylase from *Bacillus halmapalus* or variants thereof having amylolytic activity, preferably selected from amylases having SEQ ID NO: 1 and 2 as disclosed in WO 2013/001078; having SEQ ID NO:6 as described in WO 2011/098531; and variants thereof having amylolytic activity;
- amylase from *Bacillus amyloliquefaciens* or variants thereof having amylolytic activity, preferably selected from

amylases according to SEQ ID NO: 3 of WO 2016/092009;

- hybrid amylases according to WO 2014/183920 with A and B domains having at least 90% identity to SEQ ID NO:2 of WO 2014/183920 and a C domain having at least 90% identity to SEQ ID NO:6 of WO 2014/183920, wherein the hybrid amylase has amylolytic activity; preferably the hybrid alpha-amylase is at least 95% identical to SEQ ID NO: 23 of WO 2014/183920 and having amylolytic activity;

- hybrid amylase according to WO 2014/183921 with A and B domains having at least 75% identity to SEQ ID NO: 2, SEQ ID NO: 15, SEQ ID NO: 20, SEQ ID NO: 23, SEQ ID NO: 29, SEQ ID NO: 26, SEQ ID NO: 32, and SEQ ID NO: 39 as disclosed in WO 2014/183921 and a C domain having at least 90% identity to SEQ ID NO: 6 of WO 2014/183921, wherein the hybrid amylase has amylolytic activity; preferably, the hybrid alpha-amylase is at least 95% identical to SEQ ID NO: 30 as disclosed in WO 2014/183921 and having amylolytic activity;

and optionally at least one further enzyme, preferably selected from the group of proteases, lipases, cellulases, and mannanases - all as disclosed above.

In one embodiment, at least one protease is selected from the group of serine endopeptidases (EC 3.4.21), more preferably selected from the group of subtilisin type proteases (EC 3.4.21.62). The protease may be selected from subtilisin 147 and/or 309 as disclosed in WO 89/06279 or variants thereof having proteolytic activity, subtilisin from *Bacillus lentus* as disclosed in WO 91/02792 or variants thereof having proteolytic activity, and subtilisin according to SEQ ID NO:22 as described in EP 1921147 or variants thereof having proteolytic activity.

In one embodiment, at least one lipase is selected from the group of fungal triacylglycerol lipase (EC class 3.1.1.3). Fungal triacylglycerol lipase may be selected from *Thermomyces lanuginose* lipase. In one embodiment, *Thermomyces lanuginosa* lipase is selected from triacylglycerol lipase according to amino acids 1-269 of SEQ ID NO:2 of US 5869438 and variants thereof having lipolytic activity.

## Examples

The invention will be further illustrated by working examples.

General remarks: percentages are weight percent unless specifically noted otherwise.

## I. Tested compounds

A) Compounds according to formula (I) - (component (a)):

A.1 Triethylcitrate - purchased from Sigma Aldrich

A.2 Tripropylcitrate - purchased from Sigma Aldrich

A.3 Tributylcitrate - purchased from Sigma Aldrich

A.4 Acetyltributylcitrate - purchased from Sigma Aldrich

A.5 Acetyltriethylcitrate - purchased from Sigma Aldrich

A.6 Monoethylcitrate - purchased from Sigma Aldrich

A.7 Diethylcitrate
Synthesis of as described in: Journal of Chemical & Engineering Data 2018, DOI: 10.1021/acs.jced.7b01060, C. Berdugo, A. Suaza, M. Santaella, O. Sanchez

A.8 Tribenzylcitrate
Synthesis as described in WO2007/14471 A1, 2007; Location in patent: Page/Page column 19; 27-28

A.9 Trisalicylcitrate
Synthesis as described in WO2007/14471 A1, 2007; Location in patent: Page/Page column 19; 27-28

B) Comparative compounds:

B.1: citric acid - purchased from Sigma Aldrich

B.2: citric acid trisodiumsalt - purchased from Sigma Aldrich

B.3: diethyloxalate - purchased from Sigma Aldrich

B.4: glyceroltriacetate (triacetine) - purchased from Sigma Aldrich

## II. Amylase and protease stability

The storage stability of amylase was assessed at 37°C.

Base test formulations were manufactured by making base formulations I to V by mixing the components according to Table 1.

Amylases used: Amy1 = Stainzyme, Amy2 = Amplify, Amy3 = Stainzyme Plus L (12L)

Protease used: (S) Savinase Ultra 16.0L (CAS-No. 9014-01-1, EC-No. 232-752-2) was purchased from Sigma-Aldrich

The respective component (a) or comparative compound was added, if applicable, to the respective base formulation in amounts as indicated in Table 1.

Amylase (component (b)) was added, to the respective base formulation in amounts as indicated in Table 1. The amount of amylase as provided in Table 1 refers to active protein.

Protease (component (b)) was added, to the respective base formulation in amounts as indicated in Table 1. The amount of protease as provided in Table 1 refers to active protein.

Water was added to accomplish the balance to 100.

Table 1: liquid formulation

| Ingredients | wt% in formulation | | | | |
|---|---|---|---|---|---|
| | I. | II. | III. | IV | V |
| Base formulation: | | | | | |
| (Comp.1) | 20 | 15 | - | 15 | 10 |
| (Comp.2) | - | 5 | 5 | - | 5 |
| (Comp.3) | - | - | 15 | 10 | 10 |
| (Comp.4) | 5 | 2.5 | 2.5 | 5 | 2.5 |
| (Comp.5) | 5 G | 5 P | 5 P | 5 G | 5 P |
| (Comp.6) | 2.5 | 2.0 | 2.0 | 2.5 | 2.5 |
| (Comp.7) | - | 1 | 1 | - | 1 |
| (Comp.8) | 0.3 | 0.3 | 0.3 | 0.3 | 0.3 |
| Additives: | | | | | |
| Protease | 2.5 | 2.5 | 2.5 | 3.0 | 3.0 |
| Amylase* | 0.5 Amy1 | 0.5 Amy3 | 0.5 Amy2 | 0.5 Amy1 | 0.5 Amy2 |
| component (a)** | 1.5 | 1.5 | 1.5 | 2.0 | 2.0 |

(continued)

| Additives: | |
|---|---|
| balance | Water to 100 after adjust pH 7.5 with citric acid |
| (Comp.1): Trilon M fl (Max liqu)<br>(Comp.2): Citric acid<br>(Comp.3): GLDA 50% solution<br>(Comp.4): PAA, Polyacrylic acid Mw 5.000 g/mol<br>(Comp.5): Glycerol (G) or Propanediol (P)<br>(Comp.6): Dehypon WET<br>(Comp.7): $Na_4$HEDP<br>(Comp.8) Thickener (Rheocare XGN)<br>** for comparative tests without inventive compounds those were replaced by the same amount of glycerol | |

Amylase activity at certain points in time as indicated in Table 2 was measured quantitatively by the release of the chromophore *para*-nitrophenol (pNP) from the substrate (Ethyliden-blocked-pNPG7, Roche Applied Science 10880078103). The alpha-amylase degrades the substrate into smaller molecules and α-glucosidase (Roche Applied Science 11626329103), which is present in excess compared to the α-amylase, process these samller products until pNP is released; the release of pNP, measured via an increase of absorption at 405 nm, is directly proportional to the α-amylase activity of the sample. Amylase standard: Termamyl 120 L (Sigma 3403).

Table 2 displays amylase activity measured in liquid formulations after storage for 1 to 30 days at 37°C. The amylolytic activity values provided were calculated referring to the value determined in the reference formulation at the time 0.

The nomenclature of formulations is as follows: the Roman number before the full stop characterizes the base formulation, the Arabian number the type of compound (A.# compound according to invention (component (a)); B.# comparative compound).

Table 2: amylase activity in the course of time of storage at 37°C

| Formulation identifier | | T0 | 3d | 7d | 14d | 21d | 28d | 42d |
|---|---|---|---|---|---|---|---|---|
| Base formulation | compound | | | | | | | |
| I. | 0 | 100 | 76 | 59 | 48 | 34 | 27 | 18 |
| I. | A.1 | 100 | 98 | 93 | 88 | 80 | 74 | 61 |
| I. | A.2 | 101 | 100 | 96 | 90 | 84 | 79 | 66 |
| I. | A.4 | 102 | 103 | 97 | 93 | 86 | 82 | 69 |
| I. | A.5 | 97 | 91 | 80 | 72 | 63 | 55 | 37 |
| I. | A.6 | 98 | 95 | 84 | 79 | 70 | 61 | 48 |
| I. | A.7 | 99 | 96 | 86 | 81 | 74 | 63 | 50 |
| II. | 0 | 100 | 81 | 65 | 54 | 40 | 32 | 25 |
| II. | A.1 | 100 | 100 | 94 | 90 | 84 | 77 | 65 |
| II. | A.2 | 102 | 100 | 97 | 93 | 87 | 83 | 69 |
| II. | A.4 | 102 | 100 | 95 | 91 | 84 | 80 | 70 |
| II. | A.7 | 99 | 94 | 86 | 82 | 72 | 64 | 53 |
| II. | A.8 | 99 | 100 | 95 | 90 | 83 | 81 | 71 |
| II. | A.9 | 98 | 97 | 94 | 88 | 80 | 77 | 66 |
| III. | 0 | 98 | 80 | 66 | 56 | 43 | 34 | 27 |
| III. | A.1 | 100 | 98 | 95 | 91 | 86 | 78 | 68 |
| III. | A.2 | 100 | 102 | 97 | 93 | 87 | 81 | 71 |
| III. | A.4 | 99 | 100 | 96 | 93 | 85 | 80 | 70 |

(continued)

| Formulation identifier | | T0 | 3d | 7d | 14d | 21d | 28d | 42d |
|---|---|---|---|---|---|---|---|---|
| Base formulation | compound | | | | | | | |
| III. | A.5 | 96 | 96 | 92 | 87 | 78 | 71 | 55 |
| III. | A.6 | 97 | 96 | 90 | 83 | 74 | 63 | 49 |
| III. | A.7 | 98 | 96 | 91 | 85 | 78 | 70 | 60 |
| IV. | 0 | 97 | 74 | 58 | 47 | 34 | 25 | 15 |
| IV. | A.1 | 98 | 96 | 92 | 88 | 83 | 73 | 61 |
| IV. | A.3 | 98 | 95 | 90 | 84 | 77 | 68 | 57 |
| IV. | A.7 | 99 | 94 | 88 | 82 | 74 | 63 | 52 |
| IV. | A.8 | 98 | 96 | 92 | 86 | 81 | 70 | 59 |
| IV. | A.9 | 98 | 95 | 89 | 84 | 80 | 72 | 54 |
| V. | 0 | 99 | 76 | 60 | 51 | 39 | 28 | 19 |
| V. | A.1 | 98 | 96 | 91 | 87 | 80 | 73 | 62 |
| V. | A.2 | 98 | 95 | 90 | 84 | 76 | 66 | 54 |
| V. | A.4 | 100 | 94 | 88 | 83 | 72 | 65 | 52 |
| V. | A.7 | 98 | 96 | 87 | 74 | 63 | 55 | 47 |
| V. | A.8 | 98 | 93 | 86 | 80 | 77 | 66 | 59 |
| V. | A.9 | 97 | 93 | 86 | 75 | 66 | 60 | 54 |

Protease activity at certain points in time as indicated in Table 3 was be determined by employing Succinyl-Ala-Ala-Pro-Phe-p-nitroanilide (Suc-AAPF-pNA, short AAPF) as substrate. pNA is cleaved from the substrate molecule by proteolytic cleavage, resulting in release of yellow color of free pNA which was determined by measuring $OD_{405}$. Measurements were done at 20°C.

Table 3 displays protease activity measured in liquid formulations after storage for 1 to 30 days at 37°C. The proteolytic activity values provided in Table 3 were calculated referring to the value determined in the reference formulation at the time 0.

The nomenclature of formulations is as follows: the Roman number before the full stop characterizes the base formulation, the Arabian number the type of salt (A.# inventive salt (component (a)); B.# comparative compound). Zero ("0"): no salt, but diethylene glycol.

Table 3: protease activity in the course of time of storage at 37°C

| Formulation identifier | | T0 | 3d | 7d | 14d | 21d | 28d | 42d |
|---|---|---|---|---|---|---|---|---|
| Base formulation | compound | | | | | | | |
| I. | 0 | 100 | 93 | 87 | 75 | 65 | 57 | 43 |
| I. | A.1 | 100 | 97 | 94 | 89 | 83 | 79 | 67 |
| I. | A.2 | 101 | 100 | 95 | 90 | 85 | 81 | 71 |
| I. | A.4 | 102 | 100 | 94 | 90 | 84 | 82 | 70 |
| I. | A.5 | 98 | 96 | 90 | 84 | 80 | 76 | 62 |
| I. | A.6 | 100 | 94 | 90 | 81 | 73 | 67 | 56 |
| I. | A.7 | 100 | 94 | 91 | 85 | 81 | 76 | 63 |
| II. | 0 | 98 | 93 | 89 | 78 | 68 | 61 | 47 |
| II. | A.1 | 100 | 99 | 96 | 90 | 85 | 80 | 69 |

(continued)

| Formulation identifier | | T0 | 3d | 7d | 14d | 21d | 28d | 42d |
|---|---|---|---|---|---|---|---|---|
| Base formulation | compound | | | | | | | |
| II. | A.2 | 101 | 100 | 96 | 91 | 87 | 83 | 72 |
| II. | A.4 | 102 | 100 | 95 | 90 | 85 | 81 | 70 |
| II. | A.7 | 100 | 98 | 93 | 88 | 85 | 80 | 71 |
| II. | A.8 | 100 | 94 | 93 | 90 | 87 | 83 | 75 |
| II. | A.9 | 98 | 96 | 92 | 85 | 81 | 75 | 68 |
| III. | 0 | 96 | 93 | 86 | 79 | 71 | 65 | 50 |
| III. | A.1 | 100 | 99 | 93 | 88 | 85 | 74 | 67 |
| III. | A.2 | 101 | 100 | 95 | 93 | 88 | 83 | 73 |
| III. | A.4 | 101 | 99 | 95 | 91 | 86 | 81 | 70 |
| III. | A.5 | 100 | 98 | 92 | 83 | 79 | 71 | 65 |
| III. | A.6 | 100 | 94 | 90 | 84 | 78 | 70 | 62 |
| III. | A.7 | 100 | 97 | 95 | 92 | 87 | 74 | 64 |
| IV. | 0 | 96 | 91 | 84 | 74 | 68 | 59 | 45 |
| IV. | A.1 | 98 | 96 | 92 | 86 | 80 | 72 | 65 |
| IV. | A.3 | 99 | 98 | 90 | 85 | 78 | 70 | 67 |
| IV. | A.7 | 98 | 96 | 88 | 79 | 74 | 68 | 62 |
| IV. | A.8 | 98 | 96 | 93 | 88 | 83 | 75 | 68 |
| IV. | A.9 | 100 | 97 | 93 | 86 | 79 | 70 | 64 |
| V. | 0 | 98 | 92 | 87 | 76 | 69 | 62 | 48 |
| V. | A.1 | 99 | 94 | 90 | 85 | 81 | 75 | 65 |
| V. | A.2 | 100 | 95 | 92 | 87 | 82 | 77 | 70 |
| V. | A.4 | 98 | 94 | 90 | 85 | 80 | 76 | 68 |
| V. | A.7 | 97 | 93 | 88 | 79 | 72 | 66 | 54 |
| V. | A.8 | 100 | 96 | 92 | 86 | 82 | 77 | 69 |
| V. | A.9 | 98 | 98 | 93 | 86 | 81 | 74 | 67 |

**III. Stability in laundry formulation**

The storage stability of amylase was assessed at 37°C.

Base test formulations were manufactured by making base formulations VI to IX by mixing the components according to Table 4.

Table 4: liquid laundry formulations

| Ingredients | Wt-% in formulation | | | |
|---|---|---|---|---|
| | VI. | VII. | VIII. | IX. |
| Base formulation: | | | | |
| (Comp.1) | - | 4 | 4 | - |
| (Comp.2) | 3 | 2 | 2 | 3 |
| (Comp.3) | 14 | 14 | 14 | 14 |

(continued)

| Ingredients | Wt-% in formulation | | | |
|---|---|---|---|---|
| | VI. | VII. | VIII. | IX. |
| Base formulation: | | | | |
| (Comp.4) | 4 | 4 | 4 | 4 |
| (Comp.5) | 2.0 EDTA | 2.0 EDTA | 2.5 DTPA | 2.5 DTPA |
| (Comp.6) | 4 | 4 | 4 | 4 |
| Glycerol | 2,5 | 2 | 2,5 | 2 |
| Ethanol | 1,5 | 1,5 | 1,5 | 1,5 |
| Propyleneglycol | 3 | 3,5 | 3 | 3,5 |
| Additives: | | | | |
| Savinase 16.0L | 0,7 | 1,0 | - | 0.7 |
| Amylase | 0,5 | 0,5 | 0.5 | 0.5 |
| component (a)** | 2,5 | 2,5 | 2,5 | 2,5 |
| balance | Water to 100 | | | |

(Comp.1): n-$C_{18}$-alkyl-$(OCH_2CH_2)_{25}$-OH
(Comp.2): Tallow oil soap C14-C18 Carbonic acid, sodium salt
(Comp.3): Sodium $C_{10}$-$C_{12}$-alkyl benzenesulfonate
(Comp.4): Sodium laurethsulfate - n-$C_{12}H_{25}$-O-$(CH_2CH_2O)_3$-$SO_3Na$
(Comp.5): Complexing agent EDTA or DTPA
(Comp.6) mixture Na-citrate:Na-formiate 9:1
** for comparative tests without inventive compounds those were replaced by the same amount of water.

Amylase activity at certain points in time as indicated in Table 5 was measured quantitatively by the release of the chromophore *para*-nitrophenol (pNP) from the substrate (Ethyliden-blocked-pNPG7, Roche Applied Science 10880078103). The alpha-amylase degrades the substrate into smaller molecules and $\alpha$-glucosidase (Roche Applied Science 11626329103), which is present in excess compared to the $\alpha$-amylase, process these smaller products until pNP is released; the release of pNP, measured via an increase of absorption at 405 nm, is directly proportional to the $\alpha$-amylase activity of the sample. Amylase standard: Termamyl 120 L (Sigma 3403).

Table 5 displays amylase activity measured in liquid formulations after storage for 1 to 28 days at 37°C. The amylolytic activity values provided were calculated referring to the value determined in the reference formulation at the time 0.

The nomenclature of formulations is as follows: the Roman number before the full stop characterizes the base formulation, the Arabian number the type of compound (A.# compound according to invention (component (a)); B.# comparative compound).

Table 5: amylase activity in the course of time of storage at 37°C

| Formulation identifier | | T0 | 3d | 7d | 14d | 21d | 28d |
|---|---|---|---|---|---|---|---|
| Base formulation | compound | | | | | | |
| VI. | - | 100 | 71 | 52 | 40 | 28 | 18 |
| VI. | A1 | 100 | 98 | 91 | 86 | 81 | 73 |
| VI. | A.2 | 101 | 94 | 86 | 80 | 74 | 65 |
| VI. | A.9 | 100 | 95 | 87 | 82 | 75 | 67 |
| VII. | - | 100 | 73 | 55 | 43 | 32 | 23 |
| VII. | A.1 | 100 | 95 | 88 | 79 | 73 | 68 |
| VII. | A.5 | 98 | 92 | 81 | 72 | 66 | 61 |

(continued)

| Formulation identifier | | T0 | 3d | 7d | 14d | 21d | 28d |
|---|---|---|---|---|---|---|---|
| Base formulation | compound | | | | | | |
| VII. | A.7 | 99 | 90 | 82 | 69 | 62 | 56 |
| VIII. | - | 100 | 77 | 56 | 48 | 34 | 25 |
| VIII. | A.1 | 100 | 98 | 93 | 88 | 83 | 75 |
| VIII. | A.2 | 99 | 94 | 87 | 83 | 77 | 69 |
| VIII. | A.5 | 100 | 93 | 83 | 74 | 67 | 64 |
| VIII. | A.9 | 100 | 96 | 88 | 84 | 78 | 69 |
| IX. | - | 100 | 71 | 53 | 40 | 33 | 26 |
| IX. | A.1 | 99 | 98 | 90 | 85 | 78 | 70 |
| IX. | A.2 | 100 | 93 | 85 | 81 | 72 | 63 |
| IX. | A.5 | 98 | 91 | 78 | 69 | 61 | 53 |

The detergent performance of formulations according to Table 4 in cleaning amylase-sensitive stains can be carried out on applicable types of test fabrics. Pre-soiled test fabrics may be purchased from wfk test fabrics GmbH, Krefeld; EMPA = Swiss Federal Institute of Materials Testing; or CFT = Center for Test Material B.V.

The test can be performed as follows: a multi stain monitor comprising e.g. 8 standardized soiled fabric patches, each of 2.5 x 2.5 cm size and stitched on two sides to a polyester carrier is washed together in a launder-O-meter with 2.5 g of cotton fabric and 5g/L of the liquid test laundry detergent, Table 4.

The conditions may be chosen as follows: Device: Launder-0-Meter from SDL Atlas, Rock Hill, USA. Washing liquor: 250 ml, washing time: 60 minutes, washing temperature: 30°C. Water hardness: 2.5 mmol/L; Ca:Mg:$HCO_3$ 4:1:8; fabric to liquor ratio 1:12; after the wash cycle, the multi stain monitors are rinsed in water, followed by drying at ambient temperature over a time period of 14 hours.

The total level of cleaning can be evaluated using color measurements: Reflectance values of the stains on the monitors are measured using a sphere reflectance spectrometer (SF 500 type from Datacolor, USA, wavelength range 360-700nm, optical geometry d/8°) with a UV cutoff filter at 460 nm. In this case, with the aid of the CIE-Lab color space classification, the brightness L *, the value a * on the red - green color axis and the b * value on the yellow - blue color axis, are measured before and after washing and averaged for the 8 stains of the monitor. The change of the color value (Δ E) value, can be defined and calculated automatically by the evaluation color tools on the following equation:

$$\Delta E = \text{root} (\Delta \text{ Delta a} * 2 + \Delta \text{ Delta b} * 2 + \Delta \text{ Delta L} * 2),$$

Δ E is a measure of the achieved cleaning effect. All measurements may be repeated six times to yield an average number. Note that higher Δ E values show better cleaning. A difference of 1 unit can be detected by a skilled person. A non-expert can detect 2 units easily.

The launder-O-meter tests can be executed with freshly prepared formulations according to Table 4 and/or with the same formulations after storage at 37°C for a defined time such as 3 days, about 7 days, about 14 days, about 21 days, about 28 days, about. As an approximation one week at 37°C is equivalent to 3½ weeks at 20°C.

## Claims

**1.** Enzyme preparation comprising

component (a): at least one compound according to general formula (I)

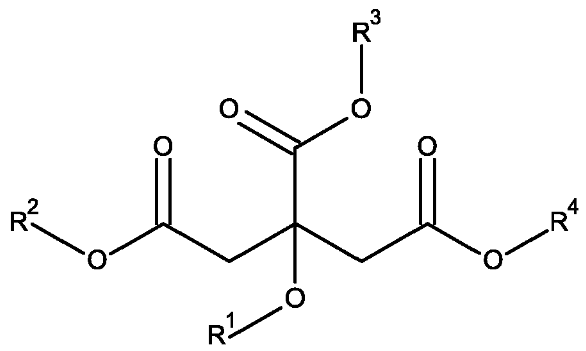

(I)

wherein the variables in formula (I) are defined as follows:

$R^1$ is selected from H and $C_1$-$C_{10}$ alkylcarbonyl, wherein alkyl may be linear or branched and may bear one or more hydroxyl groups;
$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H;

component (b): at least one enzyme selected from the group of hydrolases (EC 3), preferably at least one enzyme selected from the group of amylases, more preferably at least one enzyme selected from the group of alpha-amylases (EC 3.2.1.1); and/or at least one enzyme is selected from proteases, preferably from subtilisin type proteases (EC 3.4.21.62);
and optionally
component (c): compound selected from at least one solvent, at least one enzyme stabilizers different from component (a), and at least one compound stabilizing the liquid enzyme preparation as such.

2. Enzyme preparation according to claim 1 wherein said enzyme preparation comprises component (a) in amounts in the range of 0.1 to 30% by weight relative to the total weight of the enzyme preparation.

3. Enzyme preparation according to any of the preceding claims **characterized in** at least one hydrolase, preferably selected from alpha-amylase and subtilisin type protease, comprised in component (b) is stabilized when compared to enzyme preparation lacking component (a).

4. Process for making a stabile enzyme preparation, said process comprising the steps of mixing at least

component (a): at least one compound according to general formula (I)

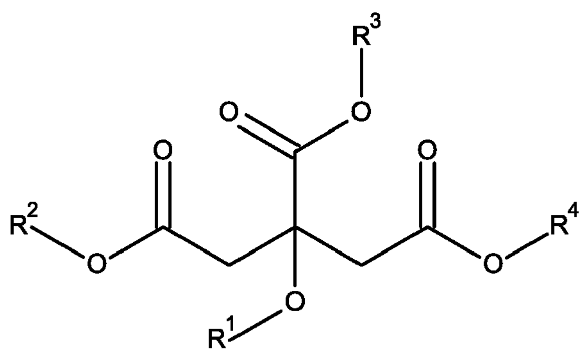

(I)

wherein the variables in formula (I) are defined as follows:

$R^1$ is selected from H and $C_1$-$C_{10}$ alkylcarbonyl, wherein alkyl may be linear or branched and may bear one or more hydroxyl groups;

$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-arylalkyl, wherein alkyl of the latter is selected from linear $C_1$-$C_8$ alkyl or branched $C_3$-$C_8$ alkyl, wherein at least one of $R^2$, $R^3$, and $R^4$ is not H,

component (b): at least one enzyme selected from the group of hydrolases (EC 3), preferably at least one enzyme selected from the group of amylases, more preferably at least one enzyme selected from the group of alpha-amylases (EC 3.2.1.1); and/or at least one enzyme is selected from proteases, preferably from subtilisin type proteases (EC 3.4.21.62);
and optionally
component (c): compound selected from at least one solvent, at least one enzyme stabilizers different from component (a), and at least one compound stabilizing the liquid enzyme preparation as such.

**5.** Method of reducing loss of amylolytic activity of at least one amylase comprised in a liquid enzyme preparation during storage by the step of adding at least one compound according to formula (I):

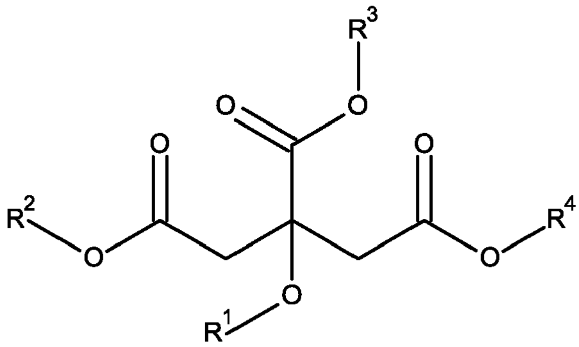

(I)

wherein the variables in formula (I) are defined as follows:

$R^1$ is selected from H and $C_1$-$C_{10}$ alkylcarbonyl, wherein alkyl may be linear or branched and may bear one or more hydroxyl groups;
$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-arylalkyl, wherein alkyl of the latter is selected from linear $C_1$-$C_8$ alkyl or branched $C_3$-$C_8$ alkyl, wherein at least one of $R^2$, $R^3$, and $R^4$ is not H.

**6.** Use of a compound according to formula (I):

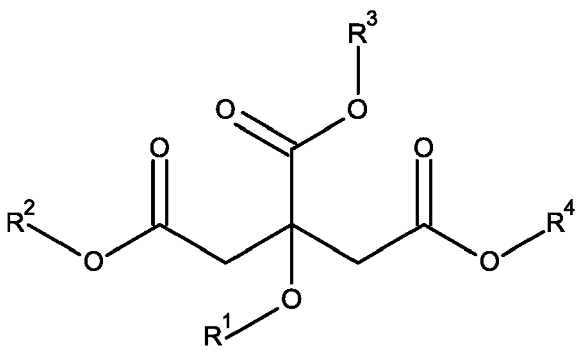

(I)

wherein the variables in formula (I) are defined as follows:

$R^1$ is selected from H and $C_1$-$C_{10}$ alkylcarbonyl, wherein alkyl may be linear or branched and may bear one or more hydroxyl groups;
$R^2$, $R^3$, $R^4$ are independently from each other selected from H, linear $C_1$-$C_8$ alkyl, and branched $C_3$-$C_8$ alkyl,

$C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H

as additive for a composition comprising at least one hydrolase, preferably selected from alpha-amylases and subtilisin type proteases, wherein the compound according to formula (I) and the amylase are solid and wherein amylolytic activity of the amylase is stabilized when the compound according to formula (I) and the amylase are contacted with at least one solvent.

**7.** Use of the enzyme preparation of claims 1 to 3 to be formulated into detergent formulations, preferably liquid detergent formulations, wherein the enzyme preparation of claims 1 to 3 is mixed in one or more steps with one or more detergent components, and wherein the detergent formulation preferably comprises at least one complexing agent preferably selected from EDTA, DTPA, MGDA and GLDA in effective amounts.

**8.** Detergent formulation comprising the enzyme preparation of claims 1 to 3 and at least one detergent component, preferably at least one complexing agent preferably selected from EDTA, DTPA, MGDA and GLDA, in effective amounts.

**9.** Method of preparation of a detergent formulation comprising the steps of mixing at least

component (a): at least one compound according to general formula (I)

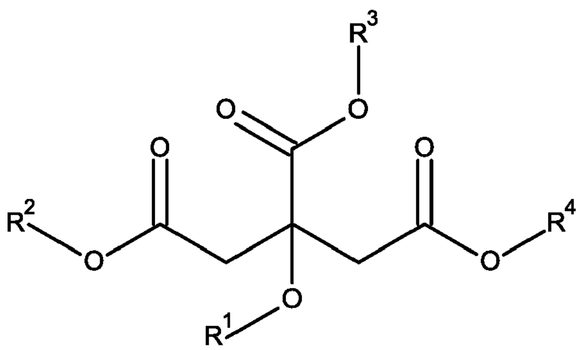

(I)

wherein the variables of formula (i) are as follows:

$R^1$ is selected from H and $C_1$-$C_{10}$ alkylcarbonyl, wherein alkyl may be linear or branched and may bear one or more hydroxyl groups;
$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-arylalkyl, wherein alkyl of the latter is selected from linear $C_1$-$C_8$ alkyl or branched $C_3$-$C_8$ alkyl, wherein at least one of $R^2$, $R^3$, and $R^4$ is not H

component (b): at least one enzyme selected from the group of hydrolases (EC 3), preferably at least one enzyme selected from the group of amylases, more preferably at least one enzyme selected from the group of alpha-amylases (EC 3.2.1.1); and/or at least one enzyme is selected from proteases, preferably from subtilisin type proteases (EC 3.4.21.62);
and at least one detergent component, preferably at least one complexing agent preferably selected from EDTA, DTPA, MGDA and GLDA, in effective amounts.

**10.** Method according to claim 9, comprising the steps of mixing the enzyme preparation of claims 1-3 and at least one detergent component, preferably at least one complexing agent preferably selected from EDTA, DTPA, MGDA and GLDA, in effective amounts.

**11.** Method for removing amylase-sensitive stains, comprising the step of contacting at least one stain with a detergent formulation according to claim 8, wherein component (b) of said detergent formulation comprises at least one alpha-amylase, and optionally further comprising at least one subtilisin type protease.

**12.** Method according to claim 11, wherein the amylase-sensitive stain is to be removed from a textile at a cleaning temperature of temperature ≤ 30°C.

**13.** Method to increase storage stability of a liquid detergent formulation comprising at least one hydrolase and optionally comprising at least one complexing agent preferably selected from EDTA, DTPA, MGDA and GLDA in effective amounts, by adding at least one compound according to formula (I) to the detergent formulation:

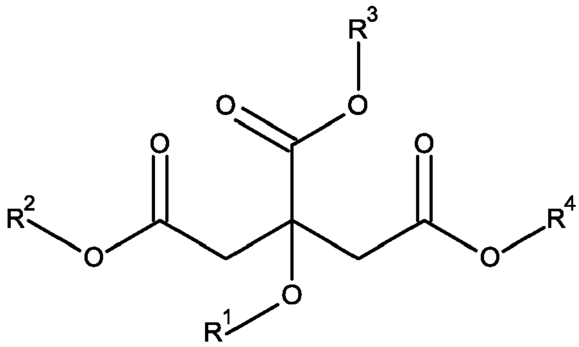

(I)

wherein the variables of formula (I) are as follows:

$R^1$ is selected from H and $C_1$-$C_{10}$ alkylcarbonyl, wherein alkyl may be linear or branched and may bear one or more hydroxyl groups;
$R^2$, $R^3$, $R^4$ are independently from each other selected from H, linear $C_1$-$C_8$ alkyl, and branched $C_3$-$C_8$ alkyl, $C_6$-$C_{10}$-aryl, non-substituted or substituted with one or more carboxylate or hydroxyl groups, and $C_6$-$C_{10}$-aryl-alkyl, wherein alkyl of the latter is selected from linear $C_1$-$C_8$ alkyl or branched $C_3$-$C_8$ alkyl, wherein at least one of $R^2$, $R^3$, and $R^4$ is not H.

**14.** Method according to claim 13, wherein the detergent is stored at 37°C for at least 20 days.

**15.** Method according to claims 13 and 14, wherein at least one hydrolase (EC 3), is selected from the group of alpha-amylases (EC 3.2.1.1) and subtilisin type proteases (EC 3.4.21.62), and combinations thereof.

Europäisches Patentamt
European Patent Office
Office européen des brevets

# EUROPEAN SEARCH REPORT

Application Number
EP 19 15 0242

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 228 703 A1 (NOVOZYMES AS [DK]) 11 October 2017 (2017-10-11) * paragraphs [0250], [0272] * * claims 19-30 * ----- | 1,4,7-12 | INV. C12N9/96 C12N9/28 C12N9/54 C11D3/386 |
| X | US 5 922 083 A (BISCARINI LAMBERTO [IT] ET AL) 13 July 1999 (1999-07-13) * claim 1 * ----- | 1,4 | |

TECHNICAL FIELDS SEARCHED (IPC)

C12N
C11D

The present search report has been drawn up for all claims

1

| Place of search | Date of completion of the search | Examiner |
|---|---|---|
| Munich | 3 July 2019 | Tudor, Mark |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 19 15 0242

This annex lists the patent family members relating to the patent documents cited in the above-mentioned European search report. The members are as contained in the European Patent Office EDP file on
The European Patent Office is in no way liable for these particulars which are merely given for the purpose of information.

03-07-2019

| Patent document cited in search report | Publication date | Patent family member(s) | Publication date |
|---|---|---|---|
| EP 3228703 A1 | 11-10-2017 | AR 108196 A1 | 25-07-2018 |
| | | BR 102017007291 A2 | 17-07-2018 |
| | | CA 2963670 A1 | 08-10-2017 |
| | | CN 107267485 A | 20-10-2017 |
| | | DK 201600210 A1 | 23-10-2017 |
| | | EP 3228703 A1 | 11-10-2017 |
| | | JP 2018019680 A | 08-02-2018 |
| | | US 2017292095 A1 | 12-10-2017 |
| US 5922083 A | 13-07-1999 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9510603 A
- WO 9402597 A
- WO 94018314 A
- WO 97043424 A
- WO 99019467 A
- WO 0210355 A
- WO 9919467 A
- WO 9623872 A
- WO 973296 A
- WO 99194671 A
- WO 2013001078 A
- WO 0022103 A
- WO 2009061380 A
- WO 2013184577 A
- WO 2010104675 A
- WO 0060060 A
- WO 2016092009 A
- WO 2006002643 A
- WO 2011098531 A
- WO 2013001087 A
- WO 2006066594 A
- WO 2014183920 A
- WO 2014183921 A
- WO 8906276 A
- EP 0283075 A
- WO 8906279 A
- WO 8909830 A
- WO 8909819 A
- WO 9106637 A
- WO 9102792 A
- EP 283075 A2
- WO 9523221 A
- DE 10064983
- WO 2003054184 A
- WO 2003056017 A
- WO 2003055974 A
- WO 2005063974 A
- WO 2005103244 A
- DE 102005028295
- WO 9219729 A
- WO 9634946 A
- WO 9820115 A
- WO 9820116 A
- WO 9911768 A
- WO 0144452 A
- WO 02088340 A
- WO 03006602 A
- WO 200403186 A
- WO 2004041979 A
- WO 2007006305 A
- WO 2011036263 A
- WO 2011036264 A
- WO 2011072099 A
- EP 1921147 A
- US 5352604 A
- EP 258068 A
- EP 305216 A

- WO 9205249 A
- WO 2009109500 A
- WO 9613580 A
- EP 218272 A
- WO 9425578 A
- WO 9530744 A
- WO 9535381 A
- WO 9600292 A
- EP 331376 A
- GB 1372034 A
- WO 9506720 A
- WO 9627002 A
- WO 9612012 A
- WO 9514783 A
- WO 2011150157 A
- WO 2012137147 A
- WO 2010065455 A
- WO 2011084412 A
- WO 2011084417 A
- WO 0060063 A
- WO 2011084599 A
- JP 64074992 B
- WO 9116422 A
- WO 9401541 A
- US 5389536 A
- WO 8809367 A
- WO 2010107560 A
- WO 9009446 A
- WO 0034450 A
- WO 0192502 A
- WO 2010111143 A
- WO 2005056782 A
- WO 200967279 A
- WO 2010100028 A
- WO 9522615 A
- WO 9704079 A
- WO 9707202 A
- WO 2007087508 A
- EP 407225 A
- EP 260105 A
- US 5869438 A
- JP 0304706 A
- JP 63056289 B
- JP 63036774 B
- JP 08051975 B
- WO 9711164 A
- WO 9118974 A
- WO 2014100018 A
- US 5476775 A
- WO 99064619 A
- WO 9324622 A
- WO 2011085747 A
- WO 09118375 A
- WO 9813459 A
- EP 0851023 A
- DE 19819187 A
- WO 200714471 A1

**Non-patent literature cited in the description**


- **NEEDLEMAN ; WUNSCH.** *J. Mol. Biol.,* 1979, vol. 48, 443-453
- **SIEZEN et al.** *Protein Eng.,* 1991, vol. 4, 719-737
- **SIEZEN et al.** *Protein Science,* 1997, vol. 6, 501-523
- **GUPTA et al.** *Appl. Microbiol. Biotechnol.,* 2002, vol. 60, 381-395
- **DELMAR et al.** *Analytical Biochem,* 1979, vol. 99, 316-320
- **VASANTHA et al.** *J. Bacteriol.,* 1984, vol. 159, 811-819
- **JA WELLS et al.** *Nucleic Acids Research,* 1983, vol. 11, 7911-7925
- **EL SMITH et al.** *J. Biol Chem,* 1968, vol. 243, 2184-2191
- **JACOBS et al.** *Nucl. Acids Res,* 1985, vol. 13, 8913-8926
- **GUPTA et al.** *Biotechnol. Appl. Biochem.,* 2003, vol. 37, 63-71
- **DARTOIS et al.** *Biochemica et Biophysica Acta,* 1992, vol. 1131, 253-360
- **HOFFMAN, W. S.** *J. Biol. Chem.,* 1937, vol. 120, 51
- Detergent Cake, Dishwashing Detergents, Liquid & Paste Detergents, Enzyme Detergents, Cleaning Powder & Spray Dried Washing Powder. complete Technology Book on Detergents with Formulations. Engineers India Research Institute (EIRI), 2015
- **C. BERDUGO ; A. SUAZA ; M. SANTAELLA ; O. SANCHEZ.** *Journal of Chemical & Engineering Data,* 2018
- *CHEMICAL ABSTRACTS,* 9014-01-1